(19) Europäisches Patentamt / European Patent Office / Office européen des brevets

(11) **EP 2 044 956 A1**

(12) **EUROPEAN PATENT APPLICATION**
published in accordance with Art. 153(4) EPC

(43) Date of publication:
**08.04.2009 Bulletin 2009/15**

(21) Application number: **07745133.4**

(22) Date of filing: **13.06.2007**

(51) Int Cl.:
***A61K 45/00*** *(2006.01)* ***A61K 39/395*** *(2006.01)*
***A61P 7/06*** *(2006.01)* ***A61P 35/02*** *(2006.01)*
***A61P 43/00*** *(2006.01)*

(86) International application number:
**PCT/JP2007/061850**

(87) International publication number:
**WO 2007/145227 (21.12.2007 Gazette 2007/51)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **14.06.2006 JP 2006165279**
**26.12.2006 JP 2006350553**

(71) Applicant: **CHUGAI SEIYAKU KABUSHIKI KAISHA**
**Tokyo, 115-8543 (JP)**

(72) Inventors:
- **YOSHIKUBO, Takashi**
  **Kamakura-shi, Kanagawa 247-8530 (JP)**
- **SHIINA, Masashi**
  **Kamakura-shi, Kanagawa 247-8530 (JP)**
- **INAGAKI, Yukiko**
  **Kamakura-shi, Kanagawa 247-8530 (JP)**

(74) Representative: **Vossius & Partner**
**Siebertstraße 4**
**81675 München (DE)**

## (54) HEMATOPOIETIC STEM CELL PROLIFERATION PROMOTER

(57) The present inventors discovered that the administration of an agonistic minibody (VB22B sc(Fv)2) against the TPO receptor resulted in not only the induction of human megakaryocyte-specific differentiation (increase in platelet precursor cells), but also the engraftment of transplanted hematopoietic stem cells derived from human cord blood (CD34-positive cells) and significant increase in multi-lineage hematopoietic precursor cells. TPO and TPO receptor agonists can be used as agents for promoting the growth of CD34-positive hematopoietic cells or agents for promoting the engraftment of transplanted cells in the bone marrow, which can be effective when administered alone (without using G-CSF and erythropoietin in combination) after hematopoietic stem cell transplantation (in particular, cord blood transplantation). Furthermore, TPO and TPO receptor agonists can be used as agents for promoting the growth and/or differentiation of multilineage hematopoietic precursor cells and agents for promoting the recovery of multilineage hematopoiesis.

FIG. 3

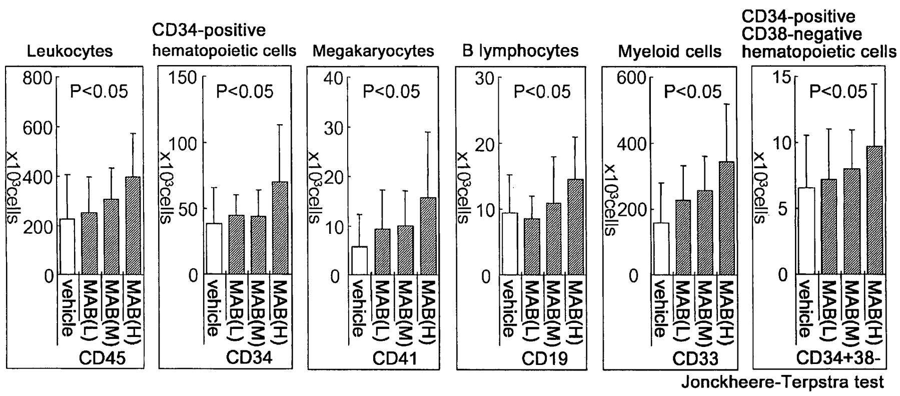


EP 2 044 956 A1

## Description

### Technical Field

The present invention relates to agents for promoting the growth of hematopoietic stem cells, which comprise an agonist for the TPO receptor (c-mpl) as an active ingredient. The present invention also relates to agents for promoting the growth and/or differentiation of CD34-positive hematopoietic cells, agents for promoting the engraftment of CD34-positive hematopoietic cells transplanted in the bone marrow, and agents for promoting the recovery of hematopoiesis after hematopoietic stem cell transplantation, wherein the agents comprise an agonist for the TPO receptor (c-mpl) as an active ingredient.

### Background Art

Thrombopoietin (TPO) is a cytokine that promotes the growth and differentiation of megakaryocytic lineage cells, and is known as a megakaryocyte colony-stimulating factor and a ligand for c-mpl. Upon ligand binding, most cytokine receptors dimerize to transduce signals into cells. It has been reported that TPO binds to its specific receptor, c-mpl, and allow the receptor to dimerize, thereby transducing signals into cells to exert the biological action (see Non-Patent Document 1).

It has been reported that among antibodies that bind to receptors with such properties, there are antibodies which exhibit agonistic activity. For example, an antibody against the erythropoietin (EPO) receptor has been reported to mimic the function of erythropoietin. When this antibody is converted into a monovalent antibody (Fab), it retains the activity to bind to the EPO receptor but loses the signal-transducing ability. This suggests that dimerization of the erythropoietin receptor mediated by divalent binding is essential for the signaling (see Non-Patent Document 2).

Furthermore, there are reports on c-mpl-binding antibodies have TPO receptor agonist activity (see Non-Patent Documents 3 and 4, and Patent Documents 1 to 3). Such antibodies have been used to promote the growth of hematopoietic stem cells.

For example, an experiment that examines the growth of cord blood cells after single administration of TPO to NOD/SCID mice to which human cord blood cells (CD34-positive cells) are transplanted (see Non-Patent Document 5) has been reported. According to this report, the frequencies of CD34 positive cells did not differ significantly with or without TPO treatment.

Furthermore, there is a report on the administration of a TPO receptor agonist, other than TPO (PEG-rHuMGDF), to cord blood transplantation model mice (see Non-Patent Document 6). This document merely reports the enhancing effect on platelet recovery, and not at all the effect on the engraftment of transplanted CD34-positive cells in the bone marrow.

In addition, another experiment has been reported, in which a c-mpl agonist was administered to NOG mice (which are more immune-deficient than NOD/SCID mice) two to six months after human cord blood cell transplantation (see Non-Patent Document 7). According to this document, CD34-positive cells were, however, not increased in the bone marrow.

Furthermore, another report describes that blood cell recovery could be actually accelerated by simultaneously administering three types of agents, TPO, G-CSF, and EPO, to a patient after human cord blood transplantation. However, since the three types of agents were administered to enhance trilineage hematopoiesis (middle of the right column of p. 198), the report does not suggest the effect of TPO alone on the proliferation and differentiation of CD34-positive cells (see Non-Patent Document 8).

Meanwhile, the mouse bone marrow transplantaion efficiency was reported to be increased by administering TPO to TPO-knockout mice (see Non-Patent Document 9). This document suggests that TPO also acts on not only platelets but also other lineages. However, it does not report whether grafted CD34-positive cells survive, or mention human cord blood.

As described above, no previous report has shown that the growth of hematopoietic stem cells could be successfully activated by administering antibodies with TPO receptor agonist activity.

Prior art documents related to the present invention are shown below:

[Patent Document 1]
WO 2002/33072
[Patent Document 2]
WO 2005/056604
[Patent Document 3]
WO 2005/107784
[Non-Patent Document 1]

Stem Cells, Vol. 14 suppl. 1, p. 124-132 (1996)
[Non-Patent Document 2]
Elliott S et al., J. Biol. Chem., Vol. 271(40), p. 24691-24697 (1996)
[Non-Patent Document 3]
Abe et al., Immunol. Lett. Vol. 61, p. 73-78 (1998)
[Non-Patent Document 4]
Bijia Deng et al., Blood, Vol. 92, p. 1981-1988 (1998)
[Non-Patent Document 5]
British Journal of Haematology, 122, 837-846 (2003)
[Non-Patent Document 6]
Japanese Journal of Transfusion Medicine, 46(3), 311-316 (2000)
[Non-Patent Document 7]
Blood, Vol. 107, p. 4300-4307 (2006)
[Non-Patent Document 8]
Bone Marrow Transplantation, 29, 197-204 (2002)
[Non-Patent Document 9]
The Journal of Clinical Investigation, 110(3), 389-394 (2002)

Disclosure of the Invention

[Problems to be Solved by the Invention]

The present invention was achieved in view of the above circumstances. An objective of the present invention is to provide agents which comprise an agonist for the TPO receptor as an active ingredient for promoting the growth of hematopoietic stem cells. Another objective of the present invention is to provide agents for promoting the growth and/or differentiation of CD34-positive hematopoietic cells, agents for promoting the engraftment of transplanted CD34-positive hematopoietic cells in the bone marrow, and agents for promoting the recovery of hematopoiesis after hematopoietic stem cell transplantation, wherein the agents comprise an agonist for the TPO receptor (c-mpl) as an active ingredient.

[Means for Solving the Problems]

The present inventors conducted dedicated studies to achieve the above-described objectives. The present inventors discovered that the administration of an agonistic minibody (VB22B sc(Fv)2) against the TPO receptor induced human megakaryocyte-specific differentiation (increase of megakaryocytic cells), as well as enhanced the engraftment of transplanted human cord blood-derived hematopoietic stem cells (CD34-positive hematopoietic cells) in the bone marrow, and induced significant growth of multilineage blood precursor cells. In view of the above, the present inventors conceived that TPO and TPO receptor agonists could be used as agents for promoting the growth of CD34-positive hematopoietic cells and agents for promoting the engraftment of transplanted cells in the bone marrow. Administration of these agents alone (without using G-CSF or erythropoietin in combination) after hematopoietic stem cell transplantation (in particular, cord blood transplantation) is expected to be effective. In addition, the present inventors conceived that TPO and TPO receptor agonists could be used as agents for promoting the growth and/or differentiation of multilineage hematopoietic precursor cells, and agents for promoting the recovery of multilineage hematopoiesis.
More specifically, the present invention provides [1] to [42] below:

[1] an agent for promoting the growth of hematopoietic stem cells, wherein the agent comprises an agonist for the TPO receptor (c-mpl) as an active ingredient;
[2] an agent for promoting the growth and/or differentiation of CD34-positive hematopoietic cells, wherein the agent comprises an agonist for the TPO receptor (c-mpl) as an active ingredient;
[3] an agent for enhancing the engraftment of transplanted CD34-positive hematopoietic cells in the bone marrow, wherein the agent comprises an agonist for the TPO receptor (c-mpl) as an active ingredient;
[4] an agent for promoting the recovery of hematopoiesis, wherein the agent comprises an agonist for the TPO receptor (c-mpl) as an active ingredient;
[5] the agent of any one of [1] to [4], wherein the agent is used for hematopoietic stem cell transplantation;
[6] the agent of any one of [1] to [5], wherein the agent is used after hematopoietic stem cell transplantation;
[7] the agent of [5] or [6], wherein the hematopoietic stem cell transplantation is selected from bone marrow transplantation, peripheral blood stem cell transplantation, and cord blood transplantation;
[8] the agent of [7], wherein the cord blood transplantation is human cord blood transplantation;

[9] the agent of [8], wherein the agent is administered more than once;
[10] an agent for promoting the growth of lymphoid cells and/or myeloid cells, wherein the agent comprises an agonist for the TPO receptor (c-mpl) as an active ingredient;
[11] an agent for promoting differentiation into lymphoid cells and/or myeloid cells, wherein the agent comprises an agonist for the TPO receptor (c-mpl) as an active ingredient;
[12] the agent of [5], wherein the hematopoietic stem cell transplantation is performed for a patient with impaired hematopoietic function of the bone marrow;
[13] the agent of [12], wherein the patient has been treated with radiotherapy or chemotherapy;
[14] the agent of [13], wherein the radiotherapy or chemotherapy is performed to treat acute myeloid leukemia (AML), acute lymphoblastic leukemia (ALL), chronic myeloid leukemia (CML), malignant lymphoma, adult T-cell leukemia, myelodysplastic syndrome (MDS), aplastic anemia (AA), or other diseases to which hematopoietic stem cell transplantation is applicable;
[15] a method for promoting the growth of hematopoietic stem cells, wherein the method comprises the step of administering an agonist for the TPO receptor (c-mpl) to a subject;
[16] a method for promoting the growth and/or differentiation of CD34-positive hematopoietic cells, wherein the method comprises the step of administering an agonist for the TPO receptor (c-mpl) to a subject;
[17] a method for enhancing the engraftment of transplanted CD34-positive hematopoietic cells in the bone marrow, wherein the method comprises the step of administering an agonist for the TPO receptor (c-mpl) to a subject;
[18] a method for promoting the recovery of hematopoiesis, wherein the method comprises the step of administering an agonist for the TPO receptor (c-mpl) to a subject;
[19] the method of any one of [15] to [18], wherein the method is performed for hematopoietic stem cell transplantation;
[20] the method of any one of [15] to [19], wherein the method is performed after hematopoietic stem cell transplantation;
[21] the method of [19] or [20], wherein the hematopoietic stem cell transplantation is selected from bone marrow transplantation, peripheral blood stem cell transplantation, and cord blood transplantation;
[22] the method of [21], wherein the cord blood transplantation is human cord blood transplantation;
[23] the method of [22], wherein the administration is carried out more than once;
[24] a method for proliferating lymphoid cells and/or myeloid cells, wherein the method comprises the step of administering an agonist for the TPO receptor (c-mpl) to a subject;
[25] a method for promoting differentiation into lymphoid cells and/or myeloid cells, wherein the method comprises the step of administering an agonist for the TPO receptor (c-mpl) to a subject;
[26] the method of [19], wherein the hematopoietic stem cell transplantation is performed for a patient with impaired hematopoietic function of the bone marrow;
[27] the method of [26], wherein the patient has been treated with radiotherapy or chemotherapy;
[28] the method of [27], wherein the radiotherapy or chemotherapy is performed to treat acute myeloid leukemia (AML), acute lymphoblastic leukemia (ALL), chronic myeloid leukemia (CML), malignant lymphoma, adult T-cell leukemia, myelodysplastic syndrome (MDS), aplastic anemia (AA), or other diseases to which hematopoietic stem cell transplantation is applicable;
[29] use of an agonist for the TPO receptor (c-mpl) in the production of an agent for promoting the growth of hematopoietic stem cells;
[30] use of an agonist for the TPO receptor (c-mpl) in the production of an agent for promoting the growth and/or differentiation of CD34-positive hematopoietic cells;
[31] use of an agonist for the TPO receptor (c-mpl) in the production of an agent for enhancing the engraftment of transplanted CD34-positive hematopoietic cells in the bone marrow;
[32] use of an agonist for the TPO receptor (c-mpl) in the production of an agent for promoting the recovery of hematopoiesis;
[33] use of an agonist for the TPO receptor (c-mpl) in the production of an agent for promoting the growth of hematopoietic stem cells, an agent for promoting the growth and/or differentiation of CD34-positive hematopoietic cells, an agent for enhancing the engraftment of transplanted CD34-positive hematopoietic cells in the bone marrow, or an agent for promoting the recovery of hematopoiesis, wherein the agent is used for hematopoietic stem cell transplantation;
[34] use of an agonist for the TPO receptor (c-mpl) in the production of an agent for promoting the growth of hematopoietic stem cells, an agent for promoting the growth and/or differentiation of CD34-positive hematopoietic cells, an agent for enhancing the engraftment of transplanted CD34-positive hematopoietic cells in the bone marrow, or an agent for promoting the recovery of hematopoiesis, wherein the agent is administered after hematopoietic stem cell transplantation;
[35] the use of [33] or [34], wherein the hematopoietic stem cell transplantation is selected from bone marrow transplantation, peripheral blood stem cell transplantation, and cord blood transplantation;

[36] the use of [35], wherein the cord blood transplantation is human cord blood transplantation;

[37] the use of [36], wherein the administration is carried out more than once;

[38] use of an agonist for the TPO receptor (c-mpl) in the production of an agent for promoting the growth of lymphoid cells and/or myeloid cells;

[39] use of an agonist for the TPO receptor (c-mpl) in the production of an agent for promoting the differentiation into lymphoid cells and/or myeloid cells;

[40] the use of [33], wherein the hematopoietic stem cell transplantation is performed for a patient with impaired hematopoietic function of the bone marrow;

[41] the use of [40], wherein the patient has been treated with radiotherapy or chemotherapy; and

[42] the use of [41], wherein the radiotherapy or chemotherapy is performed to treat acute myeloid leukemia (AML), acute lymphoblastic leukemia (ALL), chronic myeloid leukemia (CML), malignant lymphoma, adult T-cell leukemia, myelodysplastic syndrome (MDS), aplastic anemia (AA), or other diseases to which hematopoietic stem cell transplantation is applicable.

Brief Description of the Drawings

Fig. 1 presents diagrams showing the effect of sc(Fv)2 (hVB22 u2-wz4) on the number of different human blood cell lineages. Mean values are indicated by bars; SAS ver. 5.0 wilcoxon test.

Fig. 2 presents a diagram and a photograph showing the effect of sc(Fv)2 (hVB22 u2-wz4) on the number of CFU-Meg colonies. Mean values are indicated by bars; SAS ver. 5.0 wilcoxon test.

Fig. 3 presents diagrams showing the dose-dependent effect of sc(Fv)2 (hVB22 u2-wz4) on the number of different human blood cell lineages (mean (thick bar) + SD; Jonckheere-Terpstra test). In the diagrams, MAB(H), MAB(M), and MAB(L) indicate groups of high dose, medium dose, and low dose of hVB22sc(Fv)2, respectively.

Mode for Carrying Out the Invention

The present invention provides agents for promoting the growth of hematopoietic stem cells, agents for promoting the growth and/or differentiation of CD34-positive hematopoietic cells, agents for enhancing the engraftment of transplanted CD34-positive hematopoietic cells in the bone marrow, and agents for promoting the recovery of hematopoiesis, wherein the agents comprise an agonist for the TPO receptor (c-mpl) agonist as an active ingredient. Hereinafter, these agents are sometimes collectively referred to as "agents of the present invention".

The present invention provides agents which comprise an agonist for the TPO receptor (c-mpl) as an active ingredient for promoting the growth of hematopoietic stem cells. Herein, "agonist" refers to a substance that acts on a receptor, and exerts a function similar to that of a neurotransmitter, hormone, or such. The agonists of the present invention include, but are not limited to, low-molecular-weight compounds and antibodies. The antibodies of the present invention include any type of antibodies, including antibodies with altered amino acid sequences, such as minibodies, humanized antibodies, and chimeric antibodies; modified antibodies to which other molecules (for example, polymers such as polyethylene glycol) are linked; and antibodies with altered sugar chains, etc.

Herein, "hematopoietic stem cells" refers to cells that can differentiate into any type of lymphoid cells or myeloid cells. There is no particular limitation on the hematopoietic stem cells of the present invention, as long as they have the characteristics described above; however, the cells include CD34-positive hematopoietic cells. The CD34-positive hematopoietic cells are a heterogeneous cell population containing CD34-positive hematopoietic stem cells and CD34-positive hematopoietic precursor cells. The CD34-positive hematopoietic cells include, for example, multipotent stem cells, lymphoid stem cells, CFU-GEMM, CFU-GM, BFU-E, and CFU-MECz Herein, "CD34-positive hematopoietic precursor cells" refers to CD34-expressing cells that are in the process of differentiation into lymphoid cells (B cells, T cells, and such) or myeloid cells (neutrophils, monocytes, erythrocytes, megakaryocytes, and such), but have yet been directed to differentiate into each lineage, or are at a stage where the direction of cell differentiation cannot be identified morphologically. Whether or not cells express CD34 can be assessed by methods known to those skilled in the art, for example, the method described in the Journal of Hematotherapy 5, 213-226, 1996 (Robert Sutherland et al. The ISHAGE guidelines for CD34 cell determination by Flow Cytometry).

Herein, "promotion of growth" means that the growth of hematopoietic stem cells or CD34-positive hematopoietic cells (CD34-positive hematopoietic stem cells and CD34-positive hematopoietic precursor cells) is activated as compared to before administration of the agents of the present invention. Whether or not the growth of hematopoietic stem cells or CD34-positive hematopoietic cells (CD34-positive hematopoietic stem cells and CD34-positive hematopoietic precursor cells) is activated can be assessed by methods conventionally performed by those skilled in the art, for example, by detecting a change in the growth rate of hematopoietic stem cells or CD34-positive hematopoietic cells (CD34-positive hematopoietic stem cells and CD34-positive hematopoietic precursor cells), a change in the number (of colonies) of hematopoietic stem cells or CD34-positive hematopoietic cells (CD34-positive hematopoietic stem cells and CD34-positive hematopoietic precursor cells), a change in intracellular signaling involved in the growth of hematopoietic stem cells or CD34-positive hematopoietic cells (CD34-positive hematopoietic stem cells and CD34-positive hematopoietic precursor cells), or such.

Furthermore, the present invention provides agents which comprise an agonist for the TPO receptor (c-mpl) as an active ingredient for promoting the growth and/or differentiation of CD34-positive hematopoietic cells. Herein, "differentiation of CD34-positive hematopoietic cells" refers to a process in which CD34-positive hematopoietic cells that could differentiate into any type of lymphoid cells or myeloid cells break from this state, and are determined to differentiate into a particular cell type, or a process in which the cells differentiate into the determined cell type. The cell types into which the CD34-positive hematopoietic cells (CD34-positive hematopoietic stem cells and CD34-positive hematopoietic precursor cells) differentiate include, but are not particularly limited to, lymphoid cells such as T cells, B cells, and NK cells, and myeloid cells such as erythrocytes, leukocytes, platelets, neutrophils, monocytes, and eosinophils.

Herein, "promotion of differentiation" means that the differentiation is activated as compared to before administration of the agents of the present invention. Whether or not the differentiation of CD34-positive hematopoietic stem cells is activated can also be assessed by methods known to those skilled in the art, for example, by detecting a change in the growth rate of cells, a change in the number of cells, a change in the intensity of intracellular signaling involved in differentiation, a differentiation marker, a change in cell morphology, or such.

Engraftment of transplanted hematopoietic stem cells in the bone marrow is required in hematopoietic stem cell transplantation. The present invention provides agents which comprise an agonist for the TPO receptor (c-mpl) as an active ingredient for enhancing the engraftment of transplanted CD34-positive hematopoietic cells in the bone marrow. The engraftment of transplanted CD34-positive hematopoietic cells (CD34-positive hematopoietic stem cells and CD34-positive hematopoietic precursor cells) in the bone marrow can be promoted by administering the agents of the present invention. The presence or absence, or the degree of engraftment of transplanted CD34-positive hematopoietic cells in the bone marrow (the engraftment rate of transplanted CD34-positive hematopoietic cells in the bone marrow) can be assessed, for example, by determining the absolute number of human CD34-positive hematopoietic cells in the bone marrow of NOD/SCID mice to which human hematopoietic cells are transplanted. Human cells can be identified among the mouse bone marrow cells by using a fluorescent-labeled, human CD34-specific antibody for detection. Specifically, the engraftment rate of transplanted CD34-positive hematopoietic cells in the bone marrow can be determined by the following procedures. First, NOD.CB 17-Prkdc<scid>/J mice, to which human cord blood-derived CD34-positive cells have been transplanted, are euthanized three weeks after transplantation. The right and left femurs are collected, and then their epiphyseal portions are removed by scissors. Bone marrow cells are flushed out using a 26G-needle syringe and collected. After washing with IMDM containing 2% FBS, 5 ml of erythrocyte lysis solution is added, and the mixture is allowed to stand for five minutes. The mixture is centrifuged and supernatant is removed. Then, the precipitate is suspended in 2 ml of IMDM containing 2% FBS. The suspension is filtered through a 70-μm membrane to prepare a bone marrow cell suspension (2 ml/two femurs for each mouse). The cell suspension is aliquoted into 100-μl samples, and the cells are stained for 15 minutes with a fluorescent-labeled, human CD34-specific antibody and PI (final concentration: 2 μg/ml). After washing, 50 μl of Flow-Count fluorescent particles are added, and the engraftment number of human CD34-positive cells is determined using the EPICS XL cell analyzer. The determination may be performed, for example, according to Application Note 5: "Determination of the absolute number of cells using Flow-Count" (Beckman Coulter, Inc.). Likewise, the absolute number of different human blood cell lineages in the bone marrow can be determined using a variety of detection antibodies. The absolute number of human cells contained in the two femurs is represented by the following formula:

Absolute number of human cells = determined value (cells/μl) x 1/2 (Flow-Count amount added (50)/sample amount added (100)) x 2000 (2 ml/two femurs/mouse)

(Barnett D, Granger V, Whitby L, Storie I, Reilly JT. Absolute CD4+ T-lymphocyte and CD34+ stem cell counts by single-platform flow cytometry: the way forward. Br. J. Haematol., Sep;106(4) :1059-62 (1999))

Furthermore, the engraftment rate of transplanted CD34-positive hematopoietic cells in the bone marrow of human subjects who received hematopoietic stem cell transplantation can also be determined, for example, indirectly by monitoring the recovery of hematocytes in peripheral blood, instead of using the methods described above.

The present invention also provides agents which comprise an agonist for the TPO receptor (c-mpl) as an active ingredient for promoting recovery of the hematopoiesis. It generally takes one to three weeks after transplantation

for hematopoietic stem cells to engraft and leukocytes to recover. Since there are very few leukocytes in the blood during this period, the problem is susceptibility to infection by bacteria or fungi, such as pneumonia. Furthermore, it generally takes two to ten weeks after transplantation for hematopoietic stem cells to engraft and platelets to recover. Since there are very few platelets in the blood during this period, the problem is susceptibility to bleeding. Problems regarding the delay of hematopoiesis after transplantation such as these can be solved by using the present invention's agents for promoting the recovery of hematopoiesis. Herein, "promotion of the recovery of hematopoiesis" means that hematopoiesis in the bone marrow is activated as compared to before administration of the agents of the present invention. Whether or not hematopoiesis in the bone marrow is activated can be assessed by monitoring the hematocyte recovery in peripheral blood.

Furthermore, the present invention also provides agents which comprise an agonist for the TPO receptor (c-mpl) as an active ingredient for promoting growth of lymphoid cells and/or myeloid cells. The present inventors discovered that as a result of the increased engraftment of transplanted CD34-positive hematopoietic cells in the bone marrow by an agonist for the TPO receptor (c-mpl), the cells of both lymphocytic and myelocytic lineages increased (see the Examples). The agents of the present invention for promoting the growth of lymphoid cells and/or myeloid cells are based on the above findings. The lymphoid cells of the present invention include, but are not limited to, T cells, B cells, and NK cells. On the other hand, the myeloid cells include, but are not limited to, erythrocytes, leukocytes, platelets, neutrophils, monocytes, and eosinophils.

The present inventors discovered that an agonist for the TPO receptor (c-mpl) activated the differentiation of CD34-positive hematopoietic cells (CD34-positive hematopoietic stem cells and CD34-positive hematopoietic precursor cells). Thus, the present invention provides agents which comprise an agonist for the TPO receptor (c-mpl) as an active ingredient for promoting differentiation into lymphoid cells and/or myeloid cells. Herein, the cells which CD34-positive hematopoietic cells (CD34-positive hematopoietic stem cells and CD34-positive hematopoietic precursor cells) differentiate into include lymphoid cells such as T cells, B cells, and NK cells, and myeloid cells such as erythrocytes, leukocytes, platelets, neutrophils, monocytes, and eosinophils.

The agents of the present invention are useful in promoting the growth of hematopoietic stem cells, promoting the growth and/or differentiation of CD34-positive hematopoietic stem cells, enhancing the engraftment of transplanted CD34-positive hematopoietic stem cells in the bone marrow, or promoting the recovery of hematopoiesis, in hematopoietic stem cell transplantation performed to treat acute myeloid leukemia, chronic myeloid leukemia, myelodysplastic syndrome, acute lymphoblastic leukemia, adult T-cell leukemia, aplastic anemia, malignant lymphoma, and other diseases to which hematopoietic stem cell transplantation is applicable.

The hematopoietic stem cell transplantation of the present invention includes bone marrow transplantation, peripheral blood stem cell transplantation, and cord blood transplantation. When transplantation is performed to treat leukemia or such, in general, peripheral blood stem cell transplantation and cord blood transplantation are widely carried out in addition to bone marrow transplantation.

c-mpl is a TPO receptor. The gene sequence of human c-mpl has been analyzed (Palacios et al., Cell Vol. 41, p. 727-734 (1985); Genbank: NM_005373). Furthermore, the sequences of cynomolgus monkey c-mpl (nucleotide/SEQ ID NO: 52; amino acid/SEQ ID NO: 53) and mouse c-mpl (GenBank #NM_010823) are also known. The amino acid sequence of human c-mpl is shown in SEQ ID NO: 51.

Furthermore, c-mpl of the present invention includes c-mpl receptor mutants with an amino acid substitution, deletion, addition, or such, in c-mpl described above. Specifically, the c-mpl mutants include, for example, the c-mpl mutants described in Matthias Ballmaier et al., BLOOD, Vol. 97, No. 1, p. 139 (2001).

In the present invention, there is no limitation on the agonist for c-mpl, as long as it has the activity to promote the growth of hematopoietic stem cells or the growth and/or differentiation of CD34-positive hematopoietic cells, to enhance the engraftment of transplanted CD34-positive hematopoietic cells in the bone marrow, or to promote the recovery of hematopoiesis. Whether or not a candidate compound has such activity can be confirmed by methods known to those skilled in the art.

"Agonistic activity for c-mpl" refers to the activity to promote the growth of hematopoietic stem cells or the growth and/or differentiation of CD34-positive hematopoietic cells, to enhance the engraftment of transplanted CD34-positive hematopoietic cells in the bone marrow, or to promote the recovery of hematopoiesis. Determination of the agonistic activity can be performed by methods known to those skilled in the art. The agonistic activity may be determined using not only the activity of the agonist itself, but also a different activity as an indicator.

For example, the agonistic activity can be determined using cell growth as an indicator. More specifically, an antibody whose agonistic activity is to be assessed is added to cells that proliferate in an agonist-dependent manner, and the cells are cultured. Subsequently, the agonistic activity can be determined by counting the cells using a hemocytometer, or measuring the cell number using a flow cytometer. Alternatively, the agonistic activity can be determined by the following method or such. A reagent such as the tetrazolium salt WST-8 (Dojindo Laboratories), which shows a coloring reaction at a specific wavelength according to the number of viable cells, is added to cells, and the resulting absorbance is used as an indicator.

Cells that proliferate in an agonist-dependent manner can be prepared by methods known to those skilled in the art. For example, when a receptor transduces cell growth signals, cells expressing the receptor may be used. Alternatively, when a receptor does not transduce cell growth signals, a chimeric receptor consisting of the intracellular domain of a receptor capable of transducing cell growth signals and the extracellular domain of a receptor incapable of transducing cell growth signals is prepared, and the chimeric receptor may be expressed in cells. Receptors that transduce cell growth signals include, for example, the G-CSF receptors, mpl, neu, GM-CSF receptors, EPO receptors, c-kit, FLT-3. Cells for expressing the receptor include, for example, BaF3, NFS60, FDCP-1, FDCP-2, CTLL-2, DA-1, KT-3.

Any detection indicator can be used for determining the agonistic activity, as long as the indicator allows monitoring of quantitative and/or qualitative changes. For example, it is possible to use cell-free assay indicators, cell-based assay indicators, tissue-based assay indicators, and *in vivo* assay indicators. Enzymatic reactions, or quantitative and/or qualitative changes in proteins, DNAs, or RNAs can be used as indicators in cell-free assays. Such enzymatic reactions include, for example, amino acid transfer reactions, sugar transfer reactions, dehydration reactions, dehydrogenation reactions, and substrate cleavage reactions. Alternatively, phosphorylation, dephosphorylation, dimerization, multimerization, degradation, and dissociations of proteins, and such; and amplification, cleavage, and extension of DNAs and RNAs can be used. For example, phosphorylation of a protein placed downstream of a signaling pathway may be used as a detection indicator. Changes in cell phenotype, for example, quantitative and/or qualitative changes in products, change in growth activity, change in cell number, change in morphology, change in cellular properties, or such, can be used as indicators in cell-based assays. The products include, for example, secretory proteins, surface antigens, intracellular proteins, and mRNAs. Changes in morphology include, for example, change in protrusion formation and/or protrusion number, change in cell flatness, change in the degree of cell elongation/horizontal to vertical ratio, change in cell size, change in intracellular structures, heterogeneity/homogeneity of cell populations, and change in cell density. Such morphological changes can be confirmed by observation under a microscope. Cellular properties that can be used as indicators include anchorage dependency, cytokine-dependent responses, hormone dependency, drug resistance, cell motility, cell migration activity, pulsatility, and changes in intracellular substances. Cell motility includes cell infiltration activity and cell migration activity. Changes in intracellular substances include, for example, change in enzyme activity, mRNA levels, levels of intracellular signaling molecules such as $Ca^{2+}$ and cAMP, and intracellular protein levels. When a cell-membrane receptor is used, changes in the cell proliferation activity induced by stimulation of the receptor can be used as an indicator. Functional change of the tissue to be used can be detected as an indicator in tissue-based assays. Changes in tissue weight; changes in the blood system, for example, changes in blood cell number, protein levels, enzyme activity, or electrolyte levels; and changes in the circulating system, for example, changes in blood pressure or heart rate, and such, can be used as *in vivo* assay indicators.

There is no particular limitation on the methods for measuring such detection indicators. For example, it is possible to use absorbance, luminescence, color development, fluorescence, radioactivity, fluorescence polarization, surface plasmon resonance signal, time-resolved fluorescence, mass, absorption spectrum, light scattering, and fluorescence resonance energy transfer, etc. These measurement methods are well-known to those skilled in the art, and may be appropriately selected according to the purpose. For example, absorption spectra can be obtained by using a conventional photometer, plate reader, or such. Luminescence can be measured with a luminometer or such, and fluorescence can be measured with a fluorometer or such. Mass can be determined with a mass spectrometer. Radioactivity can be determined with a device such as a gamma counter according to the type of radiation. Fluorescence polarization can be measured with BEACON (TaKaRa Shuzo), etc. Surface plasmon resonance signals can be measured with BIAcore, etc. Time-resolved fluorescence, fluorescence resonance energy transfer, or such, can be measured with ARVO, etc. Alternatively, such measurements can be performed using a flow cytometer. It is possible to use one of the above methods to measure two or more detection indicators. If convenient, a number of detection indicators may also be measured by using two or more of the above methods simultaneously and/or consecutively. For example, fluorescence and fluorescence resonance energy transfer can be measured at the same time using a fluorometer.

The agonists of the present invention may be natural or artificial compounds. The agonists used in the present invention may be known compounds. Alternatively, it is possible to use novel compounds that have been assessed to have the agonistic activity by the methods described above.

In a preferred embodiment, the antibodies of the present invention comprise, for example, minibodies. The minibodies comprise antibody fragments lacking portions of the whole antibody (for example, whole IgG). The minibodies are not particularly limited as long as they have binding activity to their antigens. The minibodies of the present invention have significantly higher activities compared to their corresponding whole antibodies. There are no particular limitations on the antibody fragments of the present invention as long as they are portions of the whole antibody, and preferably contain heavy chain variable regions (VH) and/or light chain variable regions (VL). The amino acid sequences of VH or VL may contain substitutions, deletions, additions and/or insertions. Furthermore, the antibody fragment may also lack portions of VH or/and VL, as long as it has binding ability to its antigen. In addition, the variable regions may be chimerized or humanized. Such antibody fragments include, for example, Fab, Fab', $F(ab')_2$, and Fv. An example of a minibody includes Fab, Fab', $F(ab')_2$, Fv, scFv (single-chain Fv), diabody, and sc(Fv)2 (single-chain (Fv)2).

Herein, an "Fv" fragment is the smallest antibody fragment and contains a complete antigen recognition site and a binding site. The "Fv" fragment is a dimer (VH-VL dimer) in which a single VH and a single VL are strongly linked by a non-covalent bond. The three complementarity-detennining regions (CDRs) of each of the variable regions interact with each other to form an antigen-binding site on the surface of the VH-VL dimer. Six CDRs confer the antigen-binding site of an antibody. However, a single variable region (or a half of Fv containing only three CDRs specific to an antigen) alone is also capable of recognizing and binding an antigen although its affinity is lower than the affinity of the entire binding site.

scFv contains the VH and VL regions of an antibody, and these regions exist on a single polypeptide chain. Generally, an Fv polypeptide further contains a polypeptide linker between VH and VL, and therefore an scFv can form a structure required for antigen binding. See, Pluckthun "The Pharmacology of Monoclonal Antibodies" Vol. 113 (Rosenburg and Moore eds. (Springer Verlag, New York, pp.269-315, 1994) for the review of scFv. In the present invention, linkers are not especially limited as long as they do not inhibit expression of antibody variable regions linked at both ends of the linkers.

The term "diabody" refers to a bivalent antibody fragment constructed by gene fusion (Holliger P et al. , 1993, Proc. Natal. Acad. Sci. USA 90: 6444-6448; EP 404,097; WO 93/11161 and others). Diabodies are dimers comprising two polypeptide chains, where each polypeptide chain comprises a VL and a VH connected with a linker short enough to prevent interaction of these two domains, for example, a linker of about five residues. The VL and VH encoded on the same polypeptide chain will form a dimer because the linker between them is too short to form a single-chain variable region fragment. As a result, the polypeptide chains form a dimer, and thus the diabody has two antigen binding sites.

In a particularly preferred embodiment, the c-mpl-recognizing antibodies comprised in the agents of the present invention include sc(Fv)2. The present inventors discovered that sc(Fv)2 is a single-chain minibody produced by linking two units ofVH and two units of VL with linkers and such (Hudson et al., 1999, J Immunol. Methods 231:177-189). sc (Fv)2 exhibits a particularly high agonistic activity compared to the whole antibody and other minibodies. sc(Fv)2 can be produced, for example, by linking two scFv molecules.

In a preferable antibody, the two VH units and two VL units are arranged in the order of VH, VL, VH, and VL ([VH]-linker-[VL]-linker-[VH]-linker-[VL]) beginning from the N terminus of a single-chain polypeptide.

The order of the two VH units and two VL units is not limited to the above arrangement, and they may be arranged in any order. Examples of the arrangements are listed below.

[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

The linkers to be used for linking the variable regions of an antibody comprise arbitrary peptide linkers that can be introduced by genetic engineering, synthetic linkers, and linkers disclosed in, for example, Holliger, P. et al., Protein Engineering, 9(3), 299-305, 1996. Peptide linkers are preferred in the present invention. There are no limitations as to the length of the peptide linkers. The length can be selected accordingly by those skilled in the art depending on the purpose, and is typically 1-100 amino acids, preferably 3-50 amino acids, more preferably 5-30 amino acids, and even more preferably 12-18 amino acids (for example, 15 amino acids).

For example, such peptide linkers include:

Ser
Gly·Ser
Gly·Gly·Ser
Ser·Gly·Gly
Gly·Gly·Gly·Ser (SEQ ID NO: 77)
Ser·Gly·Gly·Gly (SEQ ID NO: 78)
Gly·Gly·Gly·Gly·Ser (SEQ ID NO: 79)
Ser·Gly·Gly·Gly·Gly (SEQ ID NO: 80)
Gly·Gly·Gly·Gly·Gly·Ser (SEQ ID NO: 81)
Ser·Gly·Gly·Gly·Gly·Gly (SEQ ID NO: 82)
Gly·Gly·Gly·Gly·Gly·Gly·Ser (SEQ ID NO: 83)
Ser·Gly·Gly·Gly·Gly·Gly·Gly (SEQ ID NO: 84)
(Gly·Gly·Gly·Gly·Ser (SEQ ID NO: 79))$_n$
(Ser·Gly·Gly·Gly·Gly (SEQ ID NO: 80))$_n$

where n is an integer of 1 or larger. The lengths and sequences of peptide linkers can be selected accordingly by those skilled in the art depending on the purpose.

In an embodiment of the present invention, a particularly preferable sc(Fv)2 includes, for example, the sc(Fv) 2 below.

[VH]-peptide linker (15 amino acids)-[VL]-peptide linker (15 amino acids)-[VH]-peptide linker (15 amino acids)-[VL]

Synthetic linkers (chemical crosslinking agents) include crosslinking agents routinely used to crosslink peptides, for example, N-hydroxy succinimide (NHS), disuccinimidyl suberate (DSS), bis(succinimidyl) suberate ($BS^3$), dithiobis (succinimidyl propionate) (DSP), dithiobis(succinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl] sulfone (BSOCOES), and bis[2-(succinimidoxycarbonyloxy) ethyl] sulfone (sulfo-BSOCOES). These crosslinking agents are commercially available.

In general, three linkers are required to link four antibody variable regions together. The linkers to be used may be of the same type or different types. In the present invention, a preferable minibody is a diabody, even more preferably, an sc(Fv)2. Such a minibody can be prepared by treating an antibody with an enzyme, for example, papain or pepsin, to generate antibody fragments, or by constructing DNAs encoding those antibody fragments and introducing them into expression vectors, followed by expression in an appropriate host cell (see, for example, Co, M. S. et al., 1994, J. Immunol. 152, 2968-2976; Better, M. and Horwitz, A. H., 1989, Methods Enzymol. 178, 476-496; Pluckthun, A. and Skerra, A., 1989, Methods Enzymol. 178, 497-515; Lamoyi, E., 1986, Methods Enzymol. 121, 652-663; Rousseaux, J. et al., 1986, Methods Enzymol. 121, 663-669; Bird, R. E. and Walker, B. W., 1991, Trends Biotechnol. 9, 132-137).

Since sc(Fv)2 antibodies against c-mpl have an especially high agonistic activity for c-mpl, they are particularly useful as agents for promoting the growth of hematopoietic stem cells, agents for promoting the growth and/or differentiation of CD34-positive hematopoietic cells, agents for enhancing the engraftment of transplanted CD34-positive hematopoietic cells in the bone marrow, or agents for promoting the recovery of hematopoiesis in hematopoietic stem cell transplantation.

In a preferred embodiment, the c-mpl-recognizing antibodies comprised in the agents of the present invention include modified antibodies such as chimeric antibodies, humanized antibodies. Humanized antibodies are particularly preferred.

Chimeric antibodies are antibodies prepared by combining sequences derived from different animal species, and include for example, antibodies comprising the heavy chain and light chain variable regions of a murine antibody, and the heavy chain and light chain constant regions of a human antibody. Chimeric antibodies can be prepared by known methods. For example, a DNA encoding the V region of an antibody is linked to a DNA encoding the C region of a human antibody, and the construct is inserted into an expression vector and introduced into a host to produce chimeric antibodies.

Humanized antibodies are also referred to as "reshaped human antibodies". Such a humanized antibody is obtained by transferring the complementarity-determining region (CDR) of an antibody derived from a non-human mammal, for example mouse, to the complementarity-determining region of a human antibody, and the general gene recombination procedure for this is also known (see European Patent Application No. 125023 and WO 96/02576).

Specifically, a DNA sequence designed to link a murine antibody CDR to the framework region (FR) of a human antibody can be synthesized by PCR, using primers prepared from several oligonucleotides containing overlapping portions of both CDR and FR terminal regions (see methods described in WO 98/13388).

The human antibody framework region to be linked by CDR is selected in order to form a favorable antigen-binding site in the complementarity-determining region. Amino acids of the framework region in the antibody variable region may be substituted, as necessary, for the complementarity-determining region of the reshaped human antibody to form a suitable antigen-binding site (Sato, K. et al., 1993, Cancer Res. 53, 851-856).

The constant region of a human antibody is used as the constant region of a chimeric antibody or humanized antibody. For example, Cγ1, Cγ2, Cγ3, and Cγ4 can be used as the H chain, and Cκ and Cλ an be used as the L chain. The human antibody constant region may be modified to improve the antibody or the stability of the antibody production.

Generally, chimeric antibodies comprise the variable region of an antibody from a non-human mammal and the constant region derived from a human antibody. On the other hand, humanized antibodies comprise the complementarity-determining region of an antibody from a non-human mammal, and the framework region and consort region derived from a human antibody.

In addition, after a chimeric antibody or a humanized antibody is prepared, amino acids in the variable region (for example, FR) and the constant region may be replaced with other amino acids, and such. The origin of the variable regions in chimeric antibodies or that of the CDRs in humanized antibodies is not particularly limited, and may be derived from any type of animal. For example, sequences of murine antibodies, rat antibodies, rabbit antibodies, camel antibodies may be used.

Humanized antibodies that recognize c-mpl include, for example, humanized antibodies indicated in (9) to (19) below.

Chimeric antibodies and humanized antibodies have lower antigenicity in the human body, and are thus particularly useful when administered to human. The antibodies are particularly useful as agents for promoting the growth of hematopoietic stem cells, agents for promoting the growth and/or differentiation of CD34-positive hematopoietic cells, agents for enhancing the engraftment of transplanted CD34-positive hematopoietic cells in the bone marrow, or agents for promoting the recovery of hematopoiesis in hematopoietic stem cell transplantation.

In one embodiment, the preferred c-mpl-recognizing antibodies that are comprised in the agents of the present invention include antibodies that bind to soluble c-mpl. The term "soluble c-mpl" herein refers to c-mpl molecules excluding those expressed on the cell membrane. A specific example of a soluble c-mpl is a c-mpl lacking the entire or a portion of the transmembrane domain. The transmembrane domain of human c-mpl corresponds to amino acids 492-513 in SEQ ID NO: 51.

An antibody that binds to soluble recombinant c-mpl can be used in detailed epitope analysis and kinetic analysis of receptor-ligand binding, as well as for assessing the blood concentration and dynamic behavior of the antibody in *in vivo* tests.

In one embodiment, the preferred antibodies recognizing c-mpl that are comprised in the agents of the present invention include antibodies having binding activity or agonistic activity for both human and monkey c-mpl. Antibodies having agonistic activity to both human and monkey c-mpl are expected to be highly useful since the dynamic behavior and *in vivo* effects of the antibody, which are generally difficult to determine in human body, can be examined with monkeys. Such antibodies may also have binding activity or agonistic activity for c-mpl from animals other than humans and monkeys (for example, mice).

In another embodiment, the preferred c-mpl-recognizing antibodies comprised in the agents of the present invention include antibodies with TPO agonistic activity (agonistic activity for c-mpl) of EC50 = 100 nM or lower, preferably EC50 = 30 nM or lower, more preferably EC50 = 10 nM or lower.

In another embodiment, the preferred c-mpl-recognizing antibodies comprised in the agents of the present invention include antibodies whose binding activity to soluble c-mpl is KD = $10^{-6}$ M or lower, preferably KD = $10^{-7}$ M or lower, and more preferably KD = $10^{-8}$ M or lower.

In the present invention, whether the binding activity of an antibody to soluble recombinant c-mpl is KD = $10^{-6}$ M or lower can be determined by methods known to those skilled in the art. For example, the activity can be determined using surface plasmon resonance with BIAcore. Specifically, soluble c-mpl-Fc protein is immobilized onto sensor chips, and reaction rate constant can be determined by assessing the interaction between the antibody and the soluble c-mpl-Fc protein. The binding activity can be evaluated by ELISA (enzyme-linked immunosorbent assays), EIA (enzyme immunoassays), RIA (radio immunoassays), or fluorescent antibody techniques. For example, in enzyme immunoassays, a sample containing a test antibody, such as purified antibody or culture supernatant of cells producing the test antibody, is added to a plate coated with an antigen to which the test antibody can bind. After incubating the plate with a secondary antibody labeled with an enzyme such as alkaline phosphatase, the plate is washed and an enzyme substrate such as p-nitrophenyl phosphate is added. The antigen-binding activity can then be evaluated by determining the absorbance.

There is no specific limitation as to the upper limit of the binding activity; for example, the upper limit may be set within a technically feasible range by those skilled in the art. However, the technically feasible range may expand with the advancement of technology.

In an embodiment, the preferred antibodies recognizing c-mpl that are comprised in the agents of the present invention include any one of the antibodies indicated in (1) to (19) below. The antibody of any one of (1) to (19) is preferably a minibody.

(1) an antibody comprising a VH that has CDR1, 2, and 3 comprising the amino acid sequences of SEQ ID NOs: 1, 2, and 3 (VB22B: VH CDR1, 2, and 3), respectively.

(2) an antibody comprising a VL that has CDR1, 2, and 3 comprising the amino acid sequences of SEQ ID NOs: 4, 5, and 6 (VB22B: VL CDR1, 2, and 3), respectively.

(3) an antibody comprising a VH that has CDR1, 2, and 3 comprising the amino acid sequences of SEQ ID NOs: 1, 2, and 3 (VB22B: VH CDR1, 2, and 3), respectively, and a VL that has CDR1, 2, and 3 comprising the amino acid sequences of SEQ ID NOs: 4, 5, and 6 (VB22B: VL CDR1, 2, and 3), respectively.

(4) an antibody comprising a VH that comprises the amino acid sequence of SEQ ID NO: 8 (VB22B: VH).

(5) an antibody comprising a VL that comprises the amino acid sequence of SEQ ID NO: 10 (VB22B: VL).

(6) an antibody comprising a VH that comprises the amino acid sequence of SEQ ID NO: 8 (VB22B: VH) and a VL that comprises the amino acid sequence of SEQ ID NO: 10 (VB22B: VL).

(7) an antibody having the amino acid sequence of SEQ ID NO: 12 (VB22B: scFv).

(8) an antibody having the amino acid sequence of SEQ ID NO: 14 (VB22B: sc(Fv)2).

(9) a humanized antibody comprising a VH that has FR1, 2, 3, and 4 comprising the amino acid sequences of any

one of (a) to (e) below:

(a) SEQ ID NOs: 15, 16, 17, and 18 (hVB22B p-z: VH FR1, 2, 3, and 4), respectively;
(b) SEQ ID NOs: 19,20,21, and 22 (hVB22B g-e: VH FR1, 2, 3, and 4), respectively;
(c) SEQ ID NOs: 23, 24, 25, and 26 (hVB22B e: VH FR1, 2, 3, and 4), respectively;
(d) SEQ ID NOs: 54, 55, 56, and 57 (hVB22B u2-wz4: VH FR1, 2, 3, and 4), respectively;
(e) SEQ ID NOs: 54, 55, 58, and 57 (hVB22B q-wz5: VH FR1, 2, 3, and 4), respectively.

(10) a humanized antibody comprising a VL that has FR1, 2, 3, and 4 comprising the amino acid sequences of any one of (a) to (d) below:

(a) SEQ ID NOs: 27, 28, 29, and 30 (hVB22B p-z: VL FR1, 2, 3, and 4), respectively;
(b) SEQ ID NOs: 31, 32, 33, and 34 (hVB22B g-e or hVB22B e: VL FR1, 2, 3, and 4), respectively;
(c) SEQ ID NOs: 59, 60, 61, and 62 (hVB22B u2-wz4: VL FR1, 2, 3, and 4), respectively;
(d) SEQ ID NOs: 59, 63, 64, and 62 (hVB22B q-wz5: VL FR1, 2, 3, and 4), respectively.

(11) a humanized antibody comprising a VL and a VH described in any one of (a) to (e) below:

(a) a VH that has FR1, 2, 3, and 4 comprising the amino acid sequences of SEQ ID NOs: 15, 16, 17, and 18, respectively, and a VL that has FR1, 2, 3, and 4 comprising the amino acid sequences of SEQ ID NOs: 27, 28, 29, and 30, respectively;
(b) a VH that has FR1, 2, 3, and 4 comprising the amino acid sequences of SEQ ID NOs: 19, 20, 21, and 22, respectively, and a VL that has FR1, 2, 3, and 4 comprising the amino acid sequences of SEQ ID NOs: 31, 32, 33, and 34, respectively;
(c) a VH that has FR1, 2, 3, and 4 comprising the amino acid sequences of SEQ ID NOs: 23, 24, 25, and 26, respectively, and a VL that has FR1, 2, 3, and 4 comprising the amino acid sequences of SEQ ID NOs: 31, 32, 33, and 34, respectively;
(d) a VH that has FR1, 2, 3, and 4 comprising the amino acid sequences of SEQ ID NOs: 54, 55, 56, and 57, respectively, and a VL that has FR1, 2, 3, and 4 comprising the amino acid sequences of SEQ ID NOs: 59, 60, 61, and 62, respectively;
(e) a VH that has FR1, 2, 3, and 4 comprising the amino acid sequences of SEQ ID NOs: 54, 55, 58, and 57, respectively, and a VL that has FR1, 2, 3, and 4 comprising the amino acid sequences of SEQ ID NOs: 59, 63, 64, and 62, respectively.

(12) a humanized antibody comprising a VH that has CDR1, 2, and 3 comprising the amino acid sequences of SEQ ID NOs: 1, 2, and 3, respectively.
(13) a humanized antibody comprising a VL that has CDR1, 2, and 3 comprising the amino acid sequences of SEQ ID NOs: 4, 5, and 6, respectively.
(14) a humanized antibody comprising a VH that has CDR1, 2, and 3 comprising the amino acid sequences of SEQ ID NO: 1, 2, and 3, respectively, and a VL that has CDR1, 2, and 3 comprising the amino acid sequences of SEQ ID NO: 4, 5, and 6, respectively.
(15) a humanized antibody comprising a VH that comprises the amino acid sequence of SEQ ID NO: 36 (hVB22B p-z: VH), SEQ ID NO: 38 (hVB22B g-e: VH), SEQ ID NO: 40 (hVB22B e: VH), SEQ ID NO: 65 (hVB22B u2-wz4: VH), or SEQ ID NO: 66 (hVB22B q-wz5: VH).
(16) a humanized antibody comprising a VH that comprises the amino acid sequence of SEQ ID NO: 42 (hVB22B p-z: VL), SEQ ID NO: 44 (hVB22B g-e: VL or hVB22B e: VL), SEQ ID NO: 67 (hVB22B u2-wz4: VL), or SEQ ID NO: 68 (hVB22B q-wz5: VH).
(17) a humanized antibody comprising a VH and a VL described in any one of (a) to (e) below:

(a) a VH that comprises the amino acid sequence of SEQ ID NO: 36 (hVB22B p-z: VH), and a VL that comprises the amino acid sequence of SEQ ID NO: 42 (hVB22B p-z: VL);
(b) a VH that comprises the amino acid sequence of SEQ ID NO: 38 (hVB22B g-e: VH), and a VL that comprises the amino acid sequence of SEQ ID NO: 44 (hVB22B g-e: VL or hVB22B e: VL);
(c) a VH that comprises the amino acid sequence of SEQ ID NO: 40 (hVB22B e: VH), and a VL that comprises the amino acid sequence of SEQ ID NO: 44 (hVB22B g-e: VL or hVB22B e:VL);
(d) a VH that comprises the amino acid sequence of SEQ ID NO: 65 (hVB22B u2-wz4: VH), and a VL that comprises the amino acid sequence of SEQ ID NO: 67 (hVB22B u2-wz4: VL);
(e) a VH that comprises the amino acid sequence of SEQ ID NO: 66 (hVB22B q-wz5: VH), and a VL that

comprises the amino acid sequence of SEQ ID NO: 68 (hVB22B q-wz5:VL).

In the amino acid sequence of SEQ ID NO: 36 (hVB22B p-z: VH), SEQ ID NO: 38 (hVB22B g-e: VH), SEQ ID NO: 40 (hVB22B e: VH), SEQ ID NO: 65 (hVB22B u2-wz4: VH), or SEQ ID NO: 66 (hVB22B q-wz5: VH),

the region of amino acids 31 to 35 corresponds to CDR1;
the region of amino acids 50 to 66corresponds to CDR2;
the region of amino acids 99 to 107 corresponds to CDR3;
the region of amino acids 1 to 30 corresponds to FR1;
the region of amino acids 36 to 49 corresponds to FR2;
the region of amino acids 67 to 98 corresponds to FR3; and
the region of amino acids 108 to 118 corresponds to FR4.

In the amino acid sequence of SEQ ID NO: 42 (hVB22B p-z: VL), SEQ ID NO: 44 (hVB22B g-e: VL or hVB22B e: VL), SEQ ID NO: 67 (hVB22B u2-wz4: VL), or SEQ ID NO: 68 (hVB22B q-wz5: VH),

the region of amino acids 24 to 39 corresponds to CDR1;
the region of amino acids 55 to 61 corresponds to CDR2;
the region of amino acids 94 to 102 corresponds to CDR3;
the region of amino acids 1 to 23 corresponds to FR1;
the region of amino acids 40 to 54 corresponds to FR2;
the region of amino acids 62 to 93 corresponds to FR3; and
the region of amino acids 103 to 112 corresponds to FR4.

Herein, the correspondence between CDR and FR in the hVB22B p-z VH sequence and sequence ID numbers is as follows:

hVB22B p-z VH: FR1/SEQ ID NO: 15
hVB22B p-z VH: CDR1/SEQ ID NO: 1
hVB22B p-z VH: FR2/SEQ ID NO: 16
hVB22B p-z VH: CDR2/SEQ ID NO: 2
hVB22B p-z VH: FR3/SEQ ID NO: 17
hVB22B p-z VH: CDR3/SEQ ID NO: 3
hVB22B p-z VH: FR4/SEQ ID NO: 18.

Herein, the correspondence between CDR and FR in the hVB22B p-z VL sequence and sequence ID numbers is as follows:

hVB22B p-z VL: FR1/SEQ ID NO: 27
hVB22B p-z VL: CDR1/SEQ ID NO: 4
hVB22B p-z VL: FR2/SEQ ID NO: 28
hVB22B p-z VL: CDR2/SEQ ID NO: 5
hVB22B p-z VL: FR3/SEQ ID NO: 29
hVB22B p-z VL: CDR3/SEQ ID NO: 6
hVB22B p-z VL: FR4/SEQ ID NO: 30.

Herein, the correspondence between CDR and FR in the hVB22B g-e VH sequence and sequence ID numbers is as follows:

hVB22B g-e VH: FR1/SEQ ID NO: 19
hVB22B g-e VH: CDR1/SEQ ID NO: 1
hVB22B g-e VH: FR2/SEQ ID NO: 20
hVB22B g-e VH: CDR2/SEQ ID NO: 2
hVB22B g-e VH: FR3/SEQ ID NO: 21
hVB22B g-e VH: CDR3/SEQ ID NO: 3
hVB22B g-e VH: FR4/SEQ ID NO: 22.

Herein, the correspondence between CDR and FR in the hVB22B g-e VL sequence and sequence ID numbers is

as follows:

hVB22B g-e VL: FR1/SEQ ID NO: 31
hVB22B g-e VL: CDR1/SEQ ID NO: 4
hVB22B g-e VL: FR2/SEQ ID NO: 32
hVB22B g-e VL: CDR2/SEQ ID NO: 5
hVB22B g-e VL: FR3/SEQ ID NO: 33
hVB22B g-e VL: CDR3/SEQ ID NO: 6
hVB22B g-e VL: FR4/SEQ ID NO: 34.

Herein, the correspondence between CDR and FR in the hVB22B e VH sequence and sequence ID numbers is as follows:

hVB22B e VH: FR1/SEQ ID NO: 23
hVB22B e VH: CDR1/SEQ ID NO: 1
hVB22B e VH: FR2/SEQ ID NO: 24
hVB22B e VH: CDR2/SEQ ID NO: 2
hVB22B e VH: FR3/SEQ ID NO: 25
hVB22B e VH: CDR3/SEQ ID NO: 3
hVB22B e VH: FR4/SEQ ID NO: 26.

Herein, the correspondence between CDR and FR in the hVB22B e VL sequence and sequence ID numbers is as follows:

hVB22B e VL: FR1/SEQ ID NO: 31
hVB22B e VL: CDR1/SEQ ID NO: 4
hVB22B e VL: FR2/SEQ ID NO: 32
hVB22B e VL: CDR2/SEQ ID NO: 5
hVB22B e VL: FR3/SEQ ID NO: 33
hVB22B e VL: CDR3/SEQ ID NO: 6
hVB22B e VL: FR4/SEQ ID NO: 34.

Herein, the correspondence between CDR and FR in the hVB22B u2-wz4 VH sequence and sequence ID numbers is as follows:

hVB22B u2-wz4 VH: FR1/SEQ ID NO: 54
hVB22B u2-wz4 VH: CDR1/SEQ ID NO: 1
hVB22B u2-wz4 VH: FR2/SEQ ID NO: 55
hVB22B u2-wz4 VH: CDR2/SEQ ID NO: 2
hVB22B u2-wz4 VH: FR3/SEQ ID NO: 56
hVB22B u2-wz4 VH: CDR3/SEQ ID NO: 3
hVB22B u2-wz4 VH: FR4/SEQ ID NO: 57.
Herein, the correspondence between CDR and FR in the hVB22B u2-wz4 VL sequence and sequence ID numbers is as follows:
hVB22B u2-wz4 VL: FR1/SEQ ID NO: 59

hVB22B u2-wz4 VL: CDR1/SEQ ID NO: 4

hVB22B u2-wz4 VL: FR2/SEQ ID NO: 60
hVB22B u2-wz4 VL: CDR2/SEQ ID NO: 5
hVB22B u2-wz4 VL: FR3/SEQ ID NO: 61
hVB22B u2-wz4 VL: CDR3/SEQ ID NO: 6
hVB22B u2-wz4 VL: FR4/SEQ ID NO: 62.

Herein, the correspondence between CDR and FR in the hVB22B q-wz5 VH sequence and sequence ID numbers is as follows:

hVB22B q-wz5 VH: FR1/SEQ ID NO: 54

hVB22B q-wz5 VH: CDR1/SEQ ID NO: 1
hVB22B q-wz5 VH: FR2/SEQ ID NO: 55
hVB22B q-wz5 VH: CDR2/SEQ ID NO: 2
hVB22B q-wz5 VH: FR3/SEQ ID NO: 56
hVB22B q-wz5 VH: CDR3/SEQ ID NO: 3
hVB22B q-wz5 VH: FR4/SEQ ID NO: 57.

Herein, the correspondence between CDR and FR in the hVB22B q-wz5 VL sequence and sequence ID numbers is as follows:

hVB22B q-wz5 VL: FR1/SEQ ID NO: 59
hVB22B q-wz5 VL: CDR1/SEQ ID NO: 4
hVB22B q-wz5 VL: FR2/SEQ ID NO: 63
hVB22B q-wz5 VL: CDR2/SEQ ID NO: 5
hVB22B q-wz5 VL: FR3/SEQ ID NO: 64
hVB22B q-wz5 VL: CDR3/SEQ ID NO: 6
hVB22B q-wz5 VL: FR4/SEQ ID NO: 62.

The nucleotide sequence of VB22B VH is shown in SEQ ID NO: 7; the nucleotide sequence of VB22B VL is shown in SEQ ID NO: 9; the nucleotide sequence of VB22B scFv is shown in SEQ ID NO: 11; the nucleotide sequence of VB22B sc(Fv)2 is shown in SEQ ID NO: 13; the nucleotide sequence of hVB22B p-z VH is shown in SEQ ID NO: 35; the nucleotide sequence of hVB22B g-e VH is shown in SEQ ID NO: 37; the nucleotide sequence of hVB22B e VH is shown in SEQ ID NO: 39; the nucleotide sequence of hVB22B u2-wz4 VH is shown in SEQ ID NO: 69; the nucleotide sequence of hVB22B q-wz5 VH is shown in SEQ ID NO: 71; the nucleotide sequence of hVB22B p-z VL is shown in SEQ ID NO: 41; the nucleotide sequence of hVB22B g-e VL and hVB22B e VL is shown in SEQ ID NO: 43; the nucleotide sequence of hVB22B u2-wz4 VL is shown in SEQ ID NO: 70; the nucleotide sequence of hVB22B q-wz5 VL is shown in SEQ ID NO: 72; the nucleotide sequence of hVB22B p-z sc(Fv)2 is shown in SEQ ID NO: 45; the nucleotide sequence of hVB22B g-e sc(Fv) 2 is shown in SEQ ID NO: 47; the nucleotide sequence of hVB22B e sc(Fv)2 is shown in SEQ ID NO: 49; the nucleotide sequence of hVB22B u2-wz4 sc(Fv)2 is shown in SEQ ID NO: 75; and the nucleotide sequence of hVB22B q-wz5 sc(Fv)2 is shown in SEQ ID NO: 76.

(18) a humanized antibody having the amino acid sequence of any one of SEQ ID NO: 46 (hVB22B p-z: sc(Fv)2), SEQ ID NO: 48 (hVB22B g-e: sc(Fv)2), SEQ ID NO: 50 (hVB22B e: sc(Fv)2), SEQ ID NO: 73 (hVB22B u2-wz4: sc (Fv)2), and SEQ ID NO: 74 (hVB22B q-wz5: sc(Fv)2); and

(19) an antibody in which one or more amino acids are substituted, deleted, added and/or inserted in the amino acid sequence of any one of (1) to (18) described above, and which has an activity equivalent to that of the antibody described above.

Herein, "having an activity equivalent to that of the antibody described above" means that the mutated antibody has an equivalent activity as the original antibody to promote the growth of hematopoietic stem cells, to promote the growth and/or differentiation of CD34-positive hematopoietic cells, to enhance the engraftment of transplanted CD34-positive hematopoietic cells in the bone marrow, or to promote the recovery of hematopoiesis in hematopoietic stem cell transplantation.

Since the antibodies defined in (1) to (19) above have a very high agonistic activity for c-mpl, they are particularly useful as agents for promoting the growth of hematopoietic stem cells, agents for promoting the growth and/or differentiation of CD34-positive hematopoietic cells, agents for enhancing the engraftment of transplanted CD34-positive hematopoietic cells in the bone marrow, or agents for promoting the recovery of hematopoiesis in hematopoietic stem cell transplantation.

Methods for preparing polypeptides functionally equivalent to a certain polypeptide are well known to those skilled in the art, and include methods of introducing mutations into polypeptides. For example, those skilled in the art can prepare an antibody functionally equivalent to the antibodies of the present invention by introducing appropriate mutations into the antibody using site-directed mutagenesis (Hashimoto-Gotoh, T. et al. Gene 152, 271-275, (1995); Zoller, MJ, and Smith, M. Methods Enzymol. 100, 468-500, (1983); Kramer, W. et al., Nucleic Acids Res. 12, 9441-9456, (1984); Kramer, W. and Fritz HJ, Methods Enzymol. 154, 350-367, (1987); Kunkel, TA, Proc. Natl. Acad. Sci. USA. 82, 488-492, (1985); Kunkel, Methods Enzymol. 85, 2763-2766, (1988)), or such. Amino acid mutations may occur naturally. Thus, the present invention also comprises antibodies functionally equivalent to the antibodies of the present invention and comprising the amino acid sequences of these antibodies, in which one or more amino acids is mutated. In such mutants, the number of amino acids that are mutated is generally 50 amino acids or less, preferably 30 or less, more preferably 10 or less (for example, five amino acids or less).

It is preferable that an amino acid residue be mutated to another amino acid residue whose side chain retains the properties of that of the original amino acid residue. Examples of amino acid side chain properties are: hydrophobic amino acids (A, I, L, M, F, P, W, Y, and V), hydrophilic amino acids (R, D, N, C, E, Q, G, H, K, S, and T), amino acids comprising the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); aromatic ring-containing side chains (H, F, Y, and W) (amino acids are represented by one-letter codes in parentheses).

A polypeptide comprising a modified amino acid sequence, in which one or more amino acid residues is deleted, added, and/or replaced with other amino acids, is known to retain its original biological activity (Mark, D. F. et al., Proc. Natl. Acad. Sci. USA 81, 5662-5666 (1984); Zoller, M. J. & Smith, M. Nucleic Acids Research 10, 6487-6500 (1982); Wang, A. et al., Science 224, 1431-1433; Dalbadie-McFarland, G. et al., Proc. Natl. Acad. Sci. USA 79, 6409-6413 (1982)).

Fusion proteins containing antibodies that comprise the amino acid sequence of an antibody of the present invention, in which two or more amino acid residues have been added, are included in the present invention. The fusion protein results from a fusion between one of the above antibodies and a second peptide or protein, and is included in the present invention. The fusion protein can be prepared by ligating a polynucleotide encoding an antibody of the present invention and a polynucleotide encoding a second peptide or polypeptide in frame, inserting this into an expression vector, and expressing the fusion construct in a host. Some techniques known to those skilled in the art are available for this purpose. The partner peptide or polypeptide to be fused with an antibody of the present invention may be a known peptide, for example, FLAG (Hopp, T. P. et al., BioTechnology 6, 1204-1210 (1988)), 6x His consisting of six His (histidine) residues, 10x His, influenza hemagglutinin (HA), human c-myc fragment, VSV-GP fragment, p18HIV fragment, T7-tag, HSV-tag, E-tag, SV40 T antigen fragment, 1ck tag, oc-tubulin fragment, B-tag, Protein C fragment. Other partner polypeptides to be fused with the antibodies of the present invention include, for example, GST (glutathione-S-transferase), HA (influenza hemagglutinin), immunoglobulin constant region, β-galactosidase, and MBP (maltose-binding protein). A polynucleotide encoding one of these commercially available peptides or polypeptides can be fused with a polynucleotide encoding an antibody of the present invention. The fusion polypeptide can be prepared by expressing the fusion construct.

As described below, the antibodies of the present invention may differ in amino acid sequence, molecular weight, isoelectric point, presence/absence of sugar chains, and conformation depending on the cell or host producing the antibody, or purification method. However, a resulting antibody is included in the antibodies of the present invention, as long as it is functionally equivalent to an antibody of the present invention. For example, when an antibody of the present invention is expressed in prokaryotic cells, for example *E. coli,* a methionine residue is added to the N terminus of the original antibody amino acid sequence. Such antibodies are included in the present invention.

In another embodiment, the preferred c-mpl-recognizing antibodies comprised in the agents of the present invention include antibodies that recognize the epitopes recognized by the antibodies defined in (1) to (19) above.

Such antibodies can be prepared by methods known to those skilled in the art. The antibodies can be prepared by, for example, determining the epitope recognized by the antibody defined above by conventional methods, and using a polypeptide comprising one of the epitope amino acid sequences as an immunogen. Alternatively, the antibodies can be prepared by determining the epitopes of conventionally prepared antibodies and selecting an antibody that recognizes the epitope recognized by an antibody defined above.

In the present invention, a particularly preferred antibody is an antibody that recognizes the epitope recognized by the antibody comprising the amino acid sequence of SEQ ID NO: 73. The antibody comprising the amino acid sequence of SEQ ID NO: 73 is predicted to recognize the region from Glu 26 to Leu 274, preferably the region from Ala 189 to Gly 245, more preferably the region from Gln 213 to Ala 231 of human c-mpl. Thus, antibodies recognizing the region of amino acids 26 to 274, or amino acids 189 to 245, or amino acids 213 to 231 of human c-mpl are also included in the present invention.

Antibodies that recognize the region of amino acids 26 to 274, 189 to 245, or 213 to 231 in the amino acid sequence of human c-mpl (SEQ ID NO: 51) can be obtained by methods known to those skilled in the art. The antibodies can be obtained, for example, by methods of preparing antibodies using a peptide of the region of amino acids 26 to 274, 189 to 245, or 213 to 231 in the amino acid sequence of human c-mpl (SEQ ID NO: 51) as an immunogen. Alternatively, the antibodies can be obtained by methods of determining the epitopes recognized by conventionally prepared antibodies and then selecting antibodies that recognize the same epitopes as those recognized by the antibodies of the present invention.

Antibodies that bind to c-mpl can be prepared by methods known to those skilled in the art. For example, monoclonal antibody-producing hybridomas can be essentially generated by known technologies as follows: immunizing animals with c-mpl proteins or c-mpl-expressing cells as sensitized antigens using conventional immunological methods; fusing the obtained immunocytes with known parental cells by conventional cell fusion methods; and screening for monoclonal antibody-producing cells by conventional methods.

Specifically, monoclonal antibodies can be prepared by the method below.

First, the c-mpl protein, which is used as a sensitized antigen for preparing antibodies, is prepared by expressing the c-mpl gene/amino acid sequence (GenBank accession number: NM_005373). More specifically, the gene sequence encoding c-mpl is inserted into a known expression vector, which is then transfected into an appropriate host cell. The subject human c-mpl protein is purified from the host cell or culture supernatant using known methods.

The purified c-mpl protein is then used as a sensitized antigen. Alternatively, a partial c-mpl peptide may be used as a sensitized antigen. In this case, the partial peptide can also be chemically synthesized based on the amino acid sequence of human c-mpl.

The epitopes of c-mpl molecule that are recognized by an anti-c-mpl antibody of the present invention are not limited to a particular epitope, and may be any epitope on the c-mpl molecule. Thus, any fragment can be used as an antigen for preparing anti-c-mpl antibodies of the present invention, as long as the fragment comprises an epitope of the c-mpl molecule.

There is no limitation as to the type of mammalian species to be immunized with the sensitized antigen. However, a mammal is preferably selected based on its compatibility with the parental cell to be used in cell fusion. Generally, rodents (for example, mice, rats, and hamsters), rabbits, and monkeys can be used.

Animals can be immunized with a sensitized antigen by known methods such as a routine method of injecting a sensitized antigen into a mammal intraperitoneally or subcutaneously. Specifically, the sensitized antigen is diluted appropriately with phosphate-buffered saline (PBS), physiological saline and such, and then suspended. An adequate amount of a conventional adjuvant, for example, Freund's complete adjuvant, is mixed with the suspension, as necessary. An emulsion is then prepared for administering to a mammal several times over a 4- to 21-day interval. An appropriate carrier may be used for the sensitized antigen in immunization.

A mammal is immunized as described above. After a titer increase of target antibody in the serum is confirmed, immunocytes are collected from the mammal and then subjected to cell fusion. Spleen cells are the preferred immunocytes.

Mammalian myeloma cells are used as the parental cells to be fused with the above immunocytes. Preferable myeloma cells to be used include various known cell lines, for example, P3 (P3x63Ag8.653) (Kearney JF, et al., J. Immnol. 123, 1548-1550 (1979)), P3x63Ag8U.1 (Yelton DE, et al., Current Topics in Microbiology and Immunology 81, 1-7 (1978)), NS-1 (Kohler, G. and Milstein, C. Eur. J. Immunol. 6, 511-519 (1976)), MPC-11 (Margulies, D. H. et al., Cell 8, 405-415 (1976)), SP2/0 (Shulman, M. et al., Nature 276, 269-270 (1978)), FO (deSt. Groth, S. F. et al., J. Immunol. Methods 35, 1-21 (1980)), S194 (Trowbridge, I. S., J. Exp. Med. 148, 313-323 (1978)), and R210 (Galfre, G. et al., Nature 277, 131-133 (1979)).

Cell fusions between the immunocytes and the myeloma cells as described above can be essentially carried out using known methods, for example, a method by Kohler and Milstein (Kohler, G and Milstein, C., Methods Enzymol. 73, 3-46 (1981)).

More specifically, the above-described cell fusions are carried out, for example, in a conventional culture medium in the presence of a cell fusion-promoting agent. The fusion-promoting agents include, for example, polyethylene glycol (PEG) and Sendai virus (HVJ). If required, an auxiliary substance such as dimethyl sulfoxide may also be added to improve fusion efficiency.

The ratio of immunocytes to myeloma cells may be determined at one's own discretion, preferably, for example, one myeloma cell for every one to ten immunocytes. Culture media to be used for the above cell fusions include, for example, media that are suitable for the growth of the above myeloma cell lines, such as RPMI 1640 media and MEM media, and other conventional culture media used for this type of cell culture. In addition, serum supplements such as fetal calf serum (FCS) may also be used in combination.

Cell fusion is carried out as follows. As described above, predetermined amounts of immunocytes and myeloma cells are mixed well in the culture medium. PEG solution (for example, mean molecular weight of about 1,000-6,000) pre-heated to 37°C is added to the cell suspension typically at a concentration of 30% to 60% (w/v), and mixed to produce fused cells (hybridomas). Then, an appropriate culture medium is successively added to the mixture, and the sample is centrifuged to remove supernatant. This treatment is repeated several times to remove the unwanted cell fusion-promoting agent and others that are unfavorable to hybridoma growth.

Screening of the resulting hybridomas can be carried out by culturing them in a conventional selective medium, for example, hypoxanthine, aminopterin, and thymidine (HAT) medium. Culturing in the above-descried HAT medium is continued for a period long enough (typically, for several days to several weeks) to kill cells (non-fused cells) other than the desired hybridomas. Then, hybridomas are screened for single-cell clones capable of producing the target antibody by conventional limiting dilution methods.

In addition to the method for preparing the above-descried hybridomas by immunizing non-human animals with antigens, preferred human antibodies having binding activity to c-mpl can also be obtained by: sensitizing human lymphocytes with c-mpl *in vitro;* and fusing the sensitized lymphocytes with human myeloma cells capable of dividing permanently (see, Examined Published Japanese Patent Application No. (JP-B) Hei 1-59878). Alternatively, it is possible to obtain human antibodies against c-mpl from immortalized cells producing anti-MPL antibodies. In this method, the

cells producing anti-MPL antibodies are prepared by administering c-mpl as an antigen to transgenic animals comprising a repertoire of the entire human antibody genes (see, WO 94/25585, WO 93/12227, WO 92/03918, and WO 94/02602).

The monoclonal antibody-producing hybridomas thus prepared can be passaged in a conventional culture medium, and stored in liquid nitrogen over long periods of time.

Monoclonal antibodies can be prepared from the above-described hybridomas by, for example, a routine procedure of culturing the hybridomas and obtaining antibodies from the culture supernatants. Alternatively, monoclonal antibodies can be prepared by injecting the hybridomas into a compatible mammal; growing these hybridomas in the mammal; and obtaining antibodies from the mammal's ascites. The former method is suitable for preparing highly purified antibodies, while the latter is suitable for preparing antibodies on a large scale.

Recombinant antibodies can also be prepared by: cloning an antibody gene from a hybridoma; inserting the gene into an appropriate vector; introducing the vector into a host; and producing the antibodies by using genetic recombination techniques (see, for example, Vandamme, A. M. et al., Eur. J. Biochem. 192, 767-775, (1990)).

Specifically, an mRNA encoding the variable (V) region of anti-c-mpl antibody is isolated from hybridomas producing the anti-c-mpl antibodies. For mRNA isolation, total RNAs are first prepared by conventional methods such as guanidine ultracentrifugation methods (Chirgwin, J. M. et al., Biochemistry 18, 5294-5299 (1979)), or acid guanidinium thiocyanate-phenol-chloroform (AGPC) methods (Chomczynski, P. et al., Anal. Biochem. 162, 156-159 (1987)), and then the target mRNA is prepared using an mRNA Purification Kit (Pharmacia) and such. Alternatively, the mRNA can be directly prepared using the QuickPrep mRNA Purification Kit (Pharmacia).

A cDNA of the antibody V region is synthesized from the resulting mRNA using reverse transcriptase. cDNA synthesis is carried out using the AMV Reverse Transcriptase First-strand cDNA Synthesis Kit (Seikagaku Co.), or such. Alternatively, cDNA can be synthesized and amplified by the 5'-RACE method (Frohman, M. A. et al., Proc. Natl. Acad. Sci. USA 85, 8998-9002 (1988); Belyavsky, A. et al., Nucleic Acids Res. 17, 2919-2932 (1989)) using the 5'-Ampli FINDER RACE Kit (Clontech) and PCR.

Target DNA fragments are purified from the obtained PCR products and then ligated with vector DNAs to prepare recombinant vectors. The vectors are introduced into *E. coli* and such, and colonies are selected for preparing the recombinant vector of interest. The target DNA nucleotide sequence is then confirmed by conventional methods such as the dideoxynucleotide chain termination method.

Once a DNA encoding the V region of target anti-c-mpl antibody is obtained, the DNA is inserted into an expression vector which comprises a DNA encoding the constant region (C region) of a desired antibody.

The method for producing anti-Mpl antibodies to be used in the present invention typically comprises the steps of: inserting an antibody gene into an expression vector, so that the gene is expressed under the regulation of expression regulatory regions, such as enhancer and promotor; and transforming host cells with the resulting vectors to express antibodies.

For expressing the antibody gene, polynucleotides encoding H chain and L chain, respectively, are inserted into separate expression vectors and co-transfected into a host cell. Alternatively, polynucleotides encoding both H chain and L chain are inserted into a single expression vector and transfected into a host cell (see WO 94/11523).

For example, when *E. coli* is used as the host, the vector is not particularly limited, as long as it contains an "ori" for high amplification and purification in *E. coli* (for example, JM109, DH5$\alpha$, HB101, and XL1Blue), and a marker gene for selecting the *E. coli* transformants (for example, a drug resistance gene that allows selection using a drug such as ampicillin, tetracycline, kanamycin, or chloramphenicol). The vectors include, for example, M13 vectors, pUC vectors, pBR322, pBluescript, and pCR-Script. When the objective is to subclone or excise the cDNA, the vectors include, for example, pGEM-T, pDIRECT, and pT7, in addition to the vectors described above.

When the objective is to express in *E. coli,* for example, it is essential for expression vectors to have, in addition to the above characteristics for amplification in *E. coli,* a promoter that allows efficient expression in *E. coli,* such as the lacZ promoter (Ward et al., Nature (1989) 341, 544-546,1989; FASEB J. 6, 2422-2427, 1992), araB promoter (Better et al., Science 240, 1041-1043, 1988), or T7 promoter, when JM109, DH5$\alpha$, HB101, XL1-Blue, and such, are used as E. *coli* hosts. Such vectors include, in addition to the above vectors, pGEX-5X-1 (Pharmacia), "QIAexpress system" (QIAGEN), pEGFP, and pET (BL21, which is a strain expressing T7 RNA polymerase, is preferably used as the host).

The vectors may also comprise a signal sequence for polypeptide secretion. When proteins are produced into the periplasm of *E. coli,* the pelB signal sequence (Lei, S. P. et al., J. Bacteriol. (1987) 169, 4379) may be used as the signal sequence for protein secretion. The vectors can be introduced into host cells, for example, by calcium chloride methods or electroporation methods.

In addition to *E. coli* expression vectors, the vectors include, for example, expression vectors derived from mammals (for example, pcDNA3 (Invitrogen), pEGF-BOS (Nucleic Acids Res. 18(17), 5322, 1990), pEF, and pCDM8), insect cells (for example, "Bac-to-BAC baculovirus expression system" (GIBCO-BRL) and pBacPAK8), plants (for example, pMH1 and pMH2), animal viruses (for example, pHSV, pMV, and pAdexLcw), retroviruses (for example, pZIPneo), yeasts (for example, "Pichia Expression Kit" (Invitrogen), pNV11, and SP-Q01), and *Bacillus subtilis* (for example, pPL608 and pKTH50).

When proteins are expressed in animal cells such as CHO, COS, and NIH3T3 cells, it is essential for the vectors to have a promoter necessary for expression in such cells, for example, the SV40 promoter (Mulligan et al., Nature (1979) 277: 108), MMTV-LTR promoter, EF1α promoter (Mizushima et al., Nucleic Acids Res. (1990) 18: 5322), CMV promoter or such. More preferably, the vectors additionally have a gene for selecting transformed cells (for example, a drug resistance gene for selection by drug such as neomycin, G418, etc). Vectors having such characteristics include, for example, pMAM, pDR2, pBK-RSV, pBK-CMV, pOPRSV, and pOP13.

In order to stably express a gene and amplify the gene copy number in cells, methods of introducing into CHO cells that have defective nucleic acid synthesis pathway, a vector containing the DHFR gene (for example, pCHOI) which complements the defect, and using methotrexate (MTX) for amplification, may be used. Alternatively, in order to transiently express a gene, methods of transforming COS cells harboring a gene expressing the SV40 T antigen in their chromosomes with a vector containing the SV40 replication origin (for example, pcD) may be used. Replication origins derived from polyomaviruses, adenoviruses, bovine papillomaviruses (BPVs), and such, can also be used. Furthermore, to increase the gene copy number in host cells, the expression vectors may contain, as a selection marker, the aminoglycoside transferase (APH) gene, thymidine kinase (TK) gene, *E. coli* xanthine guanine phosphoribosyl transferase (Ecogpt) gene, dihydrofolate reductase (dhfr) gene, and such.

There is no particular limitation on the host cells into which the vectors are introduced. The host cells include, for example, *E. coli* and various animal cells. The host cells may be used, for example, as a production system to produce and express the antibodies of the present invention. There are *in vitro* and *in vivo* systems for production of polypeptides. *In vitro* systems include production systems using eukaryotic or prokaryotic cells.

When eukaryotic cells are used, host cells include, for example, animal cells, plant cells, and fungal cells. Known animal cells include mammalian cells such as CHO (J. Exp. Med. 108, 945 (1995)), COS, 3T3, myeloma, BHK (baby hamster kidney), HeLa, and Vero; amphibian cells such as *Xenopus laevis* oocytes (Valle, et al., Nature 291, 358-340 (1981)); and insect cells such as Sf9, Sf21, and Tuns. In the present invention, CHO-DG44, CHO-DXB11, COS7, and BHK cells are preferably used. Of the animal cells, CHO. cells are particularly preferable for large-scale expression. Vectors can be introduced into host cells, for example, by calcium phosphate methods, DEAE-dextran methods, methods using cationic liposome DOTAP (Boehringer-Mannheim), electroporation methods, lipofection methods, etc.

It is known that plant cells such as *Nicotiana tabacum-derived* cells are protein production systems, and callus cultures from these cells may be used. Protein systems using fungal cells including yeasts, for example, the genus *Saccharomyces* such as *Saccharomyces cerevisiae* and *Saccharomyces pombe;* and filamentous fungi, for example, the genus *Aspergillus* such as *Aspergillus niger* are known.

When prokaryotic cells are used, production systems that use bacterial cells are available. Such bacterial cells include *E. coli,* for example, JM109, DH5α, and HB101, and *Bacillus subtilis.*

In the present methods, the host cells described above are cultured. Antibodies can be obtained by culturing cells transformed with a polynucleotide of interest *in vitro.* Culturing can be performed according to known methods. For example, when animal cells are cultured, DMEM, MEM, RPMI 1640, or IMDM may be used as the culture medium. The culture medium may be used with serum supplements such as FBS or fetal calf serum (FCS). Alternatively, serum-free culture medium can be used. The preferred culture pH is about 6 to 8. Incubation is carried out typically at a temperature of about 30 to 40°C for about 15 to 200 hours. The culture medium is exchanged, aerated, or agitated, as necessary.

Meanwhile, *in vivo* polypeptide production systems include, for example, production systems using animals or plants. A polynucleotide of interest is introduced into an animal or plant to produce the polypeptide in the body of the animal or plant, and then the polypeptide is collected. Herein, "hosts" encompasses these animals and plants.

When using animals, there are production systems which use mammals or insects. Mammals such as goat, pig, sheep, mouse, and cattle can be used (Vicki Glaser SPECTRUM Biotechnology Applications (1993)). When mammals are used, transgenic animals can be used.

For example, a polynucleotide of interest is prepared as a fusion gene with a gene encoding a polypeptide specifically produced in milk, such as goat β-casein. Then, DNA fragments comprising the fusion gene are injected into goat embryos, which are then transplanted into female goats. The antibodies of interest can be obtained from milk produced by the transgenic goats born from the goats that received the embryos, or by their progeny. Appropriate hormones may be administered to the transgenic goats to increase the amount of milk containing the antibodies produced by the goats (Ebert, K.M. et al., Bio/Technology 12, 699-702 (1994)).

Furthermore, insects such as silkworms can be used. When silkworms are used, they can be infected with a baculovirus into which a polynucleotide encoding an antibody of interest is introduced, and then the antibody of interest can be obtained from the body fluids of these silkworms (Susumu, M. et al., Nature 315, 592-594 (1985)).

Furthermore, plants such as tobacco may be used, for example. When tobacco is used, a polynucleotide encoding an antibody of interest is inserted into a plant expression vector, for example, pMON 530, and the vector is introduced into a bacterium such as *Agrobacterium tumefaciens.* Tobacco, for example, *Nicotiana tabacum,* can be infected with the bacterium, and then the desired antibody can be prepared from the leaves of the tobacco (Julian K.-C.

Ma et al., Eur. J. Immunol. 24, 131-138 (1994)).

The resulting antibody can be isolated from the inside or outside (such as the medium) of host cells, and purified as a substantially pure and homogenous antibody. There is no limitation on the methods of isolating and purifying an antibody, and methods used in conventional polypeptide purification may be adopted. Polypeptides can be isolated and purified by selecting an appropriate combination of, for example, chromatographic columns, filtration, ultrafiltration, salting-out, solvent precipitation, solvent extraction, distillation, immunoprecipitation, SDS-polyacrylamide gel electrophoresis, isoelectric focusing, dialysis, recrystallization, etc.

Chromatography includes, for example, affinity chromatography, ion exchange chromatography, hydrophobic chromatography, gel filtration, reverse-phase chromatography, and adsorption chromatography (Strategies for Protein Purification and Characterization: A Laboratory Course Manual. Ed Daniel R. Marshak et al., Cold Spring Harbor Laboratory Press, 1996). Such chromatographies can be carried out using liquid phase chromatography such as HPLC and FPLC. Columns used for affinity chromatography include, for example, protein A columns and protein G columns. Columns that use protein A include, for example, Hyper D, POROS, and Sepharose F. F. (Pharmacia).

An antibody can be modified arbitrarily, and peptides can be deleted partially by treating the antibody with an appropriate protein modification enzyme before or after antibody purification. Such protein modification enzymes include, for example, trypsin, chymotrypsin, lysyl endopeptidases, protein kinases, and glucosidases.

Agonists for the TPO receptor (c-mpl) of the present invention may be low-molecular-weight compounds. The low-molecular-weight compounds of the present invention may be any compounds, as long as they have the activity to promote the growth of hematopoietic stem cells, to promote the growth and/or differentiation of CD34-positive hematopoietic cells, to enhance the engraftment of transplanted CD34-positive hematopoietic cells in the bone marrow, or to promote the recovery of hematopoiesis. In an embodiment, the preferred compounds include {5-[(2-{1-[5-(3,4-dichlorophenyl)-4-hydroxy-3-thienyl]ethylidene}hydrazino) carbonyl-2-thiophenecarboxylic acid (Blood First Edition Paper, prepublished online on February 16, 2006; DOI10.1182/blood-2005-11-4433), which is represented by the following formula:

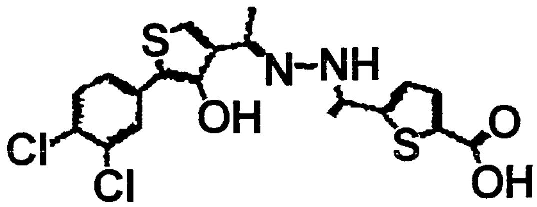

Furthermore, the agonists for the TPO receptor (c-mpl) of the present invention include Amgen AMG-531 (recombinant megakaryopoiesis stimulating protein), SB297115/Eltrombopag (Gsk's oral TPO-R agonistic compound), peg-TPOmp, YM477, and NIP004. Amgen AMG-531 is a protein with a molecular weight of 60,000 D, which contains an Fc domain and peptide receptor binding domains. This protein has the characteristics listed in Table 1 below (Clinical Pharmacology & Therapeutics. 76(6), 628 (2004); Blood, Volume 104, Abstract #511 (2004)).

Table 1

| |
|---|
| · MW=60,000 D |
| · Four Mpl binding sites |
| ·No sequence homology with TPO |
| · Expressed in *E. coli* |

SB297115/Eltrombopag is a compound represented by the formula shown below. This compound has the characteristics listed in Table 2 below (Blood, Volume 104, Abstract #2909 (2004)).

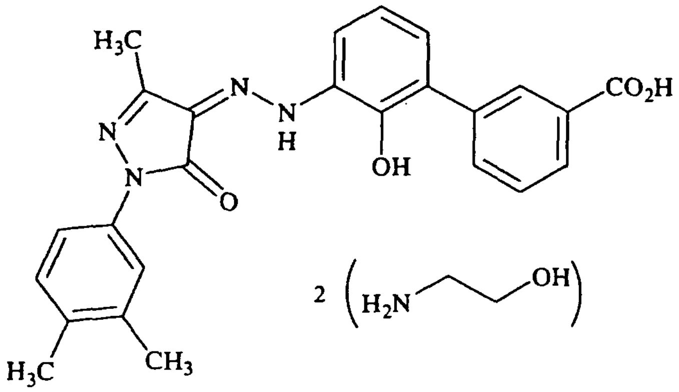

Table 2

| |
|---|
| · binding site: a hu c-mpl transmembrane domain (His499, Thr496) |
| · High species specificity |
| · No cross-reactivity: cynomolgus macaques, cat, mouse, etc. |
| · Cross-reactivity: chimpanzee |
| · *In vitro* activity |
| · H BM CD34 differentiation assay: EC50 ~ 100 nM |
| · $T_{1/2}$=12 hr |

peg-TPOmp is a PEGylated peptide found in a phage-display combinatorial peptide library, and is represented by the formula shown below. This peptide has the characteristics listed in Table 4 below (Blood, Volume 106, Number 11, Abstract #1249 (2005)).

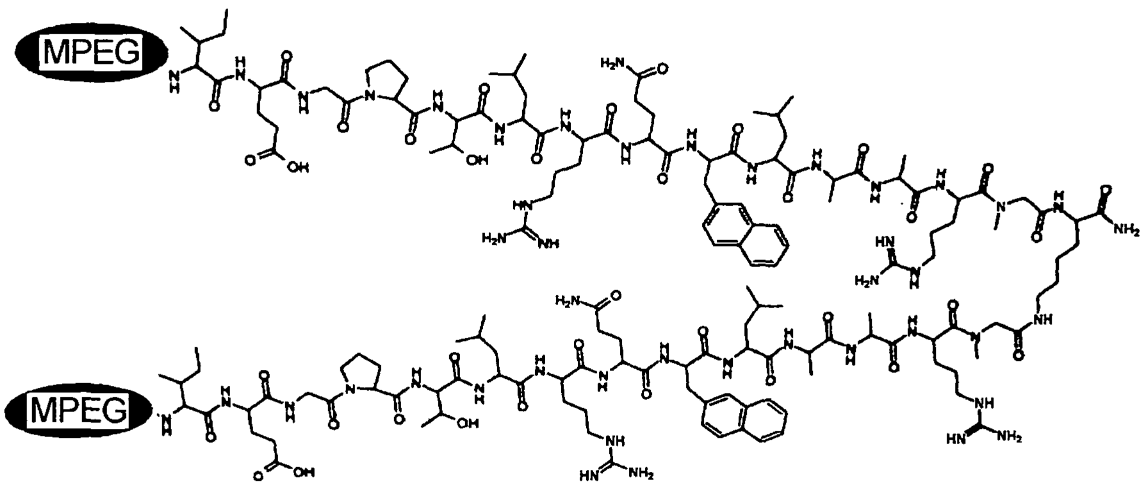

Table 3

| |
|---|
| · Two 14mer peptides are linked together via Lys, and the resulting 29mer peptide is PEGylated at both ends |
| · No homology to hTPO |
| · Cross-reactive to mouse, rat, and dog receptors |

Furthermore, the agonists for the TPO receptor (c-mpl) include YM477. Detailed information on YM477 is

disclosed in Blood, Volume 106, Number 11, Abstract #2298 (2005).

Antibodies recognizing c-mpl can be formulated by methods known to those skilled in the art. For example, the antibodies can be administered parenterally by injection of a sterile solution or suspension in water or other pharmaceutically acceptable solvents. For example, the antibodies can be formulated by appropriately combining with pharmaceutically-acceptable carriers or solvents, specifically, sterile water or physiological saline, vegetable oils, emulsifiers, suspending agents, surfactants, stabilizers, flavoring agents, excipients, vehicles, preservatives, binding agents, and such, and mixing at a unit dosage and form required by accepted pharmaceutical implementations. In such formulations, the amount of the thus obtained active ingredient should be within the required range.

A sterile composition to be injected can be formulated using a vehicle such as distilled water used for injection, according to standard protocols.

Aqueous solutions used for injections include, for example, physiological saline and isotonic solutions comprising glucose or other adjunctive agents such as D-sorbitol, D-mannose, D-mannitol, and sodium chloride. They may also be combined with an appropriate solubilizing agent such as alcohol, specifically, ethanol, polyalcohol such as propylene glycol or polyethylene glycol, or non-ionic detergent such as polysorbate 80™ or HCO-50, as necessary.

Oil solutions include sesame oils and soybean oils, and can be combined with solubilizing agents such as benzyl benzoate or benzyl alcohol. Injection solutions may also be formulated with buffers, for example, phosphate buffers or sodium acetate buffers; analgesics, for example, procaine hydrochloride; stabilizers, for example, benzyl alcohol or phenol; or anti-oxidants. The prepared injections are typically aliquoted into appropriate ampules.

The administration is preferably carried out parenterally, specifically, by injection, intranasal administration, intrapulmonary administration, percutaneous administration, or such. Injections include, for example, intravenous injections, intramuscular injections, intraperitoneal injections, and subcutaneous injections. The injection solutions can be also administered systemically or locally.

The administration methods can be selected properly according to the patient's age, condition, and such. The applied dose of a pharmaceutical composition comprising an antibody or polynucleotide encoding the antibody may be, for example, in the range of 0.0001 to 1,000 mg/kg body weight. Alternatively, the dosage may be, for example, in the range of 0.001 to 100,000 mg/kg body weight. However, the dosage is not restricted to the values described above. The dosage and administration methods depend on the patient's weight, age, and condition, and are appropriately selected by those skilled in the art.

There is no limitation on the timing of administration of the agents of the present invention. The agents can be administered, for example, when one intends to promote growth of hematopoietic stem cells, to promote growth and/or differentiation of CD34-positive hematopoietic cells, to enhance engraftment of transplanted CD34-positive hematopoietic cells in the bone marrow, or to allow recovery of hematopoiesis. For example, the agents of the present invention can be administered in hematopoietic stem cell transplantation, which is performed for patients with impaired hematopoietic function of the bone marrow. When administered alone, the agents of the present invention enhance the engraftment of not only transplanted hematopoietic stem cells but also myeloid and/or lymphoid cells in the bone marrow, in a dose-dependent manner.

When administered at a relatively high dose for a certain period immediately after transplantation, the agents of the present invention can produce the effect of promoting the engraftment of transplanted hematopoietic stem cells in the bone marrow (see Example 3). In consideration of the symptoms, age, and such of the patient who needs administration of an agent of the present invention, those skilled in the art can appropriately determine the dose of the agent of the present invention, and the period of administration of the agent immediately after transplantation. The period of administration of the agents of the present invention (administration period) includes, but is not limited to, for example, a period of three days or more, preferably seven to 28 days, or more, from the day of transplantation or from the day after transplantation. The dose may be ten times or more, preferably five times or more, and more preferably, twice or more the blood TPO concentration in a patient who received bone marrow transplantation. However, the dose is not limited thereto, and the agents can be administered at a dose necessary to maintain the blood concentration for a certain period or longer after transplantation.

There is no limitation on the number of times the agents of the present invention can be administered for each hematopoietic stem cell transplantation. The agents can be administered at any frequency at the time of or after hematopoietic stem cell transplantation. The timing, dose, and frequency of administration of the agents of the present invention can be appropriately determined according to the symptoms of the patient who received hematopoietic stem cell transplantation. The timing and dose of administration can be, for example, those described above.

The agents of the present invention are used for hematopoietic stem cell transplantation. Specifically, the agents of the present invention may be used after hematopoietic stem cell transplantation. The hematopoietic stem cell transplantation of the present invention includes, but is not limited to, bone marrow transplantation, peripheral blood stem cell transplantation, and cord blood transplantation. In a particularly preferred embodiment, hematopoietic stem cell transplantation for which the agents of the present invention are used includes human cord blood transplantation.

There is no particular limitation on the administration site for the agents of the present invention, and subcutaneous injection, intravenous injection, oral administration, and such, may be performed. In the present invention, intravenous administration by drip infusion is particularly preferred. It is possible to administer the agents of the present invention in combination with hematopoietic stem cells. When administered in combination with hematopoietic stem cells, the agents of the present invention can be simultaneously administered at the same site as that of the hematopoietic stem cells, or can be administered at a timing and/or site different from that of the hematopoietic stem cells. When administered at the same site, the agents and cells can be administered intravenously. The timing of administration can be selected in the same way as when the agents of the present invention are administered alone.

Meanwhile, when the agents of the present invention are administered at a timing different from that of hematopoietic stem cells, there is no limitation on the order or interval of administration of the agents and cells.

When the agents of the present invention are administered in combination with hematopoietic stem cells, the cells may be self-derived (autotransplantation) or provided by other persons (allotransplantation). Hematopoietic stem cells can be obtained by methods well-known to those skilled in the art, for example, by the methods described in the documents listed below.

Heike, T et al., "Ex vivo expansion of hematopoietic stem cells by cytokines", Biochimica et Biophysica Acta, vol. 1592, p. 313-321 (2002)

Yvette van Hensbergen et al., "Ex vivo culture of human CD34+ cord blood cells with thrombopoietin (TPO) accelerates platelet engraftment in a NOD/SCID mouse model", Experimental Hematology, vol. 34, p. 943-950 (2006)

In the present invention, diseases to which hematopoietic stem cell transplantation is applicable are not limited, and preferably include acute myeloid leukemia (AML), acute lymphoblastic leukemia (ALL), chronic myeloid leukemia (CML), myelodysplastic syndrome (MDS), aplastic anemia (AA), malignant lymphoma, and adult T-cell leukemia.

The present invention is based on the present inventors' fmding that the contact between hematopoietic stem cells and an agonist for the TPO receptor (c-mpl) increases the number of differentiated lymphoid cells and/or myeloid cells. Thus, the present invention relates to agents comprising an agonist for the TPO receptor (c-mpl) as an active ingredient, which are used to increase the number of lymphoid cells and/or myeloid cells differentiate from hematopoietic stem cells, by contacting the agonist with hematopoietic stem cells. The present invention also relates to agents comprising an agonist for the TPO receptor (c-mpl) as an active ingredient, which are used to increase the number of lymphoid cells and/or myeloid cells differentiate from hematopoietic stem cells, by administering the agents in combination with hematopoietic stem cells intravenously by drip infusion. The agonists, hematopoietic stem cells, lymphoid cells, myeloid cells, administration timing, dose, and such are as described above.

All prior art documents cited in this specification are incorporated herein by reference.

## Examples

Hereinbelow, the present invention will be specifically described with reference to the Examples, but it is not to be construed as being limited thereto.

[Example 1] Effect of the TPO receptor agonist on the engraftment number of different human blood cell lineages in the bone marrow of mice transplanted with human cord blood-derived hematopoietic stem cells.

The experiments were carried out by the method described below to assess the effect of the sc(Fv)2 antibody (hVB22 u2-wz4: sc(Fv)2) comprising the amino acid sequence of SEQ ID NO: 73 on the engraftment number of different human blood cell lineages in the bone marrow of mice transplanted with human cord blood-derived hematopoietic stem cells at early stages. The sc(Fv)2 antibody comprising the amino acid sequence of SEQ ID NO: 73 can be prepared by the method described in WO 2005/056604.

### Methods

Mice used were acclimatized six-week-old male NOD.CB17-Prkdc<scid>/J. After systemic irradiation with 3.0 Gy of X-ray, an anti-asialo-GM1 antibody was intraperitoneally administered to the mice once every ten days from the day of irradiation. $5x10^4$ human cord blood-derived CD34-positive cells were transplanted to each mouse at the caudal vein one day after irradiation. From the day after transplantation, the sc(Fv)2 antibody of SEQ ID NO: 73 was administered every day for ten consecutive days, and after that, administration was conducted for five days followed by two days of break. The doses were: 0.25 mg/5 ml/kg in the morning, and 2 mg/5 ml/kg in the evening. The antibody was subcutaneously administered at eight-hour intervals, twice a day for three weeks in total (n = 10). 20 mmol/l citric acid buffer containing 0.02% Tween 80 was administered as a vehicle in the same way as the sc(Fv)2 antibody of SEQ ID NO: 73. The bone marrow was collected after three weeks transplantation, and FACS analysis was performed using EPIX XL. The absolute

number of human cells was determined using Flow-Count (Beckman).

Results and Discussion

The number of human cells in the mouse right and left femurs was determined. In the group to which the sc (Fv)2 antibody of SEQ ID NO: 73 was administered, not only the number of human CD34-positive cells, which was initially expected to increase, but also the numbers of CD45-positive cells, CD41-positive cells, CD19-positive cells, and CD33-positive cells were statistically significantly increased as compared to the vehicle group (Fig. 1). These results indicate that administration of the sc(Fv)2 antibody of SEQ ID NO: 73 as a c-mpl agonist contributes to the induction of human megakaryocyte-specific differentiation, as well as the increase of CD34-positive hematopoietic cells survived after engraftment and the resulting increase in different human blood cell lineages.

[Example 2] Effect of the TPO receptor agonist on the number of human CFU-Meg colonies in the bone marrow of mice transplanted with human cord blood-derived hematopoietic stem cells.

The following experiments were carried out to assess the effect of the sc(Fv)2 antibody of SEQ ID NO: 73 on the number of human CFU-Meg colonies after transplantation of human cord blood-derived hematopoietic stem cells.

Methods

Mice used were acclimatized six-week-old male NOD.CB17-Prkdc<scid>/J. After systemic irradiation with 3.0 Gy of X-ray, an anti-asialo-GM1 antibody was intraperitoneally administered to the mice once every ten days from the day of irradiation. $5 \times 10^4$ human cord blood-derived CD34-positive cells were transplanted to each mouse at the caudal vein one day after irradiation. From the day after transplantation, the sc(Fv)2 antibody of SEQ ID NO: 73 was administered every day for ten consecutive days, and then after that, administration was conducted for five days followed by two days of break. The antibody was subcutaneously administered at 0.25 mg/5 ml/kg in the morning and 2 mg/5 ml/kg in the evening, at eight-hour intervals, twice a day for three weeks in total (n = 10). 20 mmol/l citric acid buffer containing 0.02% Tween 80 was administered as a vehicle in the same way as the sc(Fv)2 antibody of SEQ ID NO: 73. Bone marrow cells were collected after three weeks transplantation. Bone marrow cells contained in the mouse femurs were cultured with the sc(Fv)2 antibody of SEQ ID NO: 73 for 13 days using MegaCult-C (StemCell Technologies). The number of human CFU-Meg colonies was determined by counting CD41-positive colonies containing 50 or more cells under a light microscope.

Results and Discussion

It was shown that the number of CFU-Meg colonies in the bone marrow of the group to which the sc(Fv)2 antibody of SEQ ID NO: 73 was administered was statistically significantly increased than that of the vehicle-administered group (Fig. 2).

[Example 3] Dose-dependent effect of the TPO receptor agonist on the engraftment number of different human blood cell lineages in the bone marrow of mice transplanted with human cord blood-derived hematopoietic stem cells.

The following experiments were carried out to assess the dose-dependent effect of the sc(Fv)2 antibody of SEQ ID NO: 73 on the engraftment number of different human blood cell lineages in the bone marrow of mice transplanted with human cord blood-derived hematopoietic stem cells.

Methods

Mice used were acclimatized six-week-old male NOD.CB 17-Prkdc<scid>/J. After systemic irradiation with 3.0 Gy of X-ray, an anti-asialo-GM1 antibody was intraperitoneally administered to the mice on the day of irradiation and eight days after irradiation. $5 \times 10^4$ human cord blood-derived CD34-positive cells were transplanted to each mouse at the caudal vein the day after irradiation. The sc(Fv)2 antibody of SEQ ID NO: 73 was administered every day for ten consecutive days from the day after the irradiation, according to the following three dosages (subcutaneous administration; n = 9 for each group).

- High-dose group (0.25 mg/kg in the morning, an eight-hour interval, and 2 mg/kg in the evening, per day)
- Medium-dose group (0.05 mg/kg in the morning, an eight-hour interval, and 0.2 mg/kg in the evening, per day)
- Low-dose group (0.01 mg/kg in the morning, an eight-hour interval, and 0.02 mg/kg in the evening, per day)

The dose was adjusted so that the minimum drug concentration in peripheral blood during the administration period is 50 ng/ml, 10 ng/ml, or 2 ng/ml in the high-, medium-, or low-dose group, respectively.

20 mmol/l sodium citrate/150 mM NaCl buffer (pH 6.5) containing 0.02% Tween 80 was administered as a vehicle in the same way as the sc(Fv)2 antibody of SEQ ID NO: 73. Bone marrow cells were collected after two weeks transplantation, and FACS analysis was performed. The absolute number of human cells was determined using Flow-Count (Beckman).

Results and Discussion

The number of human cells in the mouse right and left femurs was determined. Not only the number of CD34-positive cells, but also the numbers of CD45-positive cells, CD41-positive cells, CD19-positive cells, CD33-positive cells, and CD38-positive cells were found to increase in a dose-dependent manner (Fig. 3). This suggests the possibility that administration of the sc(Fv)2 antibody of SEQ ID NO: 73 could be directly involved in the drug efficacy, as well as that the efficacy could be controlled by varying the dose. The mouse endogenous TPO concentration is about 1 ng/ml (reported value and in-house measurement value). X-ray irradiation elevates the concentration by about five to ten times according to in-house data on mice. Similarly, it is clinically known that, in human, the concentration is elevated from the normal level of about 80 pg/ml to about 1 to 3 ng/ml. From this Example, it was confirmed that the drug efficacy is enhanced in a dose-dependent manner by administering the sc(Fv)2 antibody of SEQ ID NO: 73 at a concentration higher than that of endogenous TPO.

In this Example, as described in the "Methods" section, the administration strategy was designed to maintain the minimum concentration level of the sc(Fv)2 antibody of SEQ ID NO: 73 in blood.

Industrial Applicability

The present invention provides novel agents for promoting the growth of hematopoietic stem cells, agents for promoting the growth and/or differentiation of CD34-positive hematopoietic cells, agents for enhancing the engraftment of transplanted CD34-positive hematopoietic cells in the bone marrow, and agents for promoting the recovery of hematopoiesis. The agents of the present invention comprise an agonist for the TPO receptor (c-mpl) as an active ingredient.

The novel agents of the present invention, namely, agents for promoting the growth of hematopoietic stem cells, agents for promoting the growth and/or differentiation of CD34-positive hematopoietic cells, agents for enhancing the engraftment of transplanted CD34-positive hematopoietic cells in the bone marrow, and agents for promoting the recovery of hematopoiesis, are expected to be effective when administered alone (without using G-CSF and erythropoietin in combination) after hematopoietic stem cell transplantation (in particular, cord blood transplantation).

The agents described above, which are provided by the present invention, are useful for promoting the growth of hematopoietic stem cells, promoting the growth and/or differentiation of CD34-positive hematopoietic cells, promoting the engraftment of transplanted CD34-positive hematopoietic cells in the bone marrow, or promoting the recovery of hematopoiesis upon hematopoietic stem cell transplantation (bone marrow transplantation, peripheral blood stem cell transplantation, and cord blood transplantation), and can be used to treat diseases to which hematopoietic stem cell transplantation is applicable, for example, acute myeloid leukemia, chronic myeloid leukemia, myelodysplastic syndrome, acute lymphoblastic leukemia, adult T-cell leukemia, aplastic anemia, and malignant lymphoma, etc.

In particular, the delay in platelet recovery after transplantation becomes a problem in cord blood transplantation. The agents of the present invention are useful, because enhancement of the engraftment of transplanted hematopoietic stem cells in the bone marrow in transplantation promotes platelet recovery, in cooperation with the inherent activity of a c-mpl agonist to promote the proliferation and differentiation of megakaryocytes.

G-CSF has been used in conventional hematopoietic stem cell transplantation. The problem with G-CSF is that its activity is specific to neutrophils. In contrast, TPO acts not only on megakaryocytes but also on stem cells, and thus can possibly recover cells of more various lineages. Furthermore, TPO is reasonably expected to produce synergistic effects in combination with currently-approved G-CSF or EPO. The agents of the present invention are also useful from this viewpoint.

## Claims

1. An agent for promoting the growth of hematopoietic stem cells, wherein the agent comprises an agonist for the TPO receptor (c-mpl) as an active ingredient.

2. An agent for promoting the growth and/or differentiation of CD34-positive hematopoietic cells, wherein the agent comprises an agonist for the TPO receptor (c-mpl) as an active ingredient.

3. An agent for enhancing the engraftment of transplanted CD34-positive hematopoietic cells in the bone marrow, wherein the agent comprises an agonist for the TPO receptor (c-mpl) as an active ingredient.

4. An agent for promoting the recovery of hematopoiesis, wherein the agent comprises an agonist for the TPO receptor (c-mpl) as an active ingredient.

5. The agent of any one of claims 1 to 4, wherein the agent is used for hematopoietic stem cell transplantation.

6. The agent of any one of claims 1 to 5, wherein the agent is used after hematopoietic stem cell transplantation.

7. The agent of claim 5 or 6, wherein the hematopoietic stem cell transplantation is selected from bone marrow transplantation, peripheral blood stem cell transplantation, and cord blood transplantation.

8. The agent of claim 7, wherein the cord blood transplantation is human cord blood transplantation.

9. The agent of claim 8, wherein the agent is administered more than once.

10. An agent for promoting the growth of lymphoid cells and/or myeloid cells, wherein the agent comprises an agonist for the TPO receptor (c-mpl) as an active ingredient.

11. An agent for promoting differentiation into lymphoid cells and/or myeloid cells, wherein the agent comprises an agonist for the TPO receptor (c-mpl) as an active ingredient.

12. A method for promoting the growth of hematopoietic stem cells, wherein the method comprises the step of administering an agonist for the TPO receptor (c-mpl) to a subject.

13. A method for promoting the growth and/or differentiation of CD34-positive hematopoietic cells, wherein the method comprises the step of administering an agonist for the TPO receptor (c-mpl) to a subject.

14. A method for enhancing the engraftment of transplanted CD34-positive hematopoietic cells in the bone marrow, wherein the method comprises the step of administering an agonist for the TPO receptor (c-mpl) to a subject.

**15.** A method for promoting the recovery of hematopoiesis, wherein the method comprises the step of administering an agonist for the TPO receptor (c-mpl) to a subject.

**16.** A method for proliferating lymphoid cells and/or myeloid cells, wherein the method comprises the step of administering an agonist for the TPO receptor (c-mpl) to a subject.

**17.** A method for promoting differentiation into lymphoid cells and/or myeloid cells, wherein the method comprises the step of administering an agonist for the TPO receptor (c-mpl) to a subject.

**18.** Use of an agonist for the TPO receptor (c-mpl) in the production of an agent for promoting the growth of hematopoietic stem cells.

**19.** Use of an agonist for the TPO receptor (c-mpl) agonist in the production of an agent for promoting the growth and/or differentiation of CD34-positive hematopoietic cells.

**20.** Use of an agonist for the TPO receptor (c-mpl) in the production of an agent for enhancing the engraftment of transplanted CD34-positive hematopoietic cells in the bone marrow.

**21.** Use of an agonist for the TPO receptor (c-mpl) in the production of an agent for promoting the recovery of hematpoiesis.

**22.** Use of an agonist for the TPO receptor (c-mpl) in the production of an agent for promoting the growth of lymphoid cells and/or myeloid cells.

**23.** Use of an agonist for the TPO receptor (c-mpl) in the production of an agent for promoting differentiation into lymphoid cells and/or myeloid cells.

FIG. 1

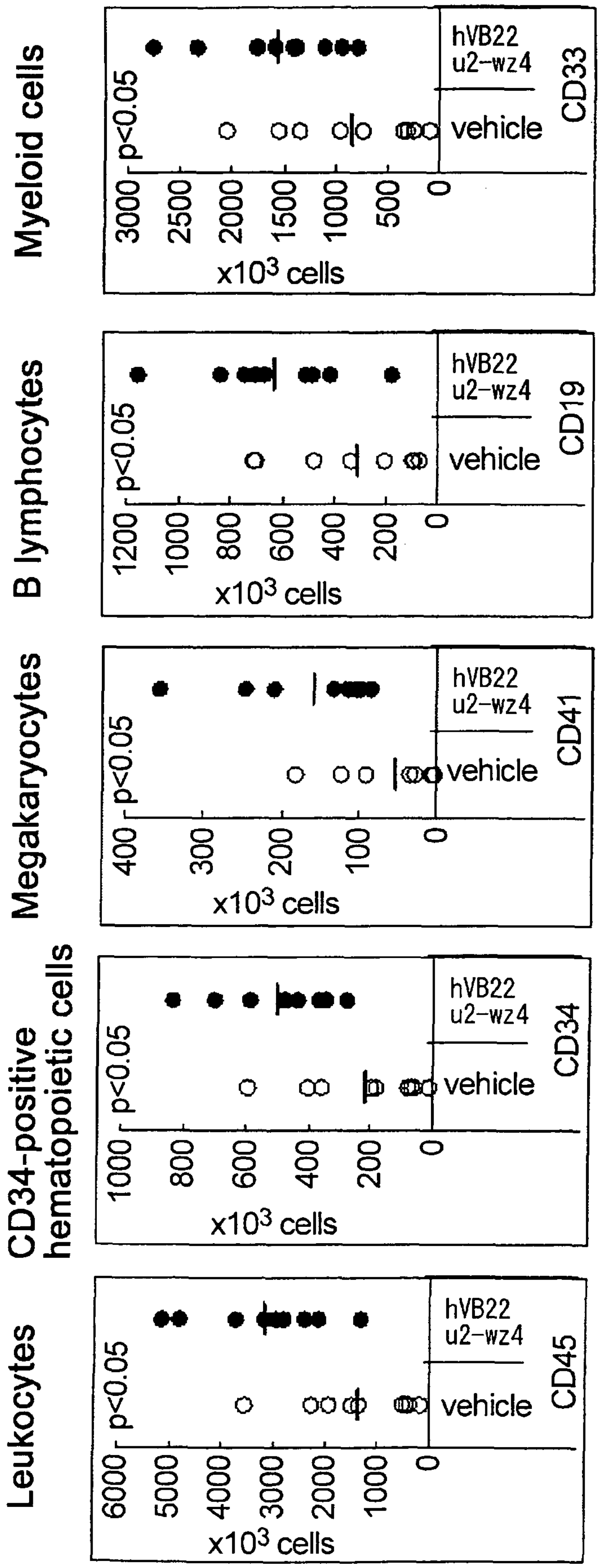
Myeloid cells
x10^3 cells
3000
2500
2000
1500
1000
500
0
p<0.05
vehicle
hVB22 u2-wz4
CD33
B lymphocytes
x10^3 cells
1200
1000
800
600
400
200
0
p<0.05
vehicle
hVB22 u2-wz4
CD19
Megakaryocytes
x10^3 cells
400
300
200
100
0
p<0.05
vehicle
hVB22 u2-wz4
CD41
CD34-positive hematopoietic cells
x10^3 cells
1000
800
600
400
200
0
p<0.05
vehicle
hVB22 u2-wz4
CD34
Leukocytes
x10^3 cells
6000
5000
4000
3000
2000
1000
0
p<0.05
vehicle
hVB22 u2-wz4
CD45

FIG. 2

Human CD41-positive colonies

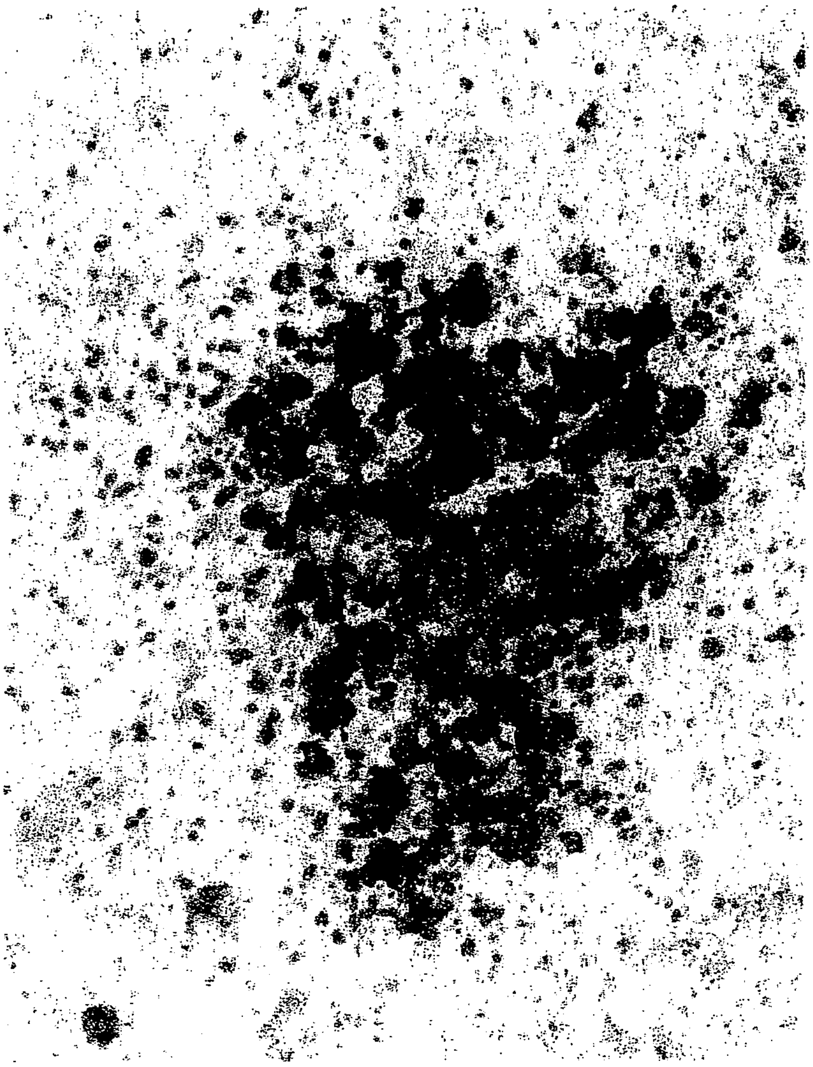

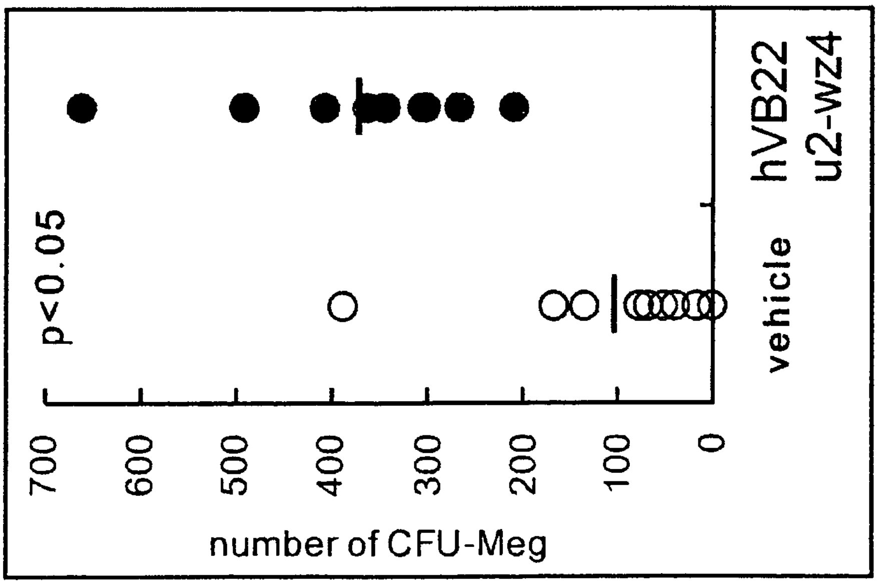

FIG. 3

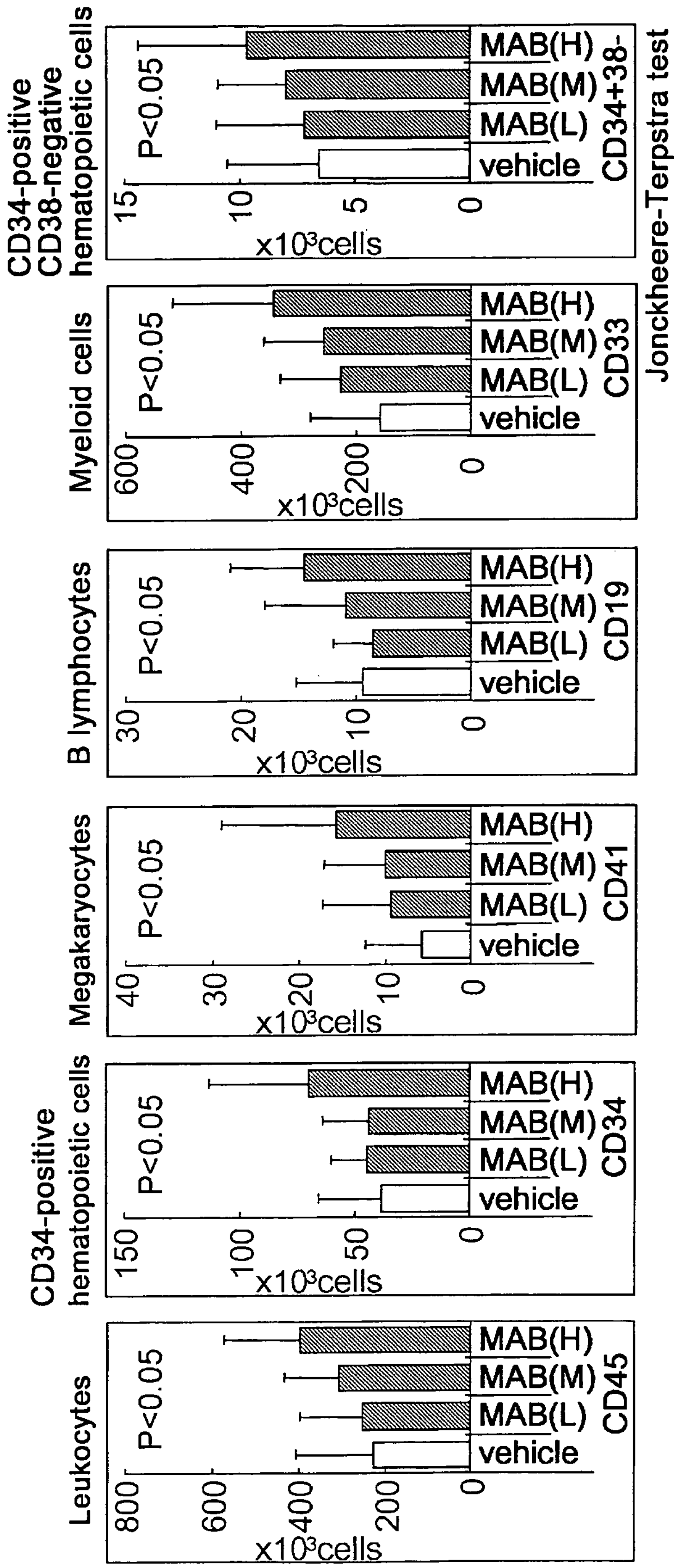
CD34-positive CD38-negative hematopoietic cells
Myeloid cells
B lymphocytes
Megakaryocytes
CD34-positive hematopoietic cells
Leukocytes
P<0.05
x10³cells
vehicle
MAB(L)
MAB(M)
MAB(H)
CD34+38-
CD33
CD19
CD41
CD34
CD45
Jonckheere-Terpstra test

**INTERNATIONAL SEARCH REPORT**

International application No.
PCT/JP2007/061850

A. CLASSIFICATION OF SUBJECT MATTER
*A61K45/00*(2006.01)i, *A61K39/395*(2006.01)i, *A61P7/06*(2006.01)i, *A61P35/02*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K45/00, A61K39/395, A61P7/06, A61P35/02, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS(STN), CAplus(STN), EMBASE(STN), MEDLINE(STN), WPI(DIALOG), JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X | JP 10-510842 A (ZymoGenetics, Inc.), 20 October, 1998 (20.10.98), Claims; page 8, lines 21 to 24; examples & WO 96/40218 A1 & AU 199662500 A & EP 831888 A1 & US 6013067 A & MX 199709244 A1 & KR 1999022420 A & CN 1190348 A | 1-11,18,19, 21-23 |
| X | JP 2005-539082 A (Ortho-Mcneil Pharmaceutical Inc.), 22 December, 2005 (22.12.05), Full text & WO 2004/026332 A1 & AU 2003275077 A1 & EP 1542714 A1 & NO 200501841 A & BR 200314591 A & US 2005/0282277 A1 & CN 1723036 A & ZA 200503036 A & KR 2005093759 A | 1-11,18-20, 22,23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 July, 2007 (24.07.07) | 07 August, 2007 (07.08.07) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2007/061850

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | NAKAMURA, T., *et al.*, A Novel Non-Peptidyl Human C-Mpl Agonist, NIP-004, Stimulates Human Megakaryopoiesis and Thrombopoiesis [online]. Blood (ASH Annual Meeting Abstracts), 2005, 106: Abstract 3148. Retrieved from the Internet: <URL:http://abstracts.hematologylibrary.org/cgi/content/abstract/ashmtg;106/11/3148?maxtoshow=&HITS=10&hits=10&RESULTFORMAT=&fulltext=NIP-004&searchid=1&FIRSTINDEX=0&volume=106&issue=11&resourcetype=HWCIT>, full text | 1,18<br>2-11,19-23 |
| X<br>Y | SUZUKI, K., *et al.*, YM477, a Novel Orally-Active Thrombopoietin Receptor Agonist [online]. Blood (ASH Annual Meeting Abstracts), 2005, 106: Abstract 2298. Retrieved from the Internet: <URL:http://abstracts.hematologylibrary.org/cgi/content/abstract/ashmtg;106/11/2298?maxtoshow=&HITS=10&hits=10&RESULTFORMAT=&fulltext=YM477&searchid=1&FIRSTINDEX=0&volume=106&issue=11&resourcetype=HWCIT>, full text | 1,18<br>2-11,19-23 |
| Y | WO 2005/107784 A1 (Chugai Pharmaceutical Co., Ltd.),<br>17 November, 2005 (17.11.05),<br>Claims; abstract<br>(Family: none) | 1-11,18-23 |
| Y | WO 2005/056604 A1 (Chugai Pharmaceutical Co., Ltd.),<br>23 June, 2005 (23.06.05),<br>Claims; abstract<br>& EP 1616881 A1 & NO 200603224 A<br>& US 2006/0222643 A1 & AU 2004297111 A1<br>& MX 2006006289 A1 & BR 200417077 A<br>& KR 2006107572 A | 1-11,18-23 |
| Y | WO 02/33072 A1 (Chugai Pharmaceutical Co., Ltd.),<br>25 April, 2002 (25.04.02),<br>Claims; abstract<br>& AU 200210917 A & EP 1327680 A1<br>& KR 2003055274 A & CN 1469925 A<br>& US 2004/0091475 A1 & CN 1721445 A | 1-11,18-23 |
| A | JP 2004-222502 A (Asahi Kasei Corp.),<br>12 August, 2004 (12.08.04),<br>(Family: none) | 1-11,18-23 |
| A | JP 2005-204539 A (Mitsubishi Pharma Corp.),<br>04 August, 2005 (04.08.05),<br>& WO 2005/071064 A1 | 1-11,18-23 |

Form PCT/ISA/210 (continuation of second sheet) (April 2005)

**INTERNATIONAL SEARCH REPORT**

International application No.
PCT/JP2007/061850

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 12-17
because they relate to subject matter not required to be searched by this Authority, namely:
The invention according to claims 12-17 includes the step of administering an agonist for TPO receptor to a test subject, and corresponds to a method for treatment of the human body by therapy because the human is included in the test subject. (PCT Article 17(2)(a)(i), PCT Rule 39.1(iv))

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2007/061850

Regarding claims 1-11, 18-23

Claims 1-11, 18-23 describe an agonist for TPO receptor as an active ingredient of a drug or an ingredient to be used for manufacturing a drug.

As the agonist for TPO receptor, a lot of compounds other than TPO can be included, however, even a person skilled in the art cannot clearly realize the whole compounds. Further, even if the description of the specification is examined, it is not described to such an extent that the whole compounds are made clear.

Thus, a sufficient support by the specification is not made by the description of the claims. Further, it is not clearly and sufficiently disclosed to such an extent that a person skilled in the art can make implementation. (PCT Articles 5 and 6)

Incidentally, in the creation of this international search report, a prior art search was carried out based on the description of the specification within a reasonable range.

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200233072 A
- WO 2005056604 A
- WO 2005107784 A
- EP 404097 A
- WO 9311161 A
- EP 125023 A
- WO 9602576 A
- WO 9813388 A
- JP HEI159878 B
- WO 9425585 A
- WO 9312227 A
- WO 9203918 A
- WO 9402602 A
- WO 9411523 A


### Non-patent literature cited in the description


- *Stem Cells,* 1996, vol. 14 (1), 124-132
- **ELLIOTT S et al.** *J. Biol. Chem.,* 1996, vol. 271 (40), 24691-24697
- **ABE et al.** *Immunol. Lett.,* 1998, vol. 61, 73-78
- **BIJIA DENG et al.** *Blood,* 1998, vol. 92, 1981-1988
- *British Journal of Haematology,* 2003, vol. 122, 837-846
- *Japanese Journal of Transfusion Medicine,* 2000, vol. 46 (3), 311-316
- *Blood,* 2006, vol. 107, 4300-4307
- *Bone Marrow Transplantation,* 2002, vol. 29, 197-204
- *The Journal of Clinical Investigation,* 2002, vol. 110 (3), 389-394
- **ROBERT SUTHERLAND et al.** The ISHAGE guidelines for CD34 cell determination by Flow Cytometry. *the Journal of Hematotherapy,* 1996, vol. 5, 213-226
- **BARNETT D ; GRANGER V ; WHITBY L ; STORIE I ; REILLY JT.** Absolute CD4+ T-lymphocyte and CD34+ stem cell counts by single-platform flow cytometry: the way forward. *Br. J. Haematol.,* September 1999, vol. 106 (4), 1059-62
- **PALACIOS et al.** *Cell,* 1985, vol. 41, 727-734
- **MATTHIAS BALLMAIER et al.** *BLOOD,* 2001, vol. 97 (1), 139
- **PLUCKTHUN.** The Pharmacology of Monoclonal Antibodies. Springer Verlag, 1994, vol. 113, 269-315
- **HOLLIGER P et al.** *Proc. Natal. Acad. Sci. USA,* 1993, vol. 90, 6444-6448
- **HUDSON et al.** *J Immunol. Methods,* 1999, vol. 231, 177-189
- **HOLLIGER, P. et al.** *Protein Engineering,* 1996, vol. 9 (3), 299-305
- **CO, M. S. et al.** *J. Immunol.,* 1994, vol. 152, 2968-2976
- **BETTER, M. ; HORWITZ, A. H.** *Methods Enzymol.,* 1989, vol. 178, 476-496
- **PLUCKTHUN, A. ; SKERRA, A.** *Methods Enzymol.,* 1989, vol. 178, 497-515
- **LAMOYI, E.** *Methods Enzymol.,* 1986, vol. 121, 652-663
- **ROUSSEAUX, J. et al.** *Methods Enzymol.,* 1986, vol. 121, 663-669
- **BIRD, R. E. ; WALKER, B. W.** *Trends Biotechnol.,* 1991, vol. 9, 132-137
- **SATO, K. et al.** *Cancer Res.,* 1993, vol. 53, 851-856
- **HASHIMOTO-GOTOH, T. et al.** *Gene,* 1995, vol. 152, 271-275
- **ZOLLER, MJ ; SMITH, M.** *Methods Enzymol.,* 1983, vol. 100, 468-500
- **KRAMER, W. et al.** *Nucleic Acids Res.,* 1984, vol. 12, 9441-9456
- **KRAMER, W. ; FRITZ HJ.** *Methods Enzymol.,* 1987, vol. 154, 350-367
- **KUNKEL, TA.** *Proc. Natl. Acad. Sci. USA.,* 1985, vol. 82, 488-492
- **KUNKEL.** *Methods Enzymol.,* 1988, vol. 85, 2763-2766
- **MARK, D. F. et al.** *Proc. Natl. Acad. Sci. USA,* 1984, vol. 81, 5662-5666
- **ZOLLER, M. J. ; SMITH, M.** *Nucleic Acids Research,* 1982, vol. 10, 6487-6500
- **WANG, A. et al.** *Science,* vol. 224, 1431-1433
- **DALBADIE-MCFARLAND, G. et al.** *Proc. Natl. Acad. Sci. USA,* 1982, vol. 79, 6409-6413
- **HOPP, T. P. et al.** *BioTechnology,* 1988, vol. 6, 1204-1210
- **KEARNEY JF et al.** *J. Immnol.,* 1979, vol. 123, 1548-1550
- **YELTON DE et al.** *Current Topics in Microbiology and Immunology,* 1978, vol. 81, 1-7

- **KOHLER, G. ; MILSTEIN, C.** *Eur. J. Immunol.,* 1976, vol. 6, 511-519
- **MARGULIES, D. H. et al.** *Cell,* 1976, vol. 8, 405-415
- **SHULMAN, M. et al.** *Nature,* 1978, vol. 276, 269-270
- **DEST. GROTH, S. F. et al.** *J. Immunol.,* 1980, vol. 35, 1-21
- **TROWBRIDGE, I. S.** *J. Exp. Med.,* 1978, vol. 148, 313-323
- **GALFRE, G. et al.** *Nature,* 1979, vol. 277, 131-133
- **KOHLER, G ; MILSTEIN, C.** *Methods Enzymol.,* 1981, vol. 73, 3-46
- **VANDAMME, A. M. et al.** *Eur. J. Biochem.,* 1990, vol. 192, 767-775
- **CHIRGWIN, J. M. et al.** *Biochemistry,* 1979, vol. 18, 5294-5299
- **CHOMCZYNSKI, P. et al.** *Anal. Biochem.,* 1987, vol. 162, 156-159
- **FROHMAN, M. A. et al.** *Proc. Natl. Acad. Sci. USA,* 1988, vol. 85, 8998-9002
- **BELYAVSKY, A. et al.** *Nucleic Acids Res.,* 1989, vol. 17, 2919-2932
- **WARD et al.** *Nature,* 1989, vol. 341, 544-546
- *FASEB J.,* 1992, vol. 6, 2422-2427
- **BETTER et al.** *Science,* 1988, vol. 240, 1041-1043
- **LEI, S. P. et al.** *J. Bacteriol.,* 1987, vol. 169, 4379
- *Nucleic Acids Res.,* 1990, vol. 18 (17), 5322
- **MULLIGAN et al.** *Nature,* 1979, vol. 277, 108
- **MIZUSHIMA et al.** *Nucleic Acids Res.,* 1990, vol. 18, 5322
- *J. Exp. Med.,* 1995, vol. 108, 945
- **VALLE et al.** *Nature,* 1981, vol. 291, 358-340
- **VICKI GLASER.** *SPECTRUM Biotechnology Applications,* 1993
- **EBERT, K.M. et al.** *Bio/Technology,* 1994, vol. 12, 699-702
- **SUSUMU, M. et al.** *Nature,* 1985, vol. 315, 592-594
- **JULIAN K.-C. MA et al.** *Eur. J. Immunol.,* 1994, vol. 24, 131-138
- Strategies for Protein Purification and Characterization: A Laboratory Course Manual. Cold Spring Harbor Laboratory Press, 1996
- *Clinical Pharmacology & Therapeutics.,* 2004, vol. 76 (6), 628
- *Blood,* 2004, vol. 104
- *Blood,* 2005, vol. 106 (11
- **HEIKE, T et al.** Ex vivo expansion of hematopoietic stem cells by cytokines. *Biochimica et Biophysica Acta,* 2002, vol. 1592, 313-321
- **YVETTE VAN HENSBERGEN et al.** Ex vivo culture of human CD34+ cord blood cells with thrombopoietin (TPO) accelerates platelet engraftment in a NOD/SCID mouse model. *Experimental Hematology,* 2006, vol. 34, 943-950

SEQUENCE LISTING

<110> CHUGAI SEIYAKU KABUSHIKI KAISHA

<120> Hematopoietic stem cell proliferation promoter

<130> C1-A0611Y1P

<140> PCT/JP2007/061850
<141> 2007-06-13

<150> JP 2006-165279
<151> 2006-06-14

<150> JP 2006-350553
<151> 2006-12-26

<160> 84

<170> PatentIn version 3.3

<210> 1
<211> 5
<212> PRT
<213> Mus musculus

<400> 1

Asn Ser Trp Met Asn
1               5

<210> 2
<211> 17
<212> PRT
<213> Mus musculus

<400> 2

Arg Ile Tyr Pro Gly Asp Gly Glu Thr Ile Tyr Asn Gly Lys Phe Arg
1 5 10 15

Val

<210> 3
<211> 9
<212> PRT
<213> Mus musculus

<400> 3

Gly Tyr Asp Asp Tyr Ser Phe Ala Tyr
1 5

<210> 4
<211> 16
<212> PRT
<213> Mus musculus

<400> 4

Arg Ser Ser Lys Ser Leu Leu His Ser Asn Gly Asn Thr Tyr Leu Tyr
1 5 10 15

<210> 5
<211> 7
<212> PRT

<213> Mus musculus

<400> 5

Arg Met Ser Asn Leu Ala Ser
1 5

<210> 6
<211> 9
<212> PRT
<213> Mus musculus

<400> 6

Met Gln His Ile Glu Tyr Pro Phe Thr
1 5

<210> 7
<211> 411
<212> DNA
<213> Mus musculus

<220>
<221> CDS
<222> (1)..(411)

<400> 7
atg gaa tgg cct ttg atc ttt ctc ttc ctc ctg tca gga act gca ggt 48
Met Glu Trp Pro Leu Ile Phe Leu Phe Leu Leu Ser Gly Thr Ala Gly
1 5 10 15

gtc cac tcc cag gtt cag ctg cag cag tct gga cct gag ctg gtg aag 96
Val His Ser Gln Val Gln Leu Gln Gln Ser Gly Pro Glu Leu Val Lys

20 25 30

cct ggg gcc tca gtg aag att tcc tgc aag gct tct ggc tat gca ttc 144
Pro Gly Ala Ser Val Lys Ile Ser Cys Lys Ala Ser Gly Tyr Ala Phe
35 40 45

act aac tcc tgg atg aac tgg gtg aag cag agg cct gga aag ggt ctt 192
Thr Asn Ser Trp Met Asn Trp Val Lys Gln Arg Pro Gly Lys Gly Leu
50 55 60

gag tgg att gga cgg att tat cct gga gat gga gaa act atc tac aat 240
Glu Trp Ile Gly Arg Ile Tyr Pro Gly Asp Gly Glu Thr Ile Tyr Asn
65 70 75 80

ggg aaa ttc agg gtc aag gcc aca ctg act gca gac aaa tcc tcc agc 288
Gly Lys Phe Arg Val Lys Ala Thr Leu Thr Ala Asp Lys Ser Ser Ser
85 90 95

aca gcc tac atg gat atc agc agc ctg aca tct gag gac tct gcg gtc 336
Thr Ala Tyr Met Asp Ile Ser Ser Leu Thr Ser Glu Asp Ser Ala Val
100 105 110

tac ttc tgt gca aga ggc tat gat gat tac tcg ttt gct tac tgg ggc 384
Tyr Phe Cys Ala Arg Gly Tyr Asp Asp Tyr Ser Phe Ala Tyr Trp Gly
115 120 125

caa ggg act ctg gtc act gtc tct gca 411
Gln Gly Thr Leu Val Thr Val Ser Ala
130 135

<210> 8
<211> 137
<212> PRT
<213> Mus musculus

<400> 8

Met Glu Trp Pro Leu Ile Phe Leu Phe Leu Leu Ser Gly Thr Ala Gly
1 5 10 15

Val His Ser Gln Val Gln Leu Gln Gln Ser Gly Pro Glu Leu Val Lys
20 25 30

Pro Gly Ala Ser Val Lys Ile Ser Cys Lys Ala Ser Gly Tyr Ala Phe
35 40 45

Thr Asn Ser Trp Met Asn Trp Val Lys Gln Arg Pro Gly Lys Gly Leu
50 55 60

Glu Trp Ile Gly Arg Ile Tyr Pro Gly Asp Gly Glu Thr Ile Tyr Asn
65 70 75 80

Gly Lys Phe Arg Val Lys Ala Thr Leu Thr Ala Asp Lys Ser Ser Ser
85 90 95

Thr Ala Tyr Met Asp Ile Ser Ser Leu Thr Ser Glu Asp Ser Ala Val
100 105 110

Tyr Phe Cys Ala Arg Gly Tyr Asp Asp Tyr Ser Phe Ala Tyr Trp Gly
115 120 125

Gln Gly Thr Leu Val Thr Val Ser Ala
130 135

<210> 9
<211> 396
<212> DNA
<213> Mus musculus

<220>
<221> CDS
<222> (1)..(396)

<400> 9
atg agg tgc cta gct gag ttc ctg ggg ctg ctt gtg ttc tgg att cct 48
Met Arg Cys Leu Ala Glu Phe Leu Gly Leu Leu Val Phe Trp Ile Pro
1 5 10 15

gga gcc att ggg gat att gtg atg act cag gct gca ccc tct ata cct 96
Gly Ala Ile Gly Asp Ile Val Met Thr Gln Ala Ala Pro Ser Ile Pro
20 25 30

gtc act cct gga gag tca gta tcc atc tcc tgt agg tct agt aag agt 144
Val Thr Pro Gly Glu Ser Val Ser Ile Ser Cys Arg Ser Ser Lys Ser
35 40 45

ctc ctg cat agt aat ggc aac act tac ttg tat tgg ttc ctg cag agg 192
Leu Leu His Ser Asn Gly Asn Thr Tyr Leu Tyr Trp Phe Leu Gln Arg
50 55 60

cca ggc cag tct cct caa ctc ctg ata tat cgg atg tcc aac ctt gcc 240
Pro Gly Gln Ser Pro Gln Leu Leu Ile Tyr Arg Met Ser Asn Leu Ala
65 70 75 80

tca gga gtc cca gat agg ttc agt ggc agt ggg tca gga act gct ttc 288
Ser Gly Val Pro Asp Arg Phe Ser Gly Ser Gly Ser Gly Thr Ala Phe
85 90 95

aca ctg aga atc agt aga gtg gag gct gag gat gtg ggt gtt tat tac 336
Thr Leu Arg Ile Ser Arg Val Glu Ala Glu Asp Val Gly Val Tyr Tyr
100 105 110

tgt atg caa cat ata gaa tat cct ttt acg ttc gga tcg ggg acc aag 384
Cys Met Gln His Ile Glu Tyr Pro Phe Thr Phe Gly Ser Gly Thr Lys
115 120 125

ctg gaa ata aaa 396
Leu Glu Ile Lys
130

<210> 10
<211> 132
<212> PRT
<213> Mus musculus

<400> 10

Met Arg Cys Leu Ala Glu Phe Leu Gly Leu Leu Val Phe Trp Ile Pro
1 5 10 15

Gly Ala Ile Gly Asp Ile Val Met Thr Gln Ala Ala Pro Ser Ile Pro
20 25 30

Val Thr Pro Gly Glu Ser Val Ser Ile Ser Cys Arg Ser Ser Lys Ser
35 40 45

Leu Leu His Ser Asn Gly Asn Thr Tyr Leu Tyr Trp Phe Leu Gln Arg
50 55 60

Pro Gly Gln Ser Pro Gln Leu Leu Ile Tyr Arg Met Ser Asn Leu Ala
65 70 75 80

Ser Gly Val Pro Asp Arg Phe Ser Gly Ser Gly Ser Gly Thr Ala Phe
85 90 95

Thr Leu Arg Ile Ser Arg Val Glu Ala Glu Asp Val Gly Val Tyr Tyr
100 105 110

Cys Met Gln His Ile Glu Tyr Pro Phe Thr Phe Gly Ser Gly Thr Lys
115 120 125

Leu Glu Ile Lys
130

<210> 11
<211> 762
<212> DNA
<213> Mus musculus

<400> 11
atggaatggc ctttgatctt tctcttcctc ctgtcaggaa ctgcaggtgt ccactcccag 60

gttcagctgc agcagtctgg acctgagctg gtgaagcctg gggcctcagt gaagatttcc 120

tgcaaggctt ctggctatgc attcactaac tcctggatga actgggtgaa gcagaggcct 180

ggaaagggtc ttgagtggat tggacggatt tatcctggag atggagaaac tatctacaat 240

gggaaattca gggtcaaggc cacactgact gcagacaaat cctccagcac agcctacatg 300

gatatcagca gcctgacatc tgaggactct gcggtctact tctgtgcaag aggctatgat 360

gattactcgt ttgcttactg ggccaaggg actctggtca ctgtctctgc aggtggtggt 420

ggttcggata ttgtgatgac tcaggctgca ccctctatac ctgtcactcc tggagagtca 480

gtatccatct cctgtaggtc tagtaagagt ctcctgcata gtaatggcaa cacttacttg 540

tattggttcc tgcagaggcc aggccagtct cctcaactcc tgatatatcg gatgtccaac 600

cttgcctcag gagtcccaga taggttcagt ggcagtgggt caggaactgc tttcacactg 660

agaatcagta gagtggaggc tgaggatgtg ggtgtttatt actgtatgca acatatagaa 720

tatcctttta cgttcggatc ggggaccaag ctggaaataa aa 762

<210> 12
<211> 254
<212> PRT
<213> Mus musculus

<400> 12

Met Glu Trp Pro Leu Ile Phe Leu Phe Leu Leu Ser Gly Thr Ala Gly
1 5 10 15

Val His Ser Gln Val Gln Leu Gln Gln Ser Gly Pro Glu Leu Val Lys
20 25 30

Pro Gly Ala Ser Val Lys Ile Ser Cys Lys Ala Ser Gly Tyr Ala Phe
35 40 45

Thr Asn Ser Trp Met Asn Trp Val Lys Gln Arg Pro Gly Lys Gly Leu
50 55 60

Glu Trp Ile Gly Arg Ile Tyr Pro Gly Asp Gly Glu Thr Ile Tyr Asn
65 70 75 80

Gly Lys Phe Arg Val Lys Ala Thr Leu Thr Ala Asp Lys Ser Ser Ser
85 90 95

Thr Ala Tyr Met Asp Ile Ser Ser Leu Thr Ser Glu Asp Ser Ala Val
100 105 110

Tyr Phe Cys Ala Arg Gly Tyr Asp Asp Tyr Ser Phe Ala Tyr Trp Gly
115 120 125

Gln Gly Thr Leu Val Thr Val Ser Ala Gly Gly Gly Gly Ser Asp Ile
130 135 140

Val Met Thr Gln Ala Ala Pro Ser Ile Pro Val Thr Pro Gly Glu Ser
145 150 155 160

Val Ser Ile Ser Cys Arg Ser Ser Lys Ser Leu Leu His Ser Asn Gly
165 170 175

Asn Thr Tyr Leu Tyr Trp Phe Leu Gln Arg Pro Gly Gln Ser Pro Gln
180 185 190

Leu Leu Ile Tyr Arg Met Ser Asn Leu Ala Ser Gly Val Pro Asp Arg
195 200 205

Phe Ser Gly Ser Gly Ser Gly Thr Ala Phe Thr Leu Arg Ile Ser Arg
210 215 220

Val Glu Ala Glu Asp Val Gly Val Tyr Tyr Cys Met Gln His Ile Glu
225 230 235 240

Tyr Pro Phe Thr Phe Gly Ser Gly Thr Lys Leu Glu Ile Lys
245 250

<210> 13
<211> 1572
<212> DNA
<213> Mus musculus

<400> 13
atggaatggc ctttgatctt tctcttcctc ctgtcaggaa ctgcaggtgt ccactcccag 60

gttcagctgc agcagtctgg acctgagctg gtgaagcctg gggcctcagt gaagatttcc 120

tgcaaggctt ctggctatgc attcactaac tcctggatga actgggtgaa gcagaggcct 180

ggaaagggtc ttgagtggat tggacggatt tatcctggag atggagaaac tatctacaat 240

gggaaattca gggtcaaggc cacactgact gcagacaaat cctccagcac agcctacatg 300

gatatcagca gcctgacatc tgaggactct gcggtctact tctgtgcaag aggctatgat 360

gattactcgt ttgcttactg gggccaaggg actctggtca ctgtctctgc aggtggtggt 420

ggttcgggtg gtggtggttc gggtggtggc ggatcggata ttgtgatgac tcaggctgca 480

ccctctatac ctgtcactcc tggagagtca gtatccatct cctgtaggtc tagtaagagt 540

ctcctgcata gtaatggcaa cacttacttg tattggttcc tgcagaggcc aggccagtct 600

cctcaactcc tgatatatcg gatgtccaac cttgcctcag gagtcccaga taggttcagt 660

ggcagtgggt caggaactgc tttcacactg agaatcagta gagtggaggc tgaggatgtg 720

ggtgtttatt actgtatgca acatatagaa tatcctttta cgttcggatc ggggaccaag 780

ctggaaataa aaggaggtgg tggatcgggt ggtggtggtt cgggaggcgg tggatcgcag 840

gttcagctgc agcagtctgg acctgagctg gtgaagcctg gggcctcagt gaagatttcc 900

tgcaaggctt ctggctatgc attcactaac tcctggatga actgggtgaa gcagaggcct 960

ggaaagggtc ttgagtggat tggacggatt tatcctggag atggagaaac tatctacaat 1020

gggaaattca gggtcaaggc cacactgact gcagacaaat cctccagcac agcctacatg 1080

gatatcagca gcctgacatc tgaggactct gcggtctact tctgtgcaag aggctatgat 1140

gattactcgt ttgcttactg gggccaaggg actctggtca ctgtctctgc aggtggtggt 1200

ggttcgggtg gtggtggttc gggtggtggc ggatcggata ttgtgatgac tcaggctgca 1260

ccctctatac ctgtcactcc tggagagtca gtatccatct cctgtaggtc tagtaagagt 1320

ctcctgcata gtaatggcaa cacttacttg tattggttcc tgcagaggcc aggccagtct 1380

cctcaactcc tgatatatcg gatgtccaac cttgcctcag gagtcccaga taggttcagt 1440

ggcagtgggt caggaactgc tttcacactg agaatcagta gagtggaggc tgaggatgtg 1500

ggtgtttatt actgtatgca acatatagaa tatcctttta cgttcggatc ggggaccaag 1560

ctggaaataa aa 1572

<210> 14
<211> 524
<212> PRT
<213> Mus musculus

<400> 14

Met Glu Trp Pro Leu Ile Phe Leu Phe Leu Leu Ser Gly Thr Ala Gly
1 5 10 15

Val His Ser Gln Val Gln Leu Gln Gln Ser Gly Pro Glu Leu Val Lys
20 25 30

Pro Gly Ala Ser Val Lys Ile Ser Cys Lys Ala Ser Gly Tyr Ala Phe
35 40 45

Thr Asn Ser Trp Met Asn Trp Val Lys Gln Arg Pro Gly Lys Gly Leu
50 55 60

Glu Trp Ile Gly Arg Ile Tyr Pro Gly Asp Gly Glu Thr Ile Tyr Asn
65 70 75 80

Gly Lys Phe Arg Val Lys Ala Thr Leu Thr Ala Asp Lys Ser Ser Ser
85 90 95

Thr Ala Tyr Met Asp Ile Ser Ser Leu Thr Ser Glu Asp Ser Ala Val
100 105 110

Tyr Phe Cys Ala Arg Gly Tyr Asp Asp Tyr Ser Phe Ala Tyr Trp Gly
115 120 125

Gln Gly Thr Leu Val Thr Val Ser Ala Gly Gly Gly Gly Ser Gly Gly
130 135 140

Gly Gly Ser Gly Gly Gly Gly Ser Asp Ile Val Met Thr Gln Ala Ala
145 150 155 160

Pro Ser Ile Pro Val Thr Pro Gly Glu Ser Val Ser Ile Ser Cys Arg
165 170 175

Ser Ser Lys Ser Leu Leu His Ser Asn Gly Asn Thr Tyr Leu Tyr Trp
180 185 190

Phe Leu Gln Arg Pro Gly Gln Ser Pro Gln Leu Leu Ile Tyr Arg Met
195 200 205

Ser Asn Leu Ala Ser Gly Val Pro Asp Arg Phe Ser Gly Ser Gly Ser
210 215 220

Gly Thr Ala Phe Thr Leu Arg Ile Ser Arg Val Glu Ala Glu Asp Val
225 230 235 240

Gly Val Tyr Tyr Cys Met Gln His Ile Glu Tyr Pro Phe Thr Phe Gly
245 250 255

Ser Gly Thr Lys Leu Glu Ile Lys Gly Gly Gly Gly Ser Gly Gly Gly
260 265 270

Gly Ser Gly Gly Gly Gly Ser Gln Val Gln Leu Gln Gln Ser Gly Pro
275 280 285

Glu Leu Val Lys Pro Gly Ala Ser Val Lys Ile Ser Cys Lys Ala Ser
290 295 300

Gly Tyr Ala Phe Thr Asn Ser Trp Met Asn Trp Val Lys Gln Arg Pro
305 310 315 320

Gly Lys Gly Leu Glu Trp Ile Gly Arg Ile Tyr Pro Gly Asp Gly Glu
325 330 335

Thr Ile Tyr Asn Gly Lys Phe Arg Val Lys Ala Thr Leu Thr Ala Asp
340 345 350

Lys Ser Ser Ser Thr Ala Tyr Met Asp Ile Ser Ser Leu Thr Ser Glu
355 360 365

Asp Ser Ala Val Tyr Phe Cys Ala Arg Gly Tyr Asp Asp Tyr Ser Phe
370 375 380

Ala Tyr Trp Gly Gln Gly Thr Leu Val Thr Val Ser Ala Gly Gly Gly
385 390 395 400

Gly Ser Gly Gly Gly Gly Ser Gly Gly Gly Gly Ser Asp Ile Val Met
405 410 415

Thr Gln Ala Ala Pro Ser Ile Pro Val Thr Pro Gly Glu Ser Val Ser
420 425 430

Ile Ser Cys Arg Ser Ser Lys Ser Leu Leu His Ser Asn Gly Asn Thr
435 440 445

Tyr Leu Tyr Trp Phe Leu Gln Arg Pro Gly Gln Ser Pro Gln Leu Leu
450 455 460

Ile Tyr Arg Met Ser Asn Leu Ala Ser Gly Val Pro Asp Arg Phe Ser
465 470 475 480

Gly Ser Gly Ser Gly Thr Ala Phe Thr Leu Arg Ile Ser Arg Val Glu
485 490 495

Ala Glu Asp Val Gly Val Tyr Tyr Cys Met Gln His Ile Glu Tyr Pro
500 505 510

Phe Thr Phe Gly Ser Gly Thr Lys Leu Glu Ile Lys
515 520

<210> 15
<211> 30
<212> PRT
<213> Homo sapiens

<400> 15

Gln Val Gln Leu Val Gln Ser Gly Pro Glu Val Lys Lys Pro Gly Ala
1 5 10 15

Ser Val Lys Val Ser Cys Lys Ala Ser Gly Tyr Thr Phe Thr
20 25 30

<210> 16
<211> 14
<212> PRT
<213> Homo sapiens

<400> 16

Trp Val Arg Gln Arg Pro Gly Lys Gly Leu Glu Trp Met Gly
1 5 10

<210> 17
<211> 32
<212> PRT
<213> Homo sapiens

<400> 17

Arg Val Thr Ile Thr Ala Asp Glu Ser Thr Ser Thr Ala Tyr Met Glu
1 5 10 15

Leu Ser Ser Leu Arg Ser Glu Asp Thr Ala Val Tyr Tyr Cys Ala Arg
20 25 30

<210> 18
<211> 11
<212> PRT
<213> Homo sapiens

<400> 18

Trp Gly Gln Gly Thr Thr Val Thr Val Ser Ser
1 5 10

<210> 19
<211> 30
<212> PRT
<213> Homo sapiens

<400> 19

Gln Val Gln Leu Val Gln Ser Gly Pro Glu Val Lys Lys Pro Gly Ala
1 5 10 15

Ser Val Lys Val Ser Cys Lys Ala Ser Gly Tyr Thr Phe Thr
20 25 30

<210> 20
<211> 14
<212> PRT
<213> Homo sapiens

<400> 20

Trp Val Arg Gln Arg Pro Gly Lys Gly Leu Glu Trp Val Gly
1 5 10

<210> 21
<211> 32
<212> PRT
<213> Homo sapiens

<400> 21

Arg Val Thr Ile Thr Ala Asp Glu Ser Thr Ser Thr Ala Tyr Met Glu
1 5 10 15

Leu Ser Ser Leu Arg Ser Glu Asp Thr Ala Val Tyr Tyr Cys Ala Arg
20 25 30

<210> 22
<211> 11
<212> PRT
<213> Homo sapiens

<400> 22

Trp Gly Gln Gly Thr Thr Val Thr Val Ser Ser
1 5 10

<210> 23
<211> 30
<212> PRT

<213> Homo sapiens

<400> 23

Gln Val Gln Leu Val Gln Ser Gly Pro Glu Val Lys Lys Pro Gly Ala
1 5 10 15

Ser Val Lys Val Ser Cys Lys Ala Ser Gly Tyr Thr Phe Thr
20 25 30

<210> 24
<211> 14
<212> PRT
<213> Homo sapiens

<400> 24

Trp Ile Arg Gln Arg Pro Gly Lys Gly Leu Glu Trp Ile Gly
1 5 10

<210> 25
<211> 32
<212> PRT
<213> Homo sapiens

<400> 25

Arg Val Thr Ile Thr Ala Asp Glu Ser Thr Ser Thr Ala Tyr Met Glu
1 5 10 15

Leu Ser Ser Leu Arg Ser Glu Asp Thr Ala Val Tyr Tyr Cys Ala Arg
20 25 30

<210> 26
<211> 11
<212> PRT
<213> Homo sapiens

<400> 26

Trp Gly Gln Gly Thr Leu Val Thr Val Ser Ser
1 5 10

<210> 27
<211> 23
<212> PRT
<213> Homo sapiens

<400> 27

Asp Ile Val Met Thr Gln Ser Ala Leu Ser Leu Pro Val Thr Pro Gly
1 5 10 15

Glu Pro Ala Ser Ile Ser Cys
20

<210> 28
<211> 15
<212> PRT
<213> Homo sapiens

<400> 28

Trp Phe Gln Gln Lys Pro Gly Gln Ser Pro Gln Leu Leu Ile Tyr

1 5 10 15

<210> 29
<211> 32
<212> PRT
<213> Homo sapiens

<400> 29

Gly Val Pro Asp Arg Phe Ser Gly Ser Gly Ser Gly Thr Ala Phe Thr
1 5 10 15

Leu Lys Ile Ser Arg Val Glu Ala Glu Asp Val Gly Val Tyr Tyr Cys
20 25 30

<210> 30
<211> 10
<212> PRT
<213> Homo sapiens

<400> 30

Phe Gly Gln Gly Thr Lys Leu Glu Ile Lys
1 5 10

<210> 31
<211> 23
<212> PRT
<213> Homo sapiens

<400> 31

Asp Ile Val Met Thr Gln Ser Ala Leu Ser Leu Pro Val Thr Pro Gly
1               5                   10                  15

Glu Pro Ala Ser Ile Ser Cys
            20

<210> 32
<211> 15
<212> PRT
<213> Homo sapiens

<400> 32

Trp Tyr Leu Gln Lys Pro Gly Gln Ser Pro Gln Leu Leu Ile Tyr
1               5                   10                  15

<210> 33
<211> 32
<212> PRT
<213> Homo sapiens

<400> 33

Gly Val Pro Asp Arg Phe Ser Gly Ser Gly Ser Gly Thr Ala Phe Thr
1               5                   10                  15

Leu Lys Ile Ser Arg Val Glu Ala Glu Asp Val Gly Val Tyr Tyr Cys
            20                  25                  30

<210> 34
<211> 10

<212> PRT
<213> Homo sapiens

<400> 34

Phe Gly Gln Gly Thr Lys Leu Glu Ile Lys
1 5 10

<210> 35
<211> 354
<212> DNA
<213> Homo sapiens

<400> 35
caggtgcagc tggtgcagtc tggacctgag gtgaagaagc ctggggcctc agtgaaggtc 60

tcctgcaagg cttctggata caccttcacc aactcctgga tgaactgggt gaggcagagg 120

cctggaaagg gtcttgagtg gatgggacgg atttatcctg gagatggaga aactatctac 180

aatgggaaat tcagggtcag agtcacgatt accgcggacg aatccacgag cacagcctac 240

atggagctga gcagcctgag atctgaggac acggccgtgt attactgtgc gagaggctat 300

gatgattact cgtttgctta ctggggccag ggaaccacgg tcaccgtctc ttca 354

<210> 36
<211> 118
<212> PRT
<213> Homo sapiens

<400> 36

Gln Val Gln Leu Val Gln Ser Gly Pro Glu Val Lys Lys Pro Gly Ala

1 5 10 15

Ser Val Lys Val Ser Cys Lys Ala Ser Gly Tyr Thr Phe Thr Asn Ser
20 25 30

Trp Met Asn Trp Val Arg Gln Arg Pro Gly Lys Gly Leu Glu Trp Met
35 40 45

Gly Arg Ile Tyr Pro Gly Asp Gly Glu Thr Ile Tyr Asn Gly Lys Phe
50 55 60

Arg Val Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Gly Tyr Asp Asp Tyr Ser Phe Ala Tyr Trp Gly Gln Gly Thr
100 105 110

Thr Val Thr Val Ser Ser
115

<210> 37
<211> 354
<212> DNA
<213> Homo sapiens

<400> 37

caggtgcagc tggtgcagtc tggacctgag gtgaagaagc ctggggcctc agtgaaggtc 60

tcctgcaagg cttctggata caccttcacc aactcctgga tgaactgggt gaggcagagg 120

cctggaaagg gtcttgagtg ggttggacgg atttatcctg gagatggaga aactatctac 180

aatgggaaat tcagggtcag agtcacgatt accgcggacg aatccacgag cacagcctac 240

atggagctga gcagcctgag atctgaggac acggccgtgt attactgtgc gagaggctat 300

gatgattact cgtttgctta ctggggccag ggaaccacgg tcaccgtctc ttca 354

<210> 38

<211> 118

<212> PRT

<213> Homo sapiens

<400> 38

Gln Val Gln Leu Val Gln Ser Gly Pro Glu Val Lys Lys Pro Gly Ala
1 5 10 15

Ser Val Lys Val Ser Cys Lys Ala Ser Gly Tyr Thr Phe Thr Asn Ser
20 25 30

Trp Met Asn Trp Val Arg Gln Arg Pro Gly Lys Gly Leu Glu Trp Val
35 40 45

Gly Arg Ile Tyr Pro Gly Asp Gly Glu Thr Ile Tyr Asn Gly Lys Phe
50 55 60

Arg Val Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Gly Tyr Asp Asp Tyr Ser Phe Ala Tyr Trp Gly Gln Gly Thr
100 105 110

Thr Val Thr Val Ser Ser
115

<210> 39
<211> 354
<212> DNA
<213> Homo sapiens

<400> 39
caggtgcagc tggtgcagtc tggacctgag gtgaagaagc ctggggcctc agtgaaggtc 60

tcctgcaagg cttctggata caccttcacc aactcctgga tgaactggat caggcagagg 120

cctggaaagg gtcttgagtg gattggacgg atttatcctg gagatggaga aactatctac 180

aatgggaaat tcagggtcag agtcacgatt accgcggacg aatccacgag cacagcctac 240

atggagctga gcagcctgag atctgaggac acggccgtgt attactgtgc gagaggctat 300

gatgattact cgtttgctta ctggggccag ggaaccctgg tcaccgtctc ttca 354

<210> 40
<211> 118
<212> PRT
<213> Homo sapiens

<400> 40

Gln Val Gln Leu Val Gln Ser Gly Pro Glu Val Lys Lys Pro Gly Ala
1 5 10 15

Ser Val Lys Val Ser Cys Lys Ala Ser Gly Tyr Thr Phe Thr Asn Ser
20 25 30

Trp Met Asn Trp Ile Arg Gln Arg Pro Gly Lys Gly Leu Glu Trp Ile
35 40 45

Gly Arg Ile Tyr Pro Gly Asp Gly Glu Thr Ile Tyr Asn Gly Lys Phe
50 55 60

Arg Val Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Gly Tyr Asp Asp Tyr Ser Phe Ala Tyr Trp Gly Gln Gly Thr
100 105 110

Leu Val Thr Val Ser Ser

115

<210> 41
<211> 336
<212> DNA
<213> Homo sapiens

<400> 41
gatattgtga tgactcagtc tgcactctcc ctgcccgtca cccctggaga gccggcctcc 60

atctcctgca ggtctagtaa gagtctcctg catagtaatg gcaacactta cttgtattgg 120

ttccagcaga agccagggca gtctccacag ctcctgatct atcggatgtc caaccttgcc 180

tcaggggtcc ctgacaggtt cagtggcagt ggatcaggca cagcttttac actgaaaatc 240

agcagagtgg aggctgagga tgttggggtt tattactgca tgcaacatat agaatatcct 300

tttacgttcg gccaagggac caaactggaa atcaaa 336

<210> 42
<211> 112
<212> PRT
<213> Homo sapiens

<400> 42

Asp Ile Val Met Thr Gln Ser Ala Leu Ser Leu Pro Val Thr Pro Gly
1 5 10 15

Glu Pro Ala Ser Ile Ser Cys Arg Ser Ser Lys Ser Leu Leu His Ser
20 25 30

Asn Gly Asn Thr Tyr Leu Tyr Trp Phe Gln Gln Lys Pro Gly Gln Ser
35 40 45

Pro Gln Leu Leu Ile Tyr Arg Met Ser Asn Leu Ala Ser Gly Val Pro
50 55 60

Asp Arg Phe Ser Gly Ser Gly Ser Gly Thr Ala Phe Thr Leu Lys Ile
65 70 75 80

Ser Arg Val Glu Ala Glu Asp Val Gly Val Tyr Tyr Cys Met Gln His
85 90 95

Ile Glu Tyr Pro Phe Thr Phe Gly Gln Gly Thr Lys Leu Glu Ile Lys
100 105 110

<210> 43
<211> 336
<212> DNA
<213> Homo sapiens

<400> 43
gatattgtga tgactcagtc tgcactctcc ctgcccgtca cccctggaga gccggcctcc 60

atctcctgca ggtctagtaa gagtctcctg catagtaatg gcaacactta cttgtattgg 120

tacctgcaga agccagggca gtctccacag ctcctgatct atcggatgtc caaccttgcc 180

tcaggggtcc ctgacaggtt cagtggcagt ggatcaggca cagcttttac actgaaaatc 240

agcagagtgg aggctgagga tgttggggtt tattactgca tgcaacatat agaatatcct 300

tttacgttcg gccaagggac caaactggaa atcaaa 336

<210> 44
<211> 112
<212> PRT
<213> Homo sapiens

<400> 44

Asp Ile Val Met Thr Gln Ser Ala Leu Ser Leu Pro Val Thr Pro Gly
1 5 10 15

Glu Pro Ala Ser Ile Ser Cys Arg Ser Ser Lys Ser Leu Leu His Ser
20 25 30

Asn Gly Asn Thr Tyr Leu Tyr Trp Tyr Leu Gln Lys Pro Gly Gln Ser
35 40 45

Pro Gln Leu Leu Ile Tyr Arg Met Ser Asn Leu Ala Ser Gly Val Pro
50 55 60

Asp Arg Phe Ser Gly Ser Gly Ser Gly Thr Ala Phe Thr Leu Lys Ile
65 70 75 80

Ser Arg Val Glu Ala Glu Asp Val Gly Val Tyr Tyr Cys Met Gln His
85 90 95

Ile Glu Tyr Pro Phe Thr Phe Gly Gln Gly Thr Lys Leu Glu Ile Lys

100 105 110

<210> 45
<211> 1572
<212> DNA
<213> Homo sapiens

<400> 45
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtggat gggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctgca 480

ctctccctgc ccgtcacccc tggagagccg gcctccatct cctgcaggtc tagtaagagt 540

ctcctgcata gtaatggcaa cacttacttg tattggttcc agcagaagcc agggcagtct 600

ccacagctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat gggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg ggccagggga accacggtca ccgtctcttc aggtggtggt 1200

ggatccggag gtggtggatc gggtggtgga ggatcggata ttgtgatgac tcagtctgca 1260

ctctccctgc ccgtcacccc tggagagccg gcctccatct cctgcaggtc tagtaagagt 1320

ctcctgcata gtaatggcaa cacttacttg tattggttcc agcagaagcc agggcagtct 1380

ccacagctcc tgatctatcg gatgtccaac cttgcctcag ggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 46
<211> 524
<212> PRT
<213> Homo sapiens

<400> 46

Met Asp Trp Thr Trp Arg Phe Leu Phe Val Val Ala Ala Ala Thr Gly

1 5 10 15

Val Gln Ser Gln Val Gln Leu Val Gln Ser Gly Pro Glu Val Lys Lys
20 25 30

Pro Gly Ala Ser Val Lys Val Ser Cys Lys Ala Ser Gly Tyr Thr Phe
35 40 45

Thr Asn Ser Trp Met Asn Trp Val Arg Gln Arg Pro Gly Lys Gly Leu
50 55 60

Glu Trp Met Gly Arg Ile Tyr Pro Gly Asp Gly Glu Thr Ile Tyr Asn
65 70 75 80

Gly Lys Phe Arg Val Arg Val Thr Ile Thr Ala Asp Glu Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Tyr Asp Asp Tyr Ser Phe Ala Tyr Trp Gly
115 120 125

Gln Gly Thr Thr Val Thr Val Ser Ser Gly Gly Gly Gly Ser Gly Gly
130 135 140

Gly Gly Ser Gly Gly Gly Gly Ser Asp Ile Val Met Thr Gln Ser Ala

145 150 155 160

Leu Ser Leu Pro Val Thr Pro Gly Glu Pro Ala Ser Ile Ser Cys Arg
165 170 175

Ser Ser Lys Ser Leu Leu His Ser Asn Gly Asn Thr Tyr Leu Tyr Trp
180 185 190

Phe Gln Gln Lys Pro Gly Gln Ser Pro Gln Leu Leu Ile Tyr Arg Met
195 200 205

Ser Asn Leu Ala Ser Gly Val Pro Asp Arg Phe Ser Gly Ser Gly Ser
210 215 220

Gly Thr Ala Phe Thr Leu Lys Ile Ser Arg Val Glu Ala Glu Asp Val
225 230 235 240

Gly Val Tyr Tyr Cys Met Gln His Ile Glu Tyr Pro Phe Thr Phe Gly
245 250 255

Gln Gly Thr Lys Leu Glu Ile Lys Gly Gly Gly Gly Ser Gly Gly Gly
260 265 270

Gly Ser Gly Gly Gly Gly Ser Gln Val Gln Leu Val Gln Ser Gly Pro
275 280 285

Glu Val Lys Lys Pro Gly Ala Ser Val Lys Val Ser Cys Lys Ala Ser

290 295 300

Gly Tyr Thr Phe Thr Asn Ser Trp Met Asn Trp Val Arg Gln Arg Pro
305 310 315 320

Gly Lys Gly Leu Glu Trp Met Gly Arg Ile Tyr Pro Gly Asp Gly Glu
325 330 335

Thr Ile Tyr Asn Gly Lys Phe Arg Val Arg Val Thr Ile Thr Ala Asp
340 345 350

Glu Ser Thr Ser Thr Ala Tyr Met Glu Leu Ser Ser Leu Arg Ser Glu
355 360 365

Asp Thr Ala Val Tyr Tyr Cys Ala Arg Gly Tyr Asp Asp Tyr Ser Phe
370 375 380

Ala Tyr Trp Gly Gln Gly Thr Thr Val Thr Val Ser Ser Gly Gly Gly
385 390 395 400

Gly Ser Gly Gly Gly Gly Ser Gly Gly Gly Gly Ser Asp Ile Val Met
405 410 415

Thr Gln Ser Ala Leu Ser Leu Pro Val Thr Pro Gly Glu Pro Ala Ser
420 425 430

Ile Ser Cys Arg Ser Ser Lys Ser Leu Leu His Ser Asn Gly Asn Thr

435 440 445

Tyr Leu Tyr Trp Phe Gln Gln Lys Pro Gly Gln Ser Pro Gln Leu Leu
450 455 460

Ile Tyr Arg Met Ser Asn Leu Ala Ser Gly Val Pro Asp Arg Phe Ser
465 470 475 480

Gly Ser Gly Ser Gly Thr Ala Phe Thr Leu Lys Ile Ser Arg Val Glu
485 490 495

Ala Glu Asp Val Gly Val Tyr Tyr Cys Met Gln His Ile Glu Tyr Pro
500 505 510

Phe Thr Phe Gly Gln Gly Thr Lys Leu Glu Ile Lys
515 520

<210> 47
<211> 1572
<212> DNA
<213> Homo sapiens

<400> 47
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtgggt tggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctgca 480

ctctccctgc ccgtcacccc tggagagccg gcctccatct cctgcaggtc tagtaagagt 540

ctcctgcata gtaatggcaa cacttacttg tattggtacc tgcagaagcc agggcagtct 600

ccacagctcc tgatctatcg gatgtccaac cttgcctcag ggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtgggt tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag ggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 48
<211> 524
<212> PRT
<213> Homo sapiens

<400> 48

Met Asp Trp Thr Trp Arg Phe Leu Phe Val Val Ala Ala Ala Thr Gly
1               5                   10                  15

Val Gln Ser Gln Val Gln Leu Val Gln Ser Gly Pro Glu Val Lys Lys
            20                  25                  30

Pro Gly Ala Ser Val Lys Val Ser Cys Lys Ala Ser Gly Tyr Thr Phe
        35                  40                  45

Thr Asn Ser Trp Met Asn Trp Val Arg Gln Arg Pro Gly Lys Gly Leu
    50                  55                  60

Glu Trp Val Gly Arg Ile Tyr Pro Gly Asp Gly Glu Thr Ile Tyr Asn

65 70 75 80

Gly Lys Phe Arg Val Arg Val Thr Ile Thr Ala Asp Glu Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Tyr Asp Asp Tyr Ser Phe Ala Tyr Trp Gly
115 120 125

Gln Gly Thr Thr Val Thr Val Ser Ser Gly Gly Gly Gly Ser Gly Gly
130 135 140

Gly Gly Ser Gly Gly Gly Gly Ser Asp Ile Val Met Thr Gln Ser Ala
145 150 155 160

Leu Ser Leu Pro Val Thr Pro Gly Glu Pro Ala Ser Ile Ser Cys Arg
165 170 175

Ser Ser Lys Ser Leu Leu His Ser Asn Gly Asn Thr Tyr Leu Tyr Trp
180 185 190

Tyr Leu Gln Lys Pro Gly Gln Ser Pro Gln Leu Leu Ile Tyr Arg Met
195 200 205

Ser Asn Leu Ala Ser Gly Val Pro Asp Arg Phe Ser Gly Ser Gly Ser

210 215 220

Gly Thr Ala Phe Thr Leu Lys Ile Ser Arg Val Glu Ala Glu Asp Val
225 230 235 240

Gly Val Tyr Tyr Cys Met Gln His Ile Glu Tyr Pro Phe Thr Phe Gly
245 250 255

Gln Gly Thr Lys Leu Glu Ile Lys Gly Gly Gly Gly Ser Gly Gly Gly
260 265 270

Gly Ser Gly Gly Gly Gly Ser Gln Val Gln Leu Val Gln Ser Gly Pro
275 280 285

Glu Val Lys Lys Pro Gly Ala Ser Val Lys Val Ser Cys Lys Ala Ser
290 295 300

Gly Tyr Thr Phe Thr Asn Ser Trp Met Asn Trp Val Arg Gln Arg Pro
305 310 315 320

Gly Lys Gly Leu Glu Trp Val Gly Arg Ile Tyr Pro Gly Asp Gly Glu
325 330 335

Thr Ile Tyr Asn Gly Lys Phe Arg Val Arg Val Thr Ile Thr Ala Asp
340 345 350

Glu Ser Thr Ser Thr Ala Tyr Met Glu Leu Ser Ser Leu Arg Ser Glu

355 360 365

Asp Thr Ala Val Tyr Tyr Cys Ala Arg Gly Tyr Asp Asp Tyr Ser Phe
370 375 380

Ala Tyr Trp Gly Gln Gly Thr Thr Val Thr Val Ser Ser Gly Gly Gly
385 390 395 400

Gly Ser Gly Gly Gly Gly Ser Gly Gly Gly Gly Ser Asp Ile Val Met
405 410 415

Thr Gln Ser Ala Leu Ser Leu Pro Val Thr Pro Gly Glu Pro Ala Ser
420 425 430

Ile Ser Cys Arg Ser Ser Lys Ser Leu Leu His Ser Asn Gly Asn Thr
435 440 445

Tyr Leu Tyr Trp Tyr Leu Gln Lys Pro Gly Gln Ser Pro Gln Leu Leu
450 455 460

Ile Tyr Arg Met Ser Asn Leu Ala Ser Gly Val Pro Asp Arg Phe Ser
465 470 475 480

Gly Ser Gly Ser Gly Thr Ala Phe Thr Leu Lys Ile Ser Arg Val Glu
485 490 495

Ala Glu Asp Val Gly Val Tyr Tyr Cys Met Gln His Ile Glu Tyr Pro

500 505 510

Phe Thr Phe Gly Gln Gly Thr Lys Leu Glu Ile Lys
515 520

<210> 49
<211> 1572
<212> DNA
<213> Homo sapiens

<400> 49
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actggatcag gcagaggcct 180

ggaaagggtc ttgagtggat tggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accctggtca ccgtctcttc aggtggtggt 420

ggatccggag gtggtggatc gggtggtgga ggatcggata ttgtgatgac tcagtctgca 480

ctctccctgc ccgtcacccc tggagagccg gcctccatct cctgcaggtc tagtaagagt 540

ctcctgcata gtaatggcaa cacttacttg tattggtacc tgcagaagcc agggcagtct 600

ccacagctcc tgatctatcg gatgtccaac cttgcctcag ggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actggatcag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accctggtca ccgtctcttc aggtggtggt 1200

ggatccggag gtggtggatc gggtggtgga ggatcggata ttgtgatgac tcagtctgca 1260

ctctccctgc ccgtcacccc tggagagccg gcctccatct cctgcaggtc tagtaagagt 1320

ctcctgcata gtaatggcaa cacttacttg tattggtacc tgcagaagcc agggcagtct 1380

ccacagctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 50
<211> 524
<212> PRT
<213> Homo sapiens

<400> 50

Met Asp Trp Thr Trp Arg Phe Leu Phe Val Val Ala Ala Ala Thr Gly
1 5 10 15

Val Gln Ser Gln Val Gln Leu Val Gln Ser Gly Pro Glu Val Lys Lys
20 25 30

Pro Gly Ala Ser Val Lys Val Ser Cys Lys Ala Ser Gly Tyr Thr Phe
35 40 45

Thr Asn Ser Trp Met Asn Trp Ile Arg Gln Arg Pro Gly Lys Gly Leu
50 55 60

Glu Trp Ile Gly Arg Ile Tyr Pro Gly Asp Gly Glu Thr Ile Tyr Asn
65 70 75 80

Gly Lys Phe Arg Val Arg Val Thr Ile Thr Ala Asp Glu Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Tyr Asp Asp Tyr Ser Phe Ala Tyr Trp Gly
115 120 125

Gln Gly Thr Leu Val Thr Val Ser Ser Gly Gly Gly Gly Ser Gly Gly

130 135 140

Gly Gly Ser Gly Gly Gly Gly Ser Asp Ile Val Met Thr Gln Ser Ala
145 150 155 160

Leu Ser Leu Pro Val Thr Pro Gly Glu Pro Ala Ser Ile Ser Cys Arg
165 170 175

Ser Ser Lys Ser Leu Leu His Ser Asn Gly Asn Thr Tyr Leu Tyr Trp
180 185 190

Tyr Leu Gln Lys Pro Gly Gln Ser Pro Gln Leu Leu Ile Tyr Arg Met
195 200 205

Ser Asn Leu Ala Ser Gly Val Pro Asp Arg Phe Ser Gly Ser Gly Ser
210 215 220

Gly Thr Ala Phe Thr Leu Lys Ile Ser Arg Val Glu Ala Glu Asp Val
225 230 235 240

Gly Val Tyr Tyr Cys Met Gln His Ile Glu Tyr Pro Phe Thr Phe Gly
245 250 255

Gln Gly Thr Lys Leu Glu Ile Lys Gly Gly Gly Gly Ser Gly Gly Gly
260 265 270

Gly Ser Gly Gly Gly Gly Ser Gln Val Gln Leu Val Gln Ser Gly Pro

275 280 285

Glu Val Lys Lys Pro Gly Ala Ser Val Lys Val Ser Cys Lys Ala Ser
290 295 300

Gly Tyr Thr Phe Thr Asn Ser Trp Met Asn Trp Ile Arg Gln Arg Pro
305 310 315 320

Gly Lys Gly Leu Glu Trp Ile Gly Arg Ile Tyr Pro Gly Asp Gly Glu
325 330 335

Thr Ile Tyr Asn Gly Lys Phe Arg Val Arg Val Thr Ile Thr Ala Asp
340 345 350

Glu Ser Thr Ser Thr Ala Tyr Met Glu Leu Ser Ser Leu Arg Ser Glu
355 360 365

Asp Thr Ala Val Tyr Tyr Cys Ala Arg Gly Tyr Asp Asp Tyr Ser Phe
370 375 380

Ala Tyr Trp Gly Gln Gly Thr Leu Val Thr Val Ser Ser Gly Gly Gly
385 390 395 400

Gly Ser Gly Gly Gly Gly Ser Gly Gly Gly Gly Ser Asp Ile Val Met
405 410 415

Thr Gln Ser Ala Leu Ser Leu Pro Val Thr Pro Gly Glu Pro Ala Ser

420 425 430

Ile Ser Cys Arg Ser Ser Lys Ser Leu Leu His Ser Asn Gly Asn Thr
435 440 445

Tyr Leu Tyr Trp Tyr Leu Gln Lys Pro Gly Gln Ser Pro Gln Leu Leu
450 455 460

Ile Tyr Arg Met Ser Asn Leu Ala Ser Gly Val Pro Asp Arg Phe Ser
465 470 475 480

Gly Ser Gly Ser Gly Thr Ala Phe Thr Leu Lys Ile Ser Arg Val Glu
485 490 495

Ala Glu Asp Val Gly Val Tyr Tyr Cys Met Gln His Ile Glu Tyr Pro
500 505 510

Phe Thr Phe Gly Gln Gly Thr Lys Leu Glu Ile Lys
515 520

<210> 51
<211> 635
<212> PRT
<213> Homo sapiens

<400> 51

Met Pro Ser Trp Ala Leu Phe Met Val Thr Ser Cys Leu Leu Leu Ala
1 5 10 15

Pro Gln Asn Leu Ala Gln Val Ser Ser Gln Asp Val Ser Leu Leu Ala
20 25 30

Ser Asp Ser Glu Pro Leu Lys Cys Phe Ser Arg Thr Phe Glu Asp Leu
35 40 45

Thr Cys Phe Trp Asp Glu Glu Glu Ala Ala Pro Ser Gly Thr Tyr Gln
50 55 60

Leu Leu Tyr Ala Tyr Pro Arg Glu Lys Pro Arg Ala Cys Pro Leu Ser
65 70 75 80

Ser Gln Ser Met Pro His Phe Gly Thr Arg Tyr Val Cys Gln Phe Pro
85 90 95

Asp Gln Glu Glu Val Arg Leu Phe Phe Pro Leu His Leu Trp Val Lys
100 105 110

Asn Val Phe Leu Asn Gln Thr Arg Thr Gln Arg Val Leu Phe Val Asp
115 120 125

Ser Val Gly Leu Pro Ala Pro Pro Ser Ile Ile Lys Ala Met Gly Gly
130 135 140

Ser Gln Pro Gly Glu Leu Gln Ile Ser Trp Glu Glu Pro Ala Pro Glu
145 150 155 160

Ile Ser Asp Phe Leu Arg Tyr Glu Leu Arg Tyr Gly Pro Arg Asp Pro
165 170 175

Lys Asn Ser Thr Gly Pro Thr Val Ile Gln Leu Ile Ala Thr Glu Thr
180 185 190

Cys Cys Pro Ala Leu Gln Arg Pro His Ser Ala Ser Ala Leu Asp Gln
195 200 205

Ser Pro Cys Ala Gln Pro Thr Met Pro Trp Gln Asp Gly Pro Lys Gln
210 215 220

Thr Ser Pro Ser Arg Glu Ala Ser Ala Leu Thr Ala Glu Gly Gly Ser
225 230 235 240

Cys Leu Ile Ser Gly Leu Gln Pro Gly Asn Ser Tyr Trp Leu Gln Leu
245 250 255

Arg Ser Glu Pro Asp Gly Ile Ser Leu Gly Gly Ser Trp Gly Ser Trp
260 265 270

Ser Leu Pro Val Thr Val Asp Leu Pro Gly Asp Ala Val Ala Leu Gly
275 280 285

Leu Gln Cys Phe Thr Leu Asp Leu Lys Asn Val Thr Cys Gln Trp Gln
290 295 300

Gln Gln Asp His Ala Ser Ser Gln Gly Phe Phe Tyr His Ser Arg Ala
305 310 315 320

Arg Cys Cys Pro Arg Asp Arg Tyr Pro Ile Trp Glu Asn Cys Glu Glu
325 330 335

Glu Glu Lys Thr Asn Pro Gly Leu Gln Thr Pro Gln Phe Ser Arg Cys
340 345 350

His Phe Lys Ser Arg Asn Asp Ser Ile Ile His Ile Leu Val Glu Val
355 360 365

Thr Thr Ala Pro Gly Thr Val His Ser Tyr Leu Gly Ser Pro Phe Trp
370 375 380

Ile His Gln Ala Val Arg Leu Pro Thr Pro Asn Leu His Trp Arg Glu
385 390 395 400

Ile Ser Ser Gly His Leu Glu Leu Glu Trp Gln His Pro Ser Ser Trp
405 410 415

Ala Ala Gln Glu Thr Cys Tyr Gln Leu Arg Tyr Thr Gly Glu Gly His
420 425 430

Gln Asp Trp Lys Val Leu Glu Pro Pro Leu Gly Ala Arg Gly Gly Thr
435 440 445

Leu Glu Leu Arg Pro Arg Ser Arg Tyr Arg Leu Gln Leu Arg Ala Arg
450 455 460

Leu Asn Gly Pro Thr Tyr Gln Gly Pro Trp Ser Ser Trp Ser Asp Pro
465 470 475 480

Thr Arg Val Glu Thr Ala Thr Glu Thr Ala Trp Ile Ser Leu Val Thr
485 490 495

Ala Leu His Leu Val Leu Gly Leu Ser Ala Val Leu Gly Leu Leu Leu
500 505 510

Leu Arg Trp Gln Phe Pro Ala His Tyr Arg Arg Leu Arg His Ala Leu
515 520 525

Trp Pro Ser Leu Pro Asp Leu His Arg Val Leu Gly Gln Tyr Leu Arg
530 535 540

Asp Thr Ala Ala Leu Ser Pro Pro Lys Ala Thr Val Ser Asp Thr Cys
545 550 555 560

Glu Glu Val Glu Pro Ser Leu Leu Glu Ile Leu Pro Lys Ser Ser Glu
565 570 575

Arg Thr Pro Leu Pro Leu Cys Ser Ser Gln Ala Gln Met Asp Tyr Arg
580 585 590

Arg Leu Gln Pro Ser Cys Leu Gly Thr Met Pro Leu Ser Val Cys Pro
595 600 605

Pro Met Ala Glu Ser Gly Ser Cys Cys Thr Thr His Ile Ala Asn His
610 615 620

Ser Tyr Leu Pro Leu Ser Tyr Trp Gln Gln Pro
625 630 635

<210> 52
<211> 1924
<212> DNA
<213> Macaca fascicularis

<220>
<221> CDS
<222> (11)..(1918)

<400> 52
gaattccacc atg ccc tcc tgg gcc ctc ttc atg gtc acc tcc tgc ctc 49
Met Pro Ser Trp Ala Leu Phe Met Val Thr Ser Cys Leu
1 5 10

ctc ctg gcc cct caa aac ctg gcc caa gtc agc agc caa gat gtc tcc 97
Leu Leu Ala Pro Gln Asn Leu Ala Gln Val Ser Ser Gln Asp Val Ser
15 20 25

ttg ctg gcc tcg gac tca gag ccc ctg aag tgt ttc tcc cga aca ttt 145
Leu Leu Ala Ser Asp Ser Glu Pro Leu Lys Cys Phe Ser Arg Thr Phe
30 35 40 45

```
gag gac ctc act tgc ttc tgg gat gag gaa gag gca gca ccc agt ggg      193
Glu Asp Leu Thr Cys Phe Trp Asp Glu Glu Glu Ala Ala Pro Ser Gly
                50                  55                  60

aca tac cag ctg ctg tat gcc tac ccg ggg gag aag ccc cgt gcc tgc      241
Thr Tyr Gln Leu Leu Tyr Ala Tyr Pro Gly Glu Lys Pro Arg Ala Cys
            65                  70                  75

ccc ctg agt tct cag agc gtg ccc cgc ttt gga acc cga tac gtg tgc      289
Pro Leu Ser Ser Gln Ser Val Pro Arg Phe Gly Thr Arg Tyr Val Cys
        80                  85                  90

cag ttt cca gcc cag gaa gaa gtg cgt ctc ttc tct ccg ctg cac ctc      337
Gln Phe Pro Ala Gln Glu Glu Val Arg Leu Phe Ser Pro Leu His Leu
    95                  100                 105

tgg gtg aag aat gtg ttc cta aac cag act cag att cag cga gtc ctc      385
Trp Val Lys Asn Val Phe Leu Asn Gln Thr Gln Ile Gln Arg Val Leu
110                 115                 120                 125

ttt gtg gac agt gta ggc ctg ccg gct ccc ccc agt atc atc aag gcc      433
Phe Val Asp Ser Val Gly Leu Pro Ala Pro Pro Ser Ile Ile Lys Ala
                130                 135                 140

atg ggt ggg agc cag cca ggg gaa ctt cag atc agc tgg gag gcc cca      481
Met Gly Gly Ser Gln Pro Gly Glu Leu Gln Ile Ser Trp Glu Ala Pro
            145                 150                 155

gct cca gaa atc agt gat ttc ctg agg tac gaa ctc cgc tat ggc ccc      529
Ala Pro Glu Ile Ser Asp Phe Leu Arg Tyr Glu Leu Arg Tyr Gly Pro
        160                 165                 170

aaa gat ctc aag aac tcc act ggt ccc acg gtc ata cag ttg atc gcc      577
Lys Asp Leu Lys Asn Ser Thr Gly Pro Thr Val Ile Gln Leu Ile Ala
    175                 180                 185
```

aca gaa acc tgc tgc cct gct ctg cag agg cca cac tca gcc tct gct 625
Thr Glu Thr Cys Cys Pro Ala Leu Gln Arg Pro His Ser Ala Ser Ala
190 195 200 205

ctg gac cag tct cca tgt gct cag ccc aca atg ccc tgg caa gat gga 673
Leu Asp Gln Ser Pro Cys Ala Gln Pro Thr Met Pro Trp Gln Asp Gly
210 215 220

cca aag cag acc tcc cca act aga gaa gct tca gct ctg aca gca gtg 721
Pro Lys Gln Thr Ser Pro Thr Arg Glu Ala Ser Ala Leu Thr Ala Val
225 230 235

ggt gga agc tgc ctc atc tca gga ctc cag cct ggc aac tcc tac tgg 769
Gly Gly Ser Cys Leu Ile Ser Gly Leu Gln Pro Gly Asn Ser Tyr Trp
240 245 250

ctg cag ctg cgc agc gaa cct gat ggg atc tcc ctc ggt ggc tcc tgg 817
Leu Gln Leu Arg Ser Glu Pro Asp Gly Ile Ser Leu Gly Gly Ser Trp
255 260 265

gga tcc tgg tcc ctc cct gtg act gtg gac ctg cct gga gat gca gtg 865
Gly Ser Trp Ser Leu Pro Val Thr Val Asp Leu Pro Gly Asp Ala Val
270 275 280 285

gca att gga ctg caa tgc ttt acc ttg gac ctg aag aat gtt acc tgt 913
Ala Ile Gly Leu Gln Cys Phe Thr Leu Asp Leu Lys Asn Val Thr Cys
290 295 300

caa tgg cag caa gag gac cat gct agt tcc caa ggt ttc ttc tac cac 961
Gln Trp Gln Gln Glu Asp His Ala Ser Ser Gln Gly Phe Phe Tyr His
305 310 315

agc agg gca cgg tgc tgc ccc aga gac agg tac ccc atc tgg gag gac 1009
Ser Arg Ala Arg Cys Cys Pro Arg Asp Arg Tyr Pro Ile Trp Glu Asp
320 325 330

tgt gaa gag gaa gag aaa aca aat cca gga tta cag acc cca cag ttc 1057
Cys Glu Glu Glu Glu Lys Thr Asn Pro Gly Leu Gln Thr Pro Gln Phe
335 340 345

tct cgc tgc cac ttc aag tca cga aat gac agc gtt att cac atc ctt 1105
Ser Arg Cys His Phe Lys Ser Arg Asn Asp Ser Val Ile His Ile Leu
350 355 360 365

gtg gag gtg acc aca gcc ctg ggt gct gtt cac agt tac ctg ggc tcc 1153
Val Glu Val Thr Thr Ala Leu Gly Ala Val His Ser Tyr Leu Gly Ser
370 375 380

cct ttc tgg atc cac cag gct gtg cgc ctc ccc acc cca aac ttg cac 1201
Pro Phe Trp Ile His Gln Ala Val Arg Leu Pro Thr Pro Asn Leu His
385 390 395

tgg agg gag atc tcc agc ggg cat ctg gaa ttg gag tgg cag cac cca 1249
Trp Arg Glu Ile Ser Ser Gly His Leu Glu Leu Glu Trp Gln His Pro
400 405 410

tca tcc tgg gca gcc caa gag acc tgc tat caa ctc cga tac aca gga 1297
Ser Ser Trp Ala Ala Gln Glu Thr Cys Tyr Gln Leu Arg Tyr Thr Gly
415 420 425

gaa ggc cat cag gac tgg aag gtg ctg gag ccg cct ctc ggg gcc cga 1345
Glu Gly His Gln Asp Trp Lys Val Leu Glu Pro Pro Leu Gly Ala Arg
430 435 440 445

gga ggg acc ctg gag ctg cgc ccg cga tct cgc tac cgt tta cag ctg 1393
Gly Gly Thr Leu Glu Leu Arg Pro Arg Ser Arg Tyr Arg Leu Gln Leu
450 455 460

cgc gcc agg ctc aat ggc ccc acc tac caa ggt ccc tgg agc tcg tgg 1441
Arg Ala Arg Leu Asn Gly Pro Thr Tyr Gln Gly Pro Trp Ser Ser Trp
465 470 475

```
tcg gac cca gct agg gtg gag acc gcc acc gag acc gcc tgg att tcc      1489
Ser Asp Pro Ala Arg Val Glu Thr Ala Thr Glu Thr Ala Trp Ile Ser
        480                 485                 490

ttg gtg acc gct ctg ctg cta gtg ctg ggc ctc agc gcc gtc ctg ggc      1537
Leu Val Thr Ala Leu Leu Leu Val Leu Gly Leu Ser Ala Val Leu Gly
    495                 500                 505

ctg ctg ctg ctg agg tgg cag ttt cct gca cac tac agg aga ctg agg      1585
Leu Leu Leu Leu Arg Trp Gln Phe Pro Ala His Tyr Arg Arg Leu Arg
510                 515                 520                 525

cat gcc ctg tgg ccc tca ctt cca gat ctg cac cga gtc cta ggc cag      1633
His Ala Leu Trp Pro Ser Leu Pro Asp Leu His Arg Val Leu Gly Gln
                530                 535                 540

tac ctt agg gac act gca gcc ctg agt ccg ccc aag gcc aca gtc tca      1681
Tyr Leu Arg Asp Thr Ala Ala Leu Ser Pro Pro Lys Ala Thr Val Ser
            545                 550                 555

gat acc tgt gaa gaa gtg gaa ccc agc ctc ctt gaa atc ctc ccc aag      1729
Asp Thr Cys Glu Glu Val Glu Pro Ser Leu Leu Glu Ile Leu Pro Lys
        560                 565                 570

tcc tca gag agg act cct ttg ccc ctg tgt tcc tcc cag tcc cag atg      1777
Ser Ser Glu Arg Thr Pro Leu Pro Leu Cys Ser Ser Gln Ser Gln Met
    575                 580                 585

gac tac cga aga ttg cag cct tct tgc ctg ggg acc atg ccc ctg tct      1825
Asp Tyr Arg Arg Leu Gln Pro Ser Cys Leu Gly Thr Met Pro Leu Ser
590                 595                 600                 605

gtg tgc cca ccc atg gct gag tca ggg tcc tgc tgt acc acc cac att      1873
Val Cys Pro Pro Met Ala Glu Ser Gly Ser Cys Cys Thr Thr His Ile
                610                 615                 620
```

gcc aac cat tcc tac cta cca cta agc tat tgg cag cag cct tga 1918
Ala Asn His Ser Tyr Leu Pro Leu Ser Tyr Trp Gln Gln Pro
625 630 635

gtcgac 1924

<210> 53
<211> 635
<212> PRT
<213> Macaca fascicularis

<400> 53

Met Pro Ser Trp Ala Leu Phe Met Val Thr Ser Cys Leu Leu Leu Ala
1 5 10 15

Pro Gln Asn Leu Ala Gln Val Ser Ser Gln Asp Val Ser Leu Leu Ala
20 25 30

Ser Asp Ser Glu Pro Leu Lys Cys Phe Ser Arg Thr Phe Glu Asp Leu
35 40 45

Thr Cys Phe Trp Asp Glu Glu Glu Ala Ala Pro Ser Gly Thr Tyr Gln
50 55 60

Leu Leu Tyr Ala Tyr Pro Gly Glu Lys Pro Arg Ala Cys Pro Leu Ser
65 70 75 80

Ser Gln Ser Val Pro Arg Phe Gly Thr Arg Tyr Val Cys Gln Phe Pro

85 90 95

Ala Gln Glu Glu Val Arg Leu Phe Ser Pro Leu His Leu Trp Val Lys
100 105 110

Asn Val Phe Leu Asn Gln Thr Gln Ile Gln Arg Val Leu Phe Val Asp
115 120 125

Ser Val Gly Leu Pro Ala Pro Pro Ser Ile Ile Lys Ala Met Gly Gly
130 135 140

Ser Gln Pro Gly Glu Leu Gln Ile Ser Trp Glu Ala Pro Ala Pro Glu
145 150 155 160

Ile Ser Asp Phe Leu Arg Tyr Glu Leu Arg Tyr Gly Pro Lys Asp Leu
165 170 175

Lys Asn Ser Thr Gly Pro Thr Val Ile Gln Leu Ile Ala Thr Glu Thr
180 185 190

Cys Cys Pro Ala Leu Gln Arg Pro His Ser Ala Ser Ala Leu Asp Gln
195 200 205

Ser Pro Cys Ala Gln Pro Thr Met Pro Trp Gln Asp Gly Pro Lys Gln
210 215 220

Thr Ser Pro Thr Arg Glu Ala Ser Ala Leu Thr Ala Val Gly Gly Ser

225 230 235 240

Cys Leu Ile Ser Gly Leu Gln Pro Gly Asn Ser Tyr Trp Leu Gln Leu
245 250 255

Arg Ser Glu Pro Asp Gly Ile Ser Leu Gly Gly Ser Trp Gly Ser Trp
260 265 270

Ser Leu Pro Val Thr Val Asp Leu Pro Gly Asp Ala Val Ala Ile Gly
275 280 285

Leu Gln Cys Phe Thr Leu Asp Leu Lys Asn Val Thr Cys Gln Trp Gln
290 295 300

Gln Glu Asp His Ala Ser Ser Gln Gly Phe Phe Tyr His Ser Arg Ala
305 310 315 320

Arg Cys Cys Pro Arg Asp Arg Tyr Pro Ile Trp Glu Asp Cys Glu Glu
325 330 335

Glu Glu Lys Thr Asn Pro Gly Leu Gln Thr Pro Gln Phe Ser Arg Cys
340 345 350

His Phe Lys Ser Arg Asn Asp Ser Val Ile His Ile Leu Val Glu Val
355 360 365

Thr Thr Ala Leu Gly Ala Val His Ser Tyr Leu Gly Ser Pro Phe Trp

370 375 380

Ile His Gln Ala Val Arg Leu Pro Thr Pro Asn Leu His Trp Arg Glu
385 390 395 400

Ile Ser Ser Gly His Leu Glu Leu Glu Trp Gln His Pro Ser Ser Trp
405 410 415

Ala Ala Gln Glu Thr Cys Tyr Gln Leu Arg Tyr Thr Gly Glu Gly His
420 425 430

Gln Asp Trp Lys Val Leu Glu Pro Pro Leu Gly Ala Arg Gly Gly Thr
435 440 445

Leu Glu Leu Arg Pro Arg Ser Arg Tyr Arg Leu Gln Leu Arg Ala Arg
450 455 460

Leu Asn Gly Pro Thr Tyr Gln Gly Pro Trp Ser Ser Trp Ser Asp Pro
465 470 475 480

Ala Arg Val Glu Thr Ala Thr Glu Thr Ala Trp Ile Ser Leu Val Thr
485 490 495

Ala Leu Leu Leu Val Leu Gly Leu Ser Ala Val Leu Gly Leu Leu Leu
500 505 510

Leu Arg Trp Gln Phe Pro Ala His Tyr Arg Arg Leu Arg His Ala Leu

515 520 525

Trp Pro Ser Leu Pro Asp Leu His Arg Val Leu Gly Gln Tyr Leu Arg
530 535 540

Asp Thr Ala Ala Leu Ser Pro Pro Lys Ala Thr Val Ser Asp Thr Cys
545 550 555 560

Glu Glu Val Glu Pro Ser Leu Leu Glu Ile Leu Pro Lys Ser Ser Glu
565 570 575

Arg Thr Pro Leu Pro Leu Cys Ser Ser Gln Ser Gln Met Asp Tyr Arg
580 585 590

Arg Leu Gln Pro Ser Cys Leu Gly Thr Met Pro Leu Ser Val Cys Pro
595 600 605

Pro Met Ala Glu Ser Gly Ser Cys Cys Thr Thr His Ile Ala Asn His
610 615 620

Ser Tyr Leu Pro Leu Ser Tyr Trp Gln Gln Pro
625 630 635

<210> 54
<211> 30
<212> PRT
<213> Homo sapiens

<400> 54

Gln Val Gln Leu Val Gln Ser Gly Pro Glu Val Lys Lys Pro Gly Ala
1 5 10 15

Ser Val Lys Val Ser Cys Lys Ala Ser Gly Tyr Thr Phe Thr
20 25 30

<210> 55
<211> 14
<212> PRT
<213> Homo sapiens

<400> 55

Trp Val Arg Gln Arg Pro Gly Lys Gly Leu Glu Trp Ile Gly
1 5 10

<210> 56
<211> 32
<212> PRT
<213> Homo sapiens

<400> 56

Arg Val Thr Ile Thr Ala Asp Glu Ser Thr Ser Thr Ala Tyr Met Gln
1 5 10 15

Leu Ser Ser Leu Arg Ser Glu Asp Thr Ala Val Tyr Tyr Cys Ala Arg
20 25 30

<210> 57
<211> 11
<212> PRT
<213> Homo sapiens

<400> 57

Trp Gly Gln Gly Thr Thr Val Thr Val Ser Ser
1 5 10

<210> 58
<211> 32
<212> PRT
<213> Homo sapiens

<400> 58

Arg Val Thr Ile Thr Ala Asp Glu Ser Thr Ser Thr Ala Tyr Met Glu
1 5 10 15

Leu Ser Ser Leu Arg Ser Glu Asp Thr Ala Val Tyr Tyr Cys Ala Arg
20 25 30

<210> 59
<211> 23
<212> PRT
<213> Homo sapiens

<400> 59

Asp Ile Val Met Thr Gln Ser Pro Leu Ser Leu Pro Val Thr Pro Gly
1 5 10 15

Glu Pro Ala Ser Ile Ser Cys
20

<210> 60
<211> 15
<212> PRT
<213> Homo sapiens

<400> 60

Trp Phe Leu Gln Lys Pro Gly Gln Ser Pro Gln Leu Leu Ile Tyr
1 5 10 15

<210> 61
<211> 32
<212> PRT
<213> Homo sapiens

<400> 61

Gly Val Pro Asp Arg Phe Ser Gly Ser Gly Ser Gly Thr Asp Phe Thr
1 5 10 15

Leu Lys Ile Ser Arg Val Glu Ala Glu Asp Val Gly Val Tyr Tyr Cys
20 25 30

<210> 62
<211> 10
<212> PRT
<213> Homo sapiens

<400> 62

Phe Gly Gln Gly Thr Lys Leu Glu Ile Lys
1 5 10

<210> 63
<211> 15
<212> PRT
<213> Homo sapiens

<400> 63

Trp Phe Gln Gln Lys Pro Gly Gln Ala Pro Arg Leu Leu Ile Tyr
1 5 10 15

<210> 64
<211> 32
<212> PRT
<213> Homo sapiens

<400> 64

Gly Val Pro Asp Arg Phe Ser Gly Ser Gly Ser Gly Thr Ala Phe Thr
1 5 10 15

Leu Lys Ile Ser Arg Val Glu Ala Glu Asp Val Gly Val Tyr Tyr Cys
20 25 30

<210> 65
<211> 118
<212> PRT
<213> Homo sapiens

<400> 65

Gln Val Gln Leu Val Gln Ser Gly Pro Glu Val Lys Lys Pro Gly Ala
1 5 10 15

Ser Val Lys Val Ser Cys Lys Ala Ser Gly Tyr Thr Phe Thr Asn Ser
20 25 30

Trp Met Asn Trp Val Arg Gln Arg Pro Gly Lys Gly Leu Glu Trp Ile
35 40 45

Gly Arg Ile Tyr Pro Gly Asp Gly Glu Thr Ile Tyr Asn Gly Lys Phe
50 55 60

Arg Val Arg Val Thr Ile Thr Ala Asp Glu Ser Thr Ser Thr Ala Tyr
65 70 75 80

Met Gln Leu Ser Ser Leu Arg Ser Glu Asp Thr Ala Val Tyr Tyr Cys
85 90 95

Ala Arg Gly Tyr Asp Asp Tyr Ser Phe Ala Tyr Trp Gly Gln Gly Thr
100 105 110

Thr Val Thr Val Ser Ser
115

<210> 66

<211> 118
<212> PRT
<213> Homo sapiens

<400> 66

Gln Val Gln Leu Val Gln Ser Gly Pro Glu Val Lys Lys Pro Gly Ala
1 5 10 15

Ser Val Lys Val Ser Cys Lys Ala Ser Gly Tyr Thr Phe Thr Asn Ser
20 25 30

Trp Met Asn Trp Val Arg Gln Arg Pro Gly Lys Gly Leu Glu Trp Ile
35 40 45

Gly Arg Ile Tyr Pro Gly Asp Gly Glu Thr Ile Tyr Asn Gly Lys Phe
50 55 60

Arg Val Arg Val Thr Ile Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Gly Tyr Asp Asp Tyr Ser Phe Ala Tyr Trp Gly Gln Gly Thr
100 105 110

Thr Val Thr Val Ser Ser
115

<210> 67
<211> 112
<212> PRT
<213> Homo sapiens

<400> 67

Asp Ile Val Met Thr Gln Ser Pro Leu Ser Leu Pro Val Thr Pro Gly
1 5 10 15

Glu Pro Ala Ser Ile Ser Cys Arg Ser Ser Lys Ser Leu Leu His Ser
20 25 30

Asn Gly Asn Thr Tyr Leu Tyr Trp Phe Leu Gln Lys Pro Gly Gln Ser
35 40 45

Pro Gln Leu Leu Ile Tyr Arg Met Ser Asn Leu Ala Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Met Gln His
85 90 95

Ile Glu Tyr Pro Phe Thr Phe Gly Gln Gly Thr Lys Leu Glu Ile Lys
100 105 110

<210> 68
<211> 112
<212> PRT
<213> Homo sapiens

<400> 68

Asp Ile Val Met Thr Gln Ser Pro Leu Ser Leu Pro Val Thr Pro Gly
1 5 10 15

Glu Pro Ala Ser Ile Ser Cys Arg Ser Ser Lys Ser Leu Leu His Ser
20 25 30

Asn Gly Asn Thr Tyr Leu Tyr Trp Phe Gln Gln Lys Pro Gly Gln Ala
35 40 45

Pro Arg Leu Leu Ile Tyr Arg Met Ser Asn Leu Ala Ser Gly Val Pro
50 55 60

Asp Arg Phe Ser Gly Ser Gly Ser Gly Thr Ala Phe Thr Leu Lys Ile
65 70 75 80

Ser Arg Val Glu Ala Glu Asp Val Gly Val Tyr Tyr Cys Met Gln His
85 90 95

Ile Glu Tyr Pro Phe Thr Phe Gly Gln Gly Thr Lys Leu Glu Ile Lys
100 105 110

<210> 69
<211> 354
<212> DNA
<213> Homo sapiens

<400> 69
caggtgcagc tggtgcagtc tggacctgag gtgaagaagc ctggggcctc agtgaaggtc 60

tcctgcaagg cttctggata caccttcacc aactcctgga tgaactgggt gaggcagagg 120

cctggaaagg gtcttgagtg gattggacgg atttatcctg gagatggaga aactatctac 180

aatgggaaat tcagggtcag agtcacgatt accgcggacg aatccacgag cacagcctac 240

atgcaactga gcagcctgag atctgaggac acggccgtgt attactgtgc gagaggctat 300

gatgattact cgtttgctta ctggggccag ggaaccacgg tcaccgtctc ttca 354

<210> 70
<211> 336
<212> DNA
<213> Homo sapiens

<400> 70
gatattgtga tgactcagtc tccactctcc ctgcccgtca cccctggaga gccggcctcc 60

atctcctgca ggtctagtaa gagtctcctg catagtaatg gcaacactta cttgtattgg 120

ttcctgcaga agccagggca gtctccacag ctcctgatct atcggatgtc caaccttgcc 180

tcaggggtcc ctgacaggtt cagtggcagt ggatcaggca cagattttac actgaaaatc 240

agcagagtgg aggctgagga tgttggggtt tattactgca tgcaacatat agaatatcct 300

tttacgttcg gccaagggac caaactggaa atcaaa 336

<210> 71
<211> 354
<212> DNA
<213> Homo sapiens

<400> 71
caggtgcagc tggtgcagtc tggacctgag gtgaagaagc ctggggcctc agtgaaggtc 60

tcctgcaagg cttctggata caccttcacc aactcctgga tgaactgggt gaggcagagg 120

cctggaaagg gtcttgagtg gattggacgg atttatcctg gagatggaga aactatctac 180

aatgggaaat tcagggtcag agtcacgatt accgcggacg aatccacgag cacagcctac 240

atggagctga gcagcctgag atctgaggac acggccgtgt attactgtgc gagaggctat 300

gatgattact cgtttgctta ctggggccag ggaaccacgg tcaccgtctc ttca 354

<210> 72
<211> 336
<212> DNA
<213> Homo sapiens

<400> 72
gatattgtga tgactcagtc tccactctcc ctgcccgtca cccctggaga gccggcctcc 60

atctcctgca ggtctagtaa gagtctcctg catagtaatg gcaacactta cttgtattgg 120

ttccagcaga agccagggca ggctccacgg ctcctgatct atcggatgtc caaccttgcc 180

tcaggggtcc ctgacaggtt cagtggcagt ggatcaggca cagcttttac actgaaaatc 240

agcagagtgg aggctgagga tgttggggtt tattactgca tgcaacatat agaatatcct 300

tttacgttcg gccaagggac caaactggaa atcaaa 336

<210> 73
<211> 524
<212> PRT
<213> Homo sapiens

<400> 73

Met Asp Trp Thr Trp Arg Phe Leu Phe Val Val Ala Ala Ala Thr Gly
1 5 10 15

Val Gln Ser Gln Val Gln Leu Val Gln Ser Gly Pro Glu Val Lys Lys
20 25 30

Pro Gly Ala Ser Val Lys Val Ser Cys Lys Ala Ser Gly Tyr Thr Phe
35 40 45

Thr Asn Ser Trp Met Asn Trp Val Arg Gln Arg Pro Gly Lys Gly Leu
50 55 60

Glu Trp Ile Gly Arg Ile Tyr Pro Gly Asp Gly Glu Thr Ile Tyr Asn
65 70 75 80

Gly Lys Phe Arg Val Arg Val Thr Ile Thr Ala Asp Glu Ser Thr Ser
85 90 95

Thr Ala Tyr Met Gln Leu Ser Ser Leu Arg Ser Glu Asp Thr Ala Val

100 105 110

Tyr Tyr Cys Ala Arg Gly Tyr Asp Asp Tyr Ser Phe Ala Tyr Trp Gly
115 120 125

Gln Gly Thr Thr Val Thr Val Ser Ser Gly Gly Gly Gly Ser Gly Gly
130 135 140

Gly Gly Ser Gly Gly Gly Gly Ser Asp Ile Val Met Thr Gln Ser Pro
145 150 155 160

Leu Ser Leu Pro Val Thr Pro Gly Glu Pro Ala Ser Ile Ser Cys Arg
165 170 175

Ser Ser Lys Ser Leu Leu His Ser Asn Gly Asn Thr Tyr Leu Tyr Trp
180 185 190

Phe Leu Gln Lys Pro Gly Gln Ser Pro Gln Leu Leu Ile Tyr Arg Met
195 200 205

Ser Asn Leu Ala Ser Gly Val Pro Asp Arg Phe Ser Gly Ser Gly Ser
210 215 220

Gly Thr Asp Phe Thr Leu Lys Ile Ser Arg Val Glu Ala Glu Asp Val
225 230 235 240

Gly Val Tyr Tyr Cys Met Gln His Ile Glu Tyr Pro Phe Thr Phe Gly

245 250 255

Gln Gly Thr Lys Leu Glu Ile Lys Gly Gly Gly Gly Ser Gly Gly Gly
260 265 270

Gly Ser Gly Gly Gly Gly Ser Gln Val Gln Leu Val Gln Ser Gly Pro
275 280 285

Glu Val Lys Lys Pro Gly Ala Ser Val Lys Val Ser Cys Lys Ala Ser
290 295 300

Gly Tyr Thr Phe Thr Asn Ser Trp Met Asn Trp Val Arg Gln Arg Pro
305 310 315 320

Gly Lys Gly Leu Glu Trp Ile Gly Arg Ile Tyr Pro Gly Asp Gly Glu
325 330 335

Thr Ile Tyr Asn Gly Lys Phe Arg Val Arg Val Thr Ile Thr Ala Asp
340 345 350

Glu Ser Thr Ser Thr Ala Tyr Met Gln Leu Ser Ser Leu Arg Ser Glu
355 360 365

Asp Thr Ala Val Tyr Tyr Cys Ala Arg Gly Tyr Asp Asp Tyr Ser Phe
370 375 380

Ala Tyr Trp Gly Gln Gly Thr Thr Val Thr Val Ser Ser Gly Gly Gly

385 390 395 400

Gly Ser Gly Gly Gly Gly Ser Gly Gly Gly Gly Ser Asp Ile Val Met
405 410 415

Thr Gln Ser Pro Leu Ser Leu Pro Val Thr Pro Gly Glu Pro Ala Ser
420 425 430

Ile Ser Cys Arg Ser Ser Lys Ser Leu Leu His Ser Asn Gly Asn Thr
435 440 445

Tyr Leu Tyr Trp Phe Leu Gln Lys Pro Gly Gln Ser Pro Gln Leu Leu
450 455 460

Ile Tyr Arg Met Ser Asn Leu Ala Ser Gly Val Pro Asp Arg Phe Ser
465 470 475 480

Gly Ser Gly Ser Gly Thr Asp Phe Thr Leu Lys Ile Ser Arg Val Glu
485 490 495

Ala Glu Asp Val Gly Val Tyr Tyr Cys Met Gln His Ile Glu Tyr Pro
500 505 510

Phe Thr Phe Gly Gln Gly Thr Lys Leu Glu Ile Lys
515 520

<210> 74

<211> 524
<212> PRT
<213> Homo sapiens

<400> 74

Met Asp Trp Thr Trp Arg Phe Leu Phe Val Val Ala Ala Ala Thr Gly
1 5 10 15

Val Gln Ser Gln Val Gln Leu Val Gln Ser Gly Pro Glu Val Lys Lys
20 25 30

Pro Gly Ala Ser Val Lys Val Ser Cys Lys Ala Ser Gly Tyr Thr Phe
35 40 45

Thr Asn Ser Trp Met Asn Trp Val Arg Gln Arg Pro Gly Lys Gly Leu
50 55 60

Glu Trp Ile Gly Arg Ile Tyr Pro Gly Asp Gly Glu Thr Ile Tyr Asn
65 70 75 80

Gly Lys Phe Arg Val Arg Val Thr Ile Thr Ala Asp Glu Ser Thr Ser
85 90 95

Thr Ala Tyr Met Glu Leu Ser Ser Leu Arg Ser Glu Asp Thr Ala Val
100 105 110

Tyr Tyr Cys Ala Arg Gly Tyr Asp Asp Tyr Ser Phe Ala Tyr Trp Gly
115 120 125

Gln Gly Thr Thr Val Thr Val Ser Ser Gly Gly Gly Gly Ser Gly Gly
130 135 140

Gly Gly Ser Gly Gly Gly Gly Ser Asp Ile Val Met Thr Gln Ser Pro
145 150 155 160

Leu Ser Leu Pro Val Thr Pro Gly Glu Pro Ala Ser Ile Ser Cys Arg
165 170 175

Ser Ser Lys Ser Leu Leu His Ser Asn Gly Asn Thr Tyr Leu Tyr Trp
180 185 190

Phe Gln Gln Lys Pro Gly Gln Ala Pro Arg Leu Leu Ile Tyr Arg Met
195 200 205

Ser Asn Leu Ala Ser Gly Val Pro Asp Arg Phe Ser Gly Ser Gly Ser
210 215 220

Gly Thr Ala Phe Thr Leu Lys Ile Ser Arg Val Glu Ala Glu Asp Val
225 230 235 240

Gly Val Tyr Tyr Cys Met Gln His Ile Glu Tyr Pro Phe Thr Phe Gly
245 250 255

Gln Gly Thr Lys Leu Glu Ile Lys Gly Gly Gly Gly Ser Gly Gly Gly
260 265 270

Gly Ser Gly Gly Gly Gly Ser Gln Val Gln Leu Val Gln Ser Gly Pro
275 280 285

Glu Val Lys Lys Pro Gly Ala Ser Val Lys Val Ser Cys Lys Ala Ser
290 295 300

Gly Tyr Thr Phe Thr Asn Ser Trp Met Asn Trp Val Arg Gln Arg Pro
305 310 315 320

Gly Lys Gly Leu Glu Trp Ile Gly Arg Ile Tyr Pro Gly Asp Gly Glu
325 330 335

Thr Ile Tyr Asn Gly Lys Phe Arg Val Arg Val Thr Ile Thr Ala Asp
340 345 350

Glu Ser Thr Ser Thr Ala Tyr Met Glu Leu Ser Ser Leu Arg Ser Glu
355 360 365

Asp Thr Ala Val Tyr Tyr Cys Ala Arg Gly Tyr Asp Asp Tyr Ser Phe
370 375 380

Ala Tyr Trp Gly Gln Gly Thr Thr Val Thr Val Ser Ser Gly Gly Gly
385 390 395 400

Gly Ser Gly Gly Gly Gly Ser Gly Gly Gly Gly Ser Asp Ile Val Met
405 410 415

Thr Gln Ser Pro Leu Ser Leu Pro Val Thr Pro Gly Glu Pro Ala Ser
420 425 430

Ile Ser Cys Arg Ser Ser Lys Ser Leu Leu His Ser Asn Gly Asn Thr
435 440 445

Tyr Leu Tyr Trp Phe Gln Gln Lys Pro Gly Gln Ala Pro Arg Leu Leu
450 455 460

Ile Tyr Arg Met Ser Asn Leu Ala Ser Gly Val Pro Asp Arg Phe Ser
465 470 475 480

Gly Ser Gly Ser Gly Thr Ala Phe Thr Leu Lys Ile Ser Arg Val Glu
485 490 495

Ala Glu Asp Val Gly Val Tyr Tyr Cys Met Gln His Ile Glu Tyr Pro
500 505 510

Phe Thr Phe Gly Gln Gly Thr Lys Leu Glu Ile Lys
515 520

<210> 75
<211> 1572
<212> DNA
<213> Homo sapiens

<400> 75

```
atggactgga cctggaggtt cctctttgtg gtggcagcag ctacaggtgt ccagtcccag     60
gtgcagctgg tgcagtctgg acctgaggtg aagaagcctg gggcctcagt gaaggtctcc    120
tgcaaggctt ctggatacac cttcaccaac tcctggatga actgggtgag gcagaggcct    180
ggaaagggtc ttgagtggat tggacggatt tatcctggag atggagaaac tatctacaat    240
gggaaattca gggtcagagt cacgattacc gcggacgaat ccacgagcac agcctacatg    300
caactgagca gcctgagatc tgaggacacg gccgtgtatt actgtgcgag aggctatgat    360
gattactcgt ttgcttactg gggccaggga accacggtca ccgtctcttc aggtggtggt    420
ggatccggag gtggtggatc gggtggtgga ggatcggata ttgtgatgac tcagtctcca    480
ctctccctgc ccgtcacccc tggagagccg gcctccatct cctgcaggtc tagtaagagt    540
ctcctgcata gtaatggcaa cacttacttg tattggttcc tgcagaagcc agggcagtct    600
ccacagctcc tgatctatcg gatgtccaac cttgcctcag gggtccctga caggttcagt    660
ggcagtggat caggcacaga ttttacactg aaaatcagca gagtggaggc tgaggatgtt    720
ggggtttatt actgcatgca acatatagaa tatcctttta cgttcggcca agggaccaaa    780
ctggaaatca aaggaggtgg tggatcgggt ggtggtggtt cgggaggcgg tggatcgcag    840
gtgcagctgg tgcagtctgg acctgaggtg aagaagcctg gggcctcagt gaaggtctcc    900
tgcaaggctt ctggatacac cttcaccaac tcctggatga actgggtgag gcagaggcct    960
ggaaagggtc ttgagtggat tggacggatt tatcctggag atggagaaac tatctacaat   1020
gggaaattca gggtcagagt cacgattacc gcggacgaat ccacgagcac agcctacatg   1080
```

caactgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc tgcagaagcc agggcagtct 1380

ccacagctcc tgatctatcg gatgtccaac cttgcctcag gggtccctga caggttcagt 1440

ggcagtggat caggcacaga ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 76
<211> 1572
<212> DNA
<213> Homo sapiens

<400> 76
atggactgga cctggaggtt cctctttgtg gtggcagcag ctacaggtgt ccagtcccag 60

gtgcagctgg tgcagtctgg acctgaggtg aagaagcctg gggcctcagt gaaggtctcc 120

tgcaaggctt ctggatacac cttcaccaac tcctggatga actgggtgag gcagaggcct 180

ggaaagggtc ttgagtggat tggacggatt tatcctggag atggagaaac tatctacaat 240

gggaaattca gggtcagagt cacgattacc gcggacgaat ccacgagcac agcctacatg 300

gagctgagca gcctgagatc tgaggacacg gccgtgtatt actgtgcgag aggctatgat 360

gattactcgt ttgcttactg gggccaggga accacggtca ccgtctcttc aggtggtggt 420

ggatccggag gtggtggatc gggtggtgga ggatcggata ttgtgatgac tcagtctcca 480

ctctccctgc ccgtcacccc tggagagccg gcctccatct cctgcaggtc tagtaagagt 540

ctcctgcata gtaatggcaa cacttacttg tattggttcc agcagaagcc agggcaggct 600

ccacggctcc tgatctatcg gatgtccaac cttgcctcag gggtccctga caggttcagt 660

ggcagtggat caggcacagc ttttacactg aaaatcagca gagtggaggc tgaggatgtt 720

ggggtttatt actgcatgca acatatagaa tatcctttta cgttcggcca agggaccaaa 780

ctggaaatca aaggaggtgg tggatcgggt ggtggtggtt cgggaggcgg tggatcgcag 840

gtgcagctgg tgcagtctgg acctgaggtg aagaagcctg gggcctcagt gaaggtctcc 900

tgcaaggctt ctggatacac cttcaccaac tcctggatga actgggtgag gcagaggcct 960

ggaaagggtc ttgagtggat tggacggatt tatcctggag atggagaaac tatctacaat 1020

gggaaattca gggtcagagt cacgattacc gcggacgaat ccacgagcac agcctacatg 1080

gagctgagca gcctgagatc tgaggacacg gccgtgtatt actgtgcgag aggctatgat 1140

gattactcgt ttgcttactg gggccaggga accacggtca ccgtctcttc aggtggtggt 1200

ggatccggag gtggtggatc gggtggtgga ggatcggata ttgtgatgac tcagtctcca 1260

ctctccctgc ccgtcacccc tggagagccg gcctccatct cctgcaggtc tagtaagagt 1320

ctcctgcata gtaatggcaa cacttacttg tattggttcc agcagaagcc agggcaggct 1380

ccacggctcc tgatctatcg gatgtccaac cttgcctcag gggtccctga caggttcagt 1440

ggcagtggat caggcacagc ttttacactg aaaatcagca gagtggaggc tgaggatgtt 1500

ggggtttatt actgcatgca acatatagaa tatcctttta cgttcggcca agggaccaaa 1560

ctggaaatca aa 1572

<210> 77
<211> 4
<212> PRT
<213> Artificial

<220>
<223> An artificially synthesized linker sequence

<400> 77

Gly Gly Gly Ser
1

<210> 78
<211> 4
<212> PRT
<213> Artificial

<220>
<223> An artificially synthesized linker sequence

<400> 78

Ser Gly Gly Gly
1

<210> 79
<211> 5
<212> PRT
<213> Artificial

<220>
<223> An artificially synthesized linker sequence

<400> 79

Gly Gly Gly Gly Ser
1 5

<210> 80
<211> 5
<212> PRT
<213> Artificial

<220>
<223> An artificially synthesized linker sequence

<400> 80

Ser Gly Gly Gly Gly
1 5

<210> 81
<211> 6
<212> PRT
<213> Artificial

<220>
<223> An artificially synthesized linker sequence

<400> 81

Gly Gly Gly Gly Gly Ser
1 5

<210> 82
<211> 6
<212> PRT
<213> Artificial

<220>
<223> An artificially synthesized linker sequence

<400> 82

Ser Gly Gly Gly Gly Gly
1 5

<210> 83
<211> 7
<212> PRT
<213> Artificial

<220>
<223> An artificially synthesized linker sequence

<400> 83

Gly Gly Gly Gly Gly Gly Ser
1 5

<210> 84
<211> 7

<212> PRT
<213> Artificial

<220>
<223> An artificially synthesized linker sequence

<400> 84

Ser Gly Gly Gly Gly Gly Gly
1 5